(19) Europäisches Patentamt / European Patent Office / Office européen des brevets

(11) EP 1 819 324 B1

(12) **EUROPEAN PATENT SPECIFICATION**

(45) Date of publication and mention of the grant of the patent:
**26.10.2016 Bulletin 2016/43**

(21) Application number: **05803257.4**

(22) Date of filing: **15.11.2005**

(51) Int Cl.:
***A61K 9/51** (2006.01)* ***B01J 13/18** (2006.01)*

(86) International application number:
**PCT/AU2005/001738**

(87) International publication number:
**WO 2006/050579 (18.05.2006 Gazette 2006/20)**

(54) **SOLID PARTICLES FROM CONTROLLED DESTABILISATION OF MICROEMULSIONS**

FESTE TEILCHEN AUS DER KONTROLLIERTEN DESTABILISATION VON MIKROEMULSIONEN

PARTICULES SOLIDES ISSUES DE LA DESTABILISATION CONTROLEE DE MICROEMULSIONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.11.2004 AU 2004906544**

(43) Date of publication of application:
**22.08.2007 Bulletin 2007/34**

(73) Proprietor: **AUSTRALIAN NUCLEAR SCIENCE & TECHNOLOGY ORGANISATION**
**Lucas Heights**
**New South Wales 2234 (AU)**

(72) Inventors:
- **KONG, Linggen**
  **Narwee, NSW 2209 (AU)**
- **BARBE, Christophe, Jean, Alexandre**
  **Five Dock, NSW 2046 (AU)**

(74) Representative: **Berglund, Magnus**
**Ström & Gulliksson AB**
**Studentgatan 1**
**P.O. Box 4188**
**203 13 Malmö (SE)**

(56) References cited:
**WO-A1-01/88540** **WO-A1-03/055469**
**US-A- 5 766 635** **US-A1- 2003 059 472**
**US-B1- 6 174 512**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 819 324 B1

## Description

### Technical Field

The present invention relates to a process for controlled destabilisation of microemulsions to form solid particles.

### Background of the Invention

Controlled delivery of drugs, for example for treatment of tumours, has been achieved by various methods. One method is to encapsulate the drug into a solid particle, which can be delivered to the site of action, such as the tumour, and there release the drug at a controlled rate.

In order to be effective, such particles must be of an appropriate size for lodging at a tumour site. Particles that are above approximately 300nm diameter will commonly be trapped by the lungs, liver, spleen and other organs, and will thus not preferentially lodge at the site of action. Particles that are below approximately 50nm diameter are capable of penetrating through the walls of blood vessels, and are therefore distributed throughout the body. Thus for this application, it is desirable to have a particle of approximately 50 to 300nm diameter. In order to have an appropriate release rate of an encapsulated drug, the particles should have nanoscale pores. The size of the pores governs the release rate. In order to achieve an appropriate release rate, the pores should be below approximately 2 nm, and preferably should be about 1.5 nm diameter. A further requirement is that the conditions of manufacture of the drug-laden particles should be such that they do not significantly degrade the drug. Commonly anti-cancer drugs, for example doxorubicin, are unstable in basic solution, and so it is preferable for any particles to be produced under neutral or acidic conditions.

Silica nanoparticles possess several intrinsic advantages as drug carriers for *in vivo* applications. In particular, they are biologically inert, intrinsically hydrophilic (which reduces their detection by the reticuloendothelial system) and provide extended shelf life to their payload. Moreover, it has been established that spherical particles in the 50-250 nm diameter range possessing the appropriate physico-chemical properties can be selectively distributed into tumour masses from the general circulation over a period of one to two days after intravenous injection.

Silica nanoparticles may also be used for controlled release of other substances, for example catalysts, enzymes etc.

The preparation of silica nanoparticles using base-catalysed sol-gel chemistry in microemulsions has been extensively investigated. However, this base catalysis chemistry poses two disadvantages for the encapsulation of bioactive species:

- many drugs (e.g. doxorubicin) decompose/denature under basic conditions;
- base catalysis produces mesoporous particles which release their contents too rapidly for any practical use.

In contrast, acid catalysis yields microporous particles, which exhibit much slower release of the encapsulated species. However, due to the intrinsic mechanisms of particle evolution in acid environment, the size of silica particles formed by acid catalysis is either less than 30 nm or larger than one micron.

There is therefore a need for a method to produce particles in the 50-500nm range with sustained release characteristics. The particles may have both appropriate particle size (between approximately 30nm and 1$\mu$m, optionally between approximately 50 and 250nm, in diameter) and pore size (i.e. microporous) for passive targeting of tumours and may be made under conditions that do not degrade a drug encapsulated in the particles.

WO 0188540 discloses silica-nanoparticles prepared by precipitating nano-sized cores from reagents dissolved in the aqueous compartment of a water-in-oil microemulsion and adding a reactive silicate to coat the cores with silica; and methods for functionalizing silica-coated nanoparticles.

US 6174512 discloses mesoporous silica hard spheres having an average pore diameter of 1.5-4 nm and a volume average diameter of 40-80 m and a method of producing a mesoporous silica material, including reacting, with stirring, a liquid mixture containing a tetraalkyl orthosilicate, a linear alkylamine, an inorganic acid and water, and drying and thermally treating the thus obtained solid product, wherein the amounts of the inorganic acid, alkylamine and water are 0.05-0.6 mol, 0.2-1.0 mol and 10-100 mol, respectively, per mol of the tetraalkyl orthosilicate.

US 2003059472 discloses a process for preparing submicron sized nanoparticles of a poorly water soluble compound by lyophilizing a dispersion or microdispersion of a multiphase system having an organic phase and an aqueous phase, the organic phase having the poorly water soluble organic compound therein.

### Object of the Invention

It is the object of the present invention to overcome or substantially ameliorate at least one of the above

disadvantages. It is a further object to at least partially satisfy the above need.

**Summary of the Invention**

In a first aspect of the invention there is provided a process for making a particulate substance comprising a plurality of particles having a releasable substance releasably immobilised therein and/or thereon, said process comprising:

- providing a precursor emulsion comprising droplets dispersed in a continuous liquid phase, wherein at least some of the droplets comprise a condensable species; and
- forming nuclei from the condensable species within the droplets such that at least some of the droplets comprise the condensable species or an at least partial condensate thereof;

so as to provide an emulsion comprising droplets, said droplets comprising a releasable substance and being dispersed in a continuous liquid phase, wherein at least some of the droplets comprise the nuclei; and

- at least partly destabilising the droplets to form a plurality of particles of the microporous particulate substance, said plurality of particles having said releasable substance releasably immobilised therein and/or thereon,

wherein the particles of the particulate substance are between 30 and 1000nm in diameter, said particles having micropores of less than about 1nm diameter, together with mesopores of between 1 and 50nm.

The nuclei may be primary particles. The nuclei may be nuclei for formation of primary particles. The nuclei may be nuclei for formation of solid particles (including porous solid particles) or gel particles. The process may comprise the step of forming primary particles from the nuclei. The particles of the particulate substance may be derived from the primary particles (e.g. seeded by the primary particles), which may in turn be derived from the nuclei (e.g. seeded by the nuclei) or correspond to the nuclei. The nuclei may be solid nuclei, or may be gel nuclei or a combination thereof. The nuclei may comprise polymeric silicate molecules. The nuclei may comprise pre-ceramic polymers. The pre-ceramic polymers may be capable of being converted into a ceramic material e.g. silica. The step of at least partly destabilising the droplets may comprise at least partly coalescing the droplets. It may comprise combining a coalescing liquid and the emulsion. The particles of the particulate substance may be formed or derived from the nuclei by association, coalescence or agglomeration of the nuclei or of the primary particles. The condensable species may be derived from a tetraalkoxysilane (e.g. tetramethoxysilane or tetraethoxysilane) or an alkyltrialkoxysilane (e.g. aminopropyltrimethoxysilane), or a mixture thereof, or it may comprise a silicate or a polysilicate or a mixture thereof. The droplets comprise a releasable substance, and the process makes a particulate substance comprising the releasable substance. The droplets may also comprise one or more non-releasable substances and the process may make a particulate substance comprising the releasable and non-releasable substances. The nuclei and the primary particles (if present) may or may not comprise the releasable substance (and optionally the non-releasable substance). The releasable substance may comprise a single releasable substance, or may comprise two or more individual releasable substances.

The process may additionally comprise the steps of:

- providing a second emulsion comprising droplets dispersed in a continuous liquid phase, wherein at least some of the droplets comprise nuclei, said droplets comprising a second releasable substance; and
- combining the emulsion and the second emulsion;

said steps being conducted prior to the step of at least partially destabilising the droplets. In this case, the step of at least partially destabilising may comprise at least partially destabilising the droplets of the emulsion and of the second emulsion. The resulting process may be suitable for making a particulate substance comprising the releasable substance and the second releasable substance.

The step of forming the nuclei may comprise ageing the precursor emulsion for sufficient time for formation of the nuclei from the condensable species.

The process of providing the precursor emulsion may comprise the steps of:

- providing an emulsion comprising emulsion droplets dispersed in a continuous liquid phase,
- adding a hydrolysable species to the emulsion; and
- at least partially hydrolysing the hydrolysable species within the emulsion droplets to form the condensable species.

The hydrolysable species may comprise a tetralkoxysilane, for example tetramethoxysilane or tetraethoxysilane.

The releasable substance may be an organic compound or an organometallic compound and may be a drug.

It may be an anti-cancer drug for example doxorubicin. The releasable substance may be a fluorescent dye, a radiopharmaceutical, an enzyme, a hormone, a biocide or some other substance. The releasable substance may be releasable into water or an aqueous fluid or some other solvent. It may be releasable on exposure of the particulate substance to water or the aqueous fluid or other solvent, or on immersion of the particles in water or the aqueous fluid, or on agitation of the particles in water or the aqueous fluid or other solvent.

The particles of the particulate substance are microporous and mesoporous. They have pores between 1nm and 50nm diameter. They may be spherical, or may be an irregular shape or some other shape. The particles have a particle size between 30 and 1000nm or between 50 and 300nm diameter.

In another embodiment the process of providing the emulsion comprises the steps of:

- providing a basic emulsion, said emulsion comprising emulsion droplets dispersed in a continuous liquid phase,
- adding a first hydrolysable species to the emulsion;
- at least partially hydrolysing the first hydrolysable species within the emulsion droplets to form the condensable species;
- acidifying the emulsion to form an acidified emulsion; and
- adding a second hydrolysable species to the acidified emulsion.

The step of at least partially hydrolysing may comprise formation of nuclei. The basic emulsion may be a water-in-oil emulsion and may be a microemulsion.

The process may also comprise one or more of the steps of:

- separating the particulate substance from the continuous liquid phase,
- washing the particulate substance, and
- drying, and/or freeze drying, the particulate substance.

The releasable substance may be temporarily or releasably immobilised in and/or on the particulate substance. That is, the releasable substance may be immobilised on the particulate substance, but be capable of being at least partially released therefrom when subjected to appropriate release conditions, e.g. immersed in a liquid capable of releasing the releasable substance. The liquid may be for example a solvent for the releasable substance. The releasable substance may be an organic compound or an organometallic compound and may be a drug. It may be an anti-cancer drug for example doxorubicin. The releasable substance may be stable to the conditions pertaining in the emulsion droplets before and during the process.

The condensable species may be a silicate, and may be a soluble silicate for example sodium silicate or potassium silicate. The process may also comprise adding a metal oxide with the condensable species. The metal may be a transition metal for example titanium, zirconium, iron, zinc, vanadium, chromium or hafnium. Other oxides, such as those of tin, aluminium, germanium, calcium or phosphorous may also be used. The oxide may be added in a ratio to the condensable species of between about 0 and 80% on a molar or w/w basis, or between about 0 and 75%, 0 and 60%, 0 and 50%, 0 and 40%, 0 and 30%, 0 and 20%, 0 and 10%, 20 and 80%, 50 and 80%, 50 and 75%, 25 and 75% or 25 and 50%, and may be added in a ratio of about 0, 10, 20, 30, 40, 50, 60, 70 or 80% on a molar or w/w basis. Biodegradable particles and/or particles suitable for promoting apatite formation, e.g. for orthopaedic applications, may be made in this manner.

In another aspect of the invention there is provided a microporous particulate substance comprising a plurality of particles having a releasable substance releasably immobilised thereon and/or therein, wherein the particles of the particulate substance are between 30 and 1000nm or between 50 and 300nm in mean particle diameter and have micropores of less than 1nm diameter, together with mesopores of between 1 and 50nm, for example between 1 and 10nm. The particles of the particulate substance may comprise nuclei associated together. They may comprise agglomerates of nuclei. The nuclei may have a mean diameter of between 1 and 50nm. The particulate substance may be made by the process of the first aspect of the invention.

The releasable substance may be unstable in a basic environment, and may be stable in an acidic environment, and may be a drug, for example a drug for treatment of cancer. The releasable substance may be a fluorescent dye, a radiopharmaceutical, an enzyme, a hormone, a biocide or some other substance. The releasable substance may be releasable into water or an aqueous fluid or some other solvent. It may be releasable on exposure of the particulate substance to water or the aqueous fluid or other solvent, or on immersion of the particles in water or the aqueous fluid or other solvent, or on agitation of the particles in water or the aqueous fluid or other solvent. The releasable substance may be releasable without substantial degradation, or dissolution or erosion of the particles of the particulate substance. Over an extended period e.g. over about 6 months, some dissolution of the particles may take place. This may influence or contribute to the release profile. Release of the releasable substance may occur by diffusion out of particles of the particulate substance.

In a further aspect of the invention there is provided a method for treating a condition in a mammal, for example a human, comprising administering to the mammal a therapeutically effective quantity of a particulate substance according to the present invention, said particles comprising a releasable substance, said releasable substance being indicated for the condition. The releasable substance may be a drug, and the drug may be an anti-cancer drug. The condition may be a disease. The condition may be for example cancer, diabetes, hormonal dysfunction, hypertension, pain (for example pain treatable by morphine and/or opiates), or asthma.

There is also provided a particulate substance according to the present invention when used for the manufacture of a medicament for the treatment of a condition in a mammal, for example a human, said particulate substance comprising a releasable substance, said releasable substance being indicated for the condition. The condition may be for example cancer, diabetes, hormonal dysfunction, hypertension, pain (for example pain treatable by morphine and/or opiates), or asthma.

There is further provided the use of a particulate substance according to the invention for the treatment of a condition in a mammal, for example a human, said particles comprising a releasable substance, said releasable substance being indicated for the condition. The condition may be for example cancer, diabetes, hormonal dysfunction, hypertension, pain (for example pain treatable by morphine and/or opiates), or asthma.

In another aspect of the invention there is provided a method for delivering a releasable substance, said method comprising exposing a particulate substance according to the present invention to a medium capable of releasing said releasable substance, said particles comprising the releasable substance. The exposing may comprise immersing the particles in the medium, and may additionally comprise one or more of stirring, shaking, swirling or otherwise agitating the medium having the particles therein. Alternatively the exposing may comprise passing the medium past and/or through the particles. The medium may be a fluid, and may be a liquid. The medium may be a biological fluid such as blood. It may be an aqueous fluid, such as water or an aqueous solution. The medium may be capable of dissolving the releasable substance. The releasable substance may be for example a fluorescent dye, a radiopharmaceutical, a drug, an enzyme, a hormone, a biocide or some other substance, or it may be a mixture of any two or more of these. The exposing may be under conditions suitable for release of the releasable substance into the medium. The method may also comprise the step of allowing the releasable substance to release into the medium.

**Brief Description of the Drawings**

A preferred form of the present invention will now be described by way of example with reference to the accompanying drawings wherein:

Figure 1 shows a flow chart diagram of a process according to the present invention;
Figure 2 shows transmission electron micrographs (TEMs) illustrating the influence of the initial surfactant concentration [NP-5] on the final particle morphology: (a) 0.2 mol/L; (b) 0.4 mol/L; (c) 0.6 mol/L; (d) 0.8 mol/L;
Figure 3 shows TEMs illustrating the influence of initial TMOS (tetramethoxysilane) concentration on final particle size with a water/TMOS molar ratio = 10: a) TMOS 4 mmol, (b) TMOS 6 mmol, (c) TMOS 8 mmol, (d) TMOS 12 mmol;
Figure 4 shows TEMs illustrating the influence of the nature of the surfactant on the final particle size and morphology ([surfactant]=0.2 mol/L): (a) Brij30, (b) NP-5, (c) NP-6 (d) Triton X-114 with 1-pentanol 10 mmol (e) NP-9 with 1-pentanol 10 mmol (f) Triton X-100 with 1-pentanol 10 mmol;
Figure 5 shows TEMs illustrating the influence of the nature of the surfactant on the final particle size and morphology ([surfactant]=0.4 mol/L): (a) Brij30, (b) NP-5, (c) NP-6 (d) Triton X-114 with 1-pentanol 10 mmol (e) NP-9 with 1-pentanol 10 mmol (f) Triton X-100 with 1-pentanol 10 mmol;
Figure 6 shows TEMs illustrating the influence of cosurfactant on final particle size and morphology: (a) NP-5 0.2 mol/L; (b) NP-5 0.4 mol/L; (c) NP-5 0.2 mol/L, 1-pentanol 0.2 mol/L;
Figure 7 shows transmission electron micrographs illustrating the influence of micro-emulsion solvent on the final particle morphology (after destabilisation with 100ml acetone/100ml corresponding solvent): (a) petroleum benzene, 1-hexanol 20 mmol; (b) hexane, 1-hexanol 20 mmol; (c) octane, 1-hexanol 20 mmol; (d) decane, 1-hexanol 20 mmol; (e) dodecane, 1-hexanol 40 mmol; (f) toluene;
Figure 8 shows TEMs and a report of results, illustrating the influence of micro-emulsion solvent on final particle morphology (after destabilisation with 50ml ethanol/100ml corresponding solvent): (a) petroleum benzene, 1-hexanol 20 mmol; (b) hexane, 1-hexanol 20 mmol; (c) octane, 1-hexanol 20 mmol; (d) decane, 1-hexanol 20 mmol; (e) dodecane, 1-hexanol 40 mmol; (f) toluene;
Figure 9 shows TEMs illustrating destabilisation of toluene based micro-emulsions using:

(a) NP-5; (b) Triton X-114; (c) NP-9 as surfactant;

Figure 10 shows TEMs illustrating the influence of ageing time prior to destabilisation on the final particle morphology:

(a) ageing 24 h, (b) ageing 48 h, (c) ageing 120 h, (d) ageing 168 h;

Figure 11 shows TEMs illustrating the influence of the nature of the silicon alkoxide on initial seed and final particle size and morphology: (a) TMOS 6 mmol before destabilisation; (b) TMOS 6 mmol after destabilisation (c) TEOS 6 mmol before destabilisation; (d) TEOS 6 mmol after destabilisation;

Figure 12 shows TEMs illustrating the influence of the way the destabilisation agent is added on final particle size, with particles obtained: (a) by pouring 50 mL acetone into microemulsion; (b) by pouring the microemulsion into 50 mL acetone; (c) by pouring the microemulsion into 100 mL acetone; (d) by pouring the microemulsion into a mixture of 100 mL acetone and 100 mL cyclohexane;

Figure 13 shows TEMs illustrating the influence of the quantity of acetone added on final particle size (a) 50 mL acetone plus 50 mL cyclohexane: (b) 100 mL acetone plus 100 mL cyclohexane; (c) 150 mL acetone plus 150 mL cyclohexane; (d) 200 mL acetone plus 200 mL cyclohexane;

Figure 14 shows TEMs illustrating the influence of mixing conditions on final particle size and morphology: (a) 1 L beaker, '/' bar; (b) 250 mL beaker, '+' bar; (c) 250 mL beaker, '/' bar; (d) ultrasonication; (e) water pool NaCl 0.2 mol/L, 250 mL beaker, '/' bar;

Figure 15 shows TEMs illustrating the influence of pH of water pools (discontinuous phase) on final particle size and morphology: (a) pH=7.0; (b) 3.54; (c) 3.04 (d) 2.70; (e) 1.80; (f) 1.50;

Figure 16 shows TEMs and reports of results illustrating the influence of the quantity of acetone on final particle morphology: (a) 10 mL; (b) 20 mL; (c) 50 mL; (d) 100 mL; (e) 200 mL;

Figure 17 shows TEMs and reports of results illustrating the influence of the quantity of ethanol on final particle morphology: (a) 5 mL; (b) 10 mL; (c) 20 mL; (d) 35 mL; (e) 50 mL; (f) 80 mL; (g) 100 mL;

Figure 18 shows TEMs and reports of results illustrating the influence of the composition of a mixed ethanol/acetone destabilising mixture on final particle morphology: a) 100 mL cyclohexane, 80 mL acetone, 20 mL ethanol b) 100 mL cyclohexane, 60 mL acetone, 40 mL ethanol c) 100 mL cyclohexane, 40 mL acetone, 60 mL ethanol d) 100 mL cyclohexane, 20 mL acetone, 80 mL ethanol;

Figure 19 shows TEMs illustrating the influence of the nature of the coalescing liquid on final particle morphology; a) isopropanol, b) 1-propanol, c) methyl ethyl ketone, d) chloroform, e) toluene, f) benzene, g) THF, h) DMF, i)pyridine, and j) 1-butanol;

Figure 20 shows TEMs illustrating the influence of composition of the coalescing liquid added to the micro-emulsions on the final particle morphology: cyclohexane mixed with (a) 40 mL acetone + 10 mL 1-propanol, b) 30 mL acetone + 20 mL 1-propanol, c) 20 mL acetone + 30 mL 1-propanol, d) 10 mL acetone + 40 mL 1-propanol;

Figure 21 shows a graph of Doxorubicin encapsulation efficiency as a function of doxorubicin added;

Figure 22 shows a graph illustrating the decomposition of pure Doxorubicin in PBS (pH 6.9/25 °C) at 37 °C;

Figure 23 shows a graph of the short-term release rate of doxorubicin from silica nanoparticles according to the present invention, in PBS (pH 6.9/25 °C) at 37 °C;

Figure-24 shows a graph of the long term release of doxorubicin from silica nanoparticles according to the present invention, in PBS (pH 6.9/25 °C) at 37 °C;

Figure 25 shows graphs of nitrogen adsorption-desorption isotherms of silica particles produced by acetone destabilisation of a microemulsion system according to the invention, NP-5/cyclohexane/ water at pH=1 and pH=7;

Figure 26 is a schematic illustration of different particle growth processes in microemulsions;

Figure 27 is flow chart showing typical particle synthesis from the additional experiments described herein;

Figure 28 shows TEMs illustrating the influence of the nature of the organically modified precursor on the particles morphology in the additional experiments described herein: (a) MTMS; (b) PTMS; (c) OTES; (d) GTES; (e) CheeTES; (f) APTMS; (ORMOCER (25 mol%) with TMOS (75 mol%) and [N-57] = 0.4 mol/L);

Figure 29 a TEM illustrating the influence of APTMS on the particles morphology in the additional experiments described herein (APTMS (25 mol%) with TMOS (75 mol%) and [N-57] = 0.4 mol/L)

Figure 30 shows transmission electron micrographs illustrating the influence of the nature and proportion of ORMOCER in the additional experiments described herein: (a) GTMS 5 mol% plus TMOS 95 mol%; (b) GTMS 10 mol% plus TMOS 90 mol%; (c) GTMS 15 mol% plus TMOS 85 mol%; (d) APTMS 5 mol% plus TMOS 95 mol%; (e) APTMS 10 mol% plus TMOS 90 mol%; (f) APTMS 15 mol% plus TMOS 85 mol%;

Figure 31 shows transmission electron micrographs illustrating the influence of the proportion of TMOS and TEOS on the particle morphology in the additional experiments described herein: (a) TEOS 25 mol% plus TMOS 75 mol%; (b) TEOS 50 mol% plus TMOS 50 mol%; (c) TEOS 75 mol% plus TMOS 25 mol%;

Figure 32 is flow chart showing a process for destabilisation of a multiple emulsion system as described in the additional experiments described herein;

Figure 33 shows UV-visible drift spectra of submicron powders synthesised by destabilisation of micro-emulsion and with different dyes encapsulated: (A) rhodamine-B; (B) methyl-violet; (c) two dye doped by mixing two emulsions; (D) two dye mixed before adding to emulsion;

Figure 34 shows transmission electron micrographs illustrating destabilisation of mixed seed and oligomeric system

in the additional experiments described herein: (a) seed particles synthesized in base; b) with the addition of 1.269 mL1.00M $HNO_3$, 0.024 mmol $F^-$ and 1.2 mmol of TEOS followed by destabilisation; c) with the addition of 1.269 mL of 1.50M $HNO_3$, 0.024 mmol $F^-$ and 1.2 mmol of TEOS followed by destabilisation; d) with the addition of 1.269 mL of 1.00M $HNO_3$, 0.024 mmol $F^-$ and 1.2 mmol of TMOS followed by destabilisation; e) with the addition of 1.269 mL of 1.50M $HNO_3$, 0.024 mmol $F^-$ and 1.2 mmol of TMOS followed by destabilisation;

Figure 35 is a flowchart showing a process for destabilisation of an emulsion containing both nanoparticles synthesised using base catalysis (i.e. seeds) and monomer hydrolysed in acid media;

Figure 36 shows transmission? electron micrographs illustrating destabilisation of micro-emulsions containing hybrid seed particles in the additional experiments described herein, in which the seeds are made from: (a): TMOS 100%; (b): TMOS 75% + VTMS 25%; (c):TMOS 75% + MTMS 25%; (d):TMOS 75% + PTMS 25%; (e):TMOS 75% + OTES 25%; (f):TMOS 75% + APTMS 25%; (g):TMOS 75% + DATMS 25%; (h):TMOS 75% + MPTMS 25%;

Figure 37 shows transmission? electron micrographs illustrating destabilisation of multiple emulsions in the additional experiments described herein using 100 ml of cyclohexane and 100ml of (a) acetone; (b) ethanol; (c) iso-propanol; (d) 1-propanol;

Figure 38 shows a graph of pore size distribution of sample LK-425 and LK 428 corresponding to isotherm in figure 25;

Figure 39 shows graphs of particle size distribution of samples synthesized without sonication during destabilisation;

Figure 40 shows graphs of particle size distribution of samples synthesized with sonication during destabilisation;

Figure 41 shows a graph illustrating the release profiles of particles containing doxorubicin at pH < 4 at 37 °C);

Figure 42 shows a comparison of release of doxorubicin, and formation of degradation products;

Figure 43 shows a graph illustrating the release of doxorubicin from nanoparticles at pH=7.4;

Figure 44 shows a comparison of the decomposition of a sample of doxorubicin at pH < 4 and pH 7.4;

Figure 45 shows a graph illustrating the total quantity of doxorubicin released from microparticles according to the invention;

Figure 46 shows a graph illustrating the cumulative release of doxorubicin from nanoparticles according to the invention;

Figure 47 shows a graph illustrating the continuous release of Camptothecin from nanoparticles according to the invention;

Figure 48 shows a graph illustrating the continuous release of Camptothecin from nanoparticles according to the invention, in which the aqueous phase was replaced at each data point; and

Fig. 49 shows TEMs illustrating the influence of the nature of the surfactant on particle size morphology prior to and after destabilisation by acetone ([surfactant] = 0.2 mol/L). a) Tween 21 ; b) Tween 61; c) Tween 81; d) AOT

**Detailed Description of the Preferred Embodiments**

The present invention describes a process for making particles of a desired size. In one aspect, the process comprises initially producing an emulsion, for example a microemulsion, with well-controlled particle size through well-known processes, infusing a hydrolysable species into the emulsion droplets of the emulsion, hydrolysing the hydrolysable species in the emulsion droplets to form a condensable species and condensing the condensable species within the emulsion droplets using well-known sol-gel chemistry. The resulting particles are then coalesced by initiating a controlled destabilisation of the emulsion to produce a particulate substance with the desired particle size. The chemistry required for making particles having controlled pore sizes is known, and by combining that technology with the present process for controlled destabilisation, it is possible to produce a particulate substance comprising particles of a desired mean particle size with controlled pore size. Particular combinations of particle size and pore size are thus achievable that were hitherto difficult to produce. If a releasable substance such as a drug is incorporated into the emulsion droplets of the initial microemulsion, then that releasable substance may be releasably immobilised in and/or on the particulate substance, which may then (if the releasable substance is a drug) be used for therapeutic purposes. By appropriate control of the particle size, the particulate substance may be targeted at particular parts of a patient's body, and by appropriate control of the pore size, the release rate of the releasable substance may be controlled to a desired rate.

The process of the present invention may be used to make a microporous particulate substance which comprises a releasable substance for treatment of conditions such as cancer in a mammal, for example a human. The releasable substance is releasably immobilised on and/or in the microporous particulate substance. The microporous particulate substance comprises particles that are between 30 and 1000nm in diameter, or may be between 30 and 500 or 30 and 100 or about 50 and 1000 or about 100 and 1000 or about 500 and 1000 or about 50 and 500 or about 50 and 300 or about 50 and 250 or about 100 and 400 or about 100 and 300 or about 100 and 250 or about 150 and 250nm, and may be about 30, 40, 50, 60, 70, 80, 90, 100, 150, 200, 250, 300, 350, 400, 450, 500, 600, 700, 800, 900 or 1000nm in diameter. The particles of the particulate substance are microporous (that is they may have a pore diameter of less than about 1.7nm) and mesoporous. They have mesopores between about 1 and about 50nm in diameter and micropores below about 1nm in diameter. The pore size may be tailored by adjusting the conditions under which the particles of the

invention are made. In the process of the present invention, these particles are produced from nuclei. The nuclei may be primary particles. The nuclei may have a mean particle diameter about 1 and 50nm, or between about 1 and 20 or about 1 and 10 or about 1 and 5 or about 1 and 2 or about 2 and 50 or about 5 and 50 or about 10 and 50 or about 20 and 50 or about 2 and 20 or about 2 and 10 or about 5 and 10nm, and may have a mean particle diameter of about 1, 2, 3, 4, 5, 6, 7, 8, 9, 10, 12, 14, 16, 18, 20, 25, 30, 35, 40, 45 or 50nm The weight of the releasable substance may depend on the nature of the drug and the nature of the particulate substance, and may be between about 0.01 and 100mg per gram of particulate substance, or between about 0.01 and 20mg or 0.01 and 10mg or 0.01 and 5mg or about 0.01 and 1mg or about 0.01 and 0.5mg or about 0.01 and 0.1mg or about 0.01 and 0.05mg or about 1 and 100mg or about 10 and 100mg or about 50 and 100mg or about 1 and 10mg or about 5 and 10mg or about 0.1 and 1mg or about 0.1 and 0.5mg per gram of particulate substance, and may be about 0.01, 0.05, 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95 or 100mg per gram of particulate substance, or may be less than 0.01 or greater than 100mg per gram of particulate substance.

The process of the invention comprises providing a precursor emulsion having a condensable species in the emulsion droplets, wherein the emulsion droplets comprise the discontinuous phase of the emulsion. The emulsion may be a w/o microemulsion, so that the condensable species is located in the aqueous emulsion droplets ("water pools"). The emulsion may be made by:

- providing an emulsion comprising emulsion droplets dispersed in a continuous liquid phase,
- infusing a hydrolysable species into the emulsion droplets (i.e. the discontinuous phase) of the emulsion, and
- hydrolysing the hydrolysable species within the emulsion droplets to form the condensable species.

Alternatively the condensable species may be infused into the emulsion droplets. The step of providing an emulsion may comprise combining a hydrophobic liquid, an aqueous liquid and a surfactant in quantities and under conditions of agitation suitable for producing an emulsion. The surfactant may be for example NP-5 and NP-6 or Tween 21, or a mixture of any two or three of these. The agitation may comprise one or more of stirring, shaking, sonification or ultrasonification, and may be mild, moderate or vigorous. The aqueous liquid may be an aqueous solution, and may comprise components that are required in the discontinuous phase of the emulsion. The aqueous liquid may comprise a catalyst for hydrolysis of the hydrolysable species and optionally a catalyst for condensation of the condensable species. The catalyst may be for example fluoride, base (i.e. hydroxide ion, which may be from an alkali metal hydroxide or ammonia), or a transition metal alkoxide (e.g. titanium isopropoxide) which may catalyse hydrolysis of an alkoxysilane. The aqueous liquid may comprise a releasable substance, which may be immobilised in and/or on the particulate substance made by the process of the invention. The releasable substance may be a drug, and may be an anti-cancer drug, and may be doxorubicin, or it may be a fluorescent dye, a radiopharmaceutical, an enzyme, a hormone, a biocide or some other substance. The aqueous liquid may be at a pH that does not promote decomposition of the releasable substance. The pH may be between about 1 and 8 or between 1 and 7 or between about 2 and 7, between about 3 and 7, between about 4 and 7, between about 5 and 7, between about 6 and 7, between about 1 and 6, between about 2 and 6, between about 3 and 6, between about 4 and 6, between about 5 and 6, between about 1 and 5, between about 2 and 5, between about 3 and 5, between about 4 and 5, between about 1 and 4, between about 2 and 4, between about 3 and 4, between about 1 and 3, between about 2 and 3 or between about 1 and 2 or between about 7 and 8, and may be about 1, 1.5, 2, 2.5, 3, 3.5, 4, 4.5, 5, 5.5, 6, 6.5, 7, 7.5 or 8. The hydrolysable species may be a species that is capable of being hydrolysed to produce a condensable species. It may be for example a hydrolysable silane. It may comprise a hydrolysable silane having 1, 2, 3 or 4 hydrolysable groups attached to silicon, and may comprise a mixture of such silanes, or a mixture of such silanes. Suitable silanes include, but are not restricted to tri- and tetra-alkoxysilanes such as tetramethoxysilane (TMOS), tetraethoxysilane (TEOS), tetrabutoxysilane (TBOS), tetrapropoxysilane (TPOS), methyltrimethoxysilane (MTMS), methyltriethoxysilane (MTES), ethyltriethoxysilane (ETES), octyltriethoxysilane (OTES), octyltrimethoxysilane (OTMS), hexadecyltrimethoxisilane (HDTMS) and hexadecyltriethoxisilane (HDTES), octadecyltrimethoxysilane (ODTMS), octadecyltriethoxyisilane (ODTES) as well as methyl polysilicate (MPS), ethyl polysilicate (EPS), polydiethoxysilane (PDES), hexamethyl disilicate, hexaethyl disilicate or functional trialkoxysilanes (eg methacryloyloxypropyltrimethoxysilane, phenyltriethoxysilane (PTES), phenyltrimethoxysilane (PTMS), glycidoxypropoxyltrimethoxysilane (GLYMO), glycidoxypropyltriethoxysilane (GLYEO), mercaptopropyltriethoxysilane (MPTES), mercaptopropyltrimethoxysilane (MPTMS), aminopropyltrimethoxysilane (APTMS), aminopropyltriethoxysilane (APTES), 3-(2-aminoethylamino)propyltrimethoxysilane (DATMS), 3-[2-(2-aminoethylamino)ethylamino]propyltrimethoxysilane (TATMS), [2-(cyclohexenyl)ethyl]triethoxysilane (CHEETES), vinyltrimethoxysilane (VTMS), vinyltriethoxysilane (VTES) or a mixture of any two or more of the above. The condensable species may comprise at least 80% tetraalkoxysilane, or at least 85, 90 or 95% tetraalkoxysilane, and may comprise about 80, 85, 90, 95, 96, 97, 98, 99 or 100% tetraalkoxysilane. It may comprise less than about 20% trialkoxysilane, for example less than about 15, 10 or 5% trialkoxylsilane, and may comprise about 20, 15, 10, 5, 4, 3, 2, 1 or 0% trialkoxysilane. Some trialkoxysilanes may only be usable in the process in low concentration. The hydrolysable species may be capable of hydrolysing to produce a condensable species. The

condensable species may be a silanol species, and may have 1, 2, 3 or 4 silanol groups per molecule. It may be an at least partially condensed material having 1 or more silanol groups per molecule. It may be a mixture of silanol species.

The hydrolysable species may be added to an emulsion, and may infuse into the emulsion droplets. Commonly a material in an emulsion may partition between the two phases of the emulsion, the partitioning being dependent on the relative affinities of the material for the two phases. Thus a highly hydrophilic material will be predominantly located in the aqueous phase, whereas a highly hydrophobic material will be predominantly in the hydrophobic phase. If the material partitions into the aqueous phase, and reacts there, then further material may partition into the aqueous phase. Addition of a hydrolysable species to the continuous (hydrophobic) phase may lead to partitioning of the hydrolysable species into the aqueous phase (the emulsion droplets), where conditions may pertain which promote hydrolysis of the hydrolysable species to form the condensable species, and subsequent formation of nuclei. The formation of nuclei may comprise at least partial condensation of the condensable species. The addition of the condensable species may or may not be accompanied by at least some agitation or swirling. Brownian motion may provide sufficient energy for mixing without externally applied mixing.

The process of the present invention may comprise hydrolysing the hydrolysable species and condensation of the resulting condensable species to form nuclei within the emulsion droplets. If the hydrolysable species is an alkoxysilane, and the emulsion droplets comprise an acidic fluoride solution, then the step may comprise allowing sufficient time for the alkoxysilane to be hydrolysed and for the resulting condensable species to condense to form nuclei.

Alternatively, nuclei and/or primary particles may be formed under basic conditions and then acidified, and the resulting emulsion may be destabilised to form the particulate substance.

Alternatively the nuclei and/or primary particles may be preformed. For example the nuclei may be particles of colloidal or fumed silica, of some other colloidal material or may be some other particles of appropriate size. The condensable species may be capable of forming the particles of the particulate substance from the nuclei, for example by agglomerating the nuclei by condensing in the presence of the nuclei. The condensable species may be compatible with the nuclei and may be capable of reacting with the nuclei.

In one embodiment of the invention, the process of providing the emulsion comprises the steps of:

- providing a basic emulsion, said emulsion comprising emulsion droplets dispersed in a continuous liquid phase,
- adding a first hydrolysable species to the emulsion;
- at least partially hydrolysing the first hydrolysable species within the emulsion droplets to form the condensable species;
- acidifying the emulsion to form an acidified emulsion; and
- adding a second hydrolysable species to the acidified emulsion.

The basic emulsion may be a water in oil emulsion and may be a microemulsion. It may comprise a surfactant, e.g. NP9, and may additionally comprise a cosurfactant, e.g. 1-pentanol. The continuous liquid may be a hydrocarbon e.g. cyclohexane. The first and second hydrolysable species may comprise alkoxysilanes, for example tetraalkoxysilanes, as described elsewhere herein. They may be the same or may be different. The pH of the basic emulsion may be between about 8 and 13, or between about 8 and 10, 8 and 9, 9 and 13, 11 and 13 or 9 and 11, and may be about 8, 8.5, 9, 9.5, 10, 10.5, 11, 11.5, 12, 12.5 or 13 or may be greater than 13. After addition of the first hydrolysable species, the emulsion may be aged for sufficient time to at least partially hydrolysing the first hydrolysable species within the emulsion droplets to form the condensable species, and to form nuclei, for example primary particles. The time of ageing may be between about 5 and 100 hours, or between about 5 and 80, 5 and 60, 5 and 40, 5 and 20, 10 and 100, 20 and 100, 50 and 100, 10 and 50, 20 and 5 or 40 and 50 hours, and may be about 6, 12, 18, 24, 30, 36, 42, 43, 44, 45, 46, 47, 48, 49, 50, 51, 52, 53, 54, 60, 66, 72, 78, 84, 92 or 96 hours. The time may depend on the temperature of ageing, which may be between about 15 and 95°C or some other temperature, for example room temperature or ambient temperature. The temperature may be for example between about 20 and 80, 50 and 80, 10 and 50 or 30 and 70°C, and may be about 15, 20, 25, 30, 35, 40, 45, 50, 55, 60, 65, 70, 75, 80, 85 or 90°C. After acidification of the emulsion, the acidified emulsion may be stirred or aged for at least about 1 hour, or at least about 2, 3, 4, 5, 6, 7, 8, 9 or 10 hours, for example between about 1 and 10 hours or between about 1 and 8, 1 and 6, 1 and 4, 2 and 10, 5 and 10, 2 and 8 or 4 and 6 hours, e.g. 1, 2, 3, 4, 5, 6, 7, 8, 9 or 10 hours. The ageing may be at between about 30 and 90°C, or between about 30 and 70, 30 and 50, 50 and 90, 70 and 90, 40 and 80 or 30 and 70°C, for example about 30, 35, 40, 45, 50, 55, 60, 65, 70, 75, 80, 85 or 90°C. The addition of the second hydrolysable species may be conducted during or ageing this latter step of stirring or ageing. Before or concurrently with addition of the second hydrolysable species, a surfactant, optionally the same as the surfactant used in making the basic emulsion, together with a solvent and optionally a cosurfactant (which may be the same as or different to that used in the basic emulsion), may be added. The solvent may be miscible with, and may be the same as, the continuous liquid phase. The ratios of solvent, surfactant and cosurfactant (if present) may be the same as or different to the ratios in the basic emulsion. After addition of the second hydrolysable species, the acidified emulsion may be aged for sufficient time to at least partially hydrolyse the second hydrolysable species. This may be at least

about 24 hours, or at least about 30, 36, 42, 48, 54 or 60 hours, may be between about 24 and 60 hours, or between about 24 and 48, 48 and 60, 36 and 54 or 40 and 50 hours, and may be about 30, 36, 42, 48, 54 or 60 hours.

Following the step of forming the nuclei, the processes of the invention may comprise the step of combining a coalescing liquid with the emulsion to form the particulate substance. The step of combining may comprise adding the emulsion to a coalescing liquid, or may comprise adding the coalescing liquid to the emulsion. The adding may comprising dropping, pouring or otherwise adding, and may be accompanied by agitation, swirling, mixing, stirring etc. and may be accomplished rapidly or slowly. The addition may be at a rate of between about 1 and 1000ml/min, or between about 1 and 500, 1 and 200, 1 and 100, 1 and 50, 100 and 1000, 500 and 1000, 10 and 500 or 100 and 500ml/minute, and may be at a rate of about 1, 2, 5, 10, 25, 50, 100, 200, 300, 400, 500, 600, 700, 800, 900 or 1000ml/minute, or may be at some other rate. Following the addition, further time may be allowed for coalescence of the nuclei to proceed. The further time may be up to about 10 hours, or up to about 5, 2 or 1 hour, or up to about 30, 20, 10, 5, 2, 1 or 0.5 minutes, and may be about 0, 0.5, 1, 2, 3, 4, 5, 10, 15, 20, 25, 30, 40 or 50 minutes, or about 1, 2, 3, 4, 5, 6, 7, 8, 9 or 10 hours. The coalescing liquid may be a liquid that is miscible with both the continuous phase and the emulsion droplets. It may comprise a polar liquid, and may be for example acetone or ethanol. It may comprise a mixture of liquids. It may for example comprise a mixture of a destabilising liquid, such as a polar liquid, and a non-polar liquid. The non-polar liquid may be the same as the continuous phase of the emulsion, or it may be different. For example the coalescing liquid may be a mixture of acetone and cyclohexane or a mixture of ethanol and cyclohexane. The ratio of destabilising liquid to non-polar liquid may be between about 1:3 and 3:1 w/w or v/v, and may be between about 1:3 and 2:1, 1:3 and 1:1, 1:3 and 1:2, 1:2 and 3:1, 1:1 and 3:1 or 2:1 and 3:1, and may be about 3:1, 2:1, 1:1, 1:2 or 1:3 w/w or v/v. The amount of coalescing liquid may be sufficient to cause formation of the particulate substance. It may be sufficient to cause coalescence of the nuclei. The coalescing liquid may be in a quantity that does not lead to formation of a gel. The amount of coalescing liquid may be between about 2 and 6ml per ml of emulsion, and may be between about 3 and 5 or 2 and 4 or 4 and 6ml per ml of emulsion, and may be about 2, 2.5, 3, 3.5, 4, 4.5, 5, 5.5 or 6ml per ml of emulsion, and may be some other amount depending on the nature of the continuous phase and the coalescing liquid.

The process may also comprise one or more of the steps of:

- separating the particulate substance from the continuous liquid phase,
- washing the particulate substance, and
- drying, and/or freeze drying, the particulate substance.

The separating may comprise one or more of settling, centrifuging, decanting, ultracentrifuging, filtering, microfiltering or some other suitable separation process. The washing may comprise exposing the particulate substance to a suitable solvent, optionally agitating the solvent, and then separating the solvent from the particulate substance. It may comprise suspending the particulate substance in the solvent, or it may comprise passing the solvent through the particulate substance. The washing may at least partially remove surfactant from the particulate substance. The solvent may be a solvent for the surfactant. The solvent for particle washing may depend on the solubility and molecular size of dopant. If the dopant is highly soluble in polar solvents such as acetone or less polar solvents such as chloroform, non-polar solvents may used for particle washing. As the HLB of the surfactant is commonly around 10, the surfactant is generally soluble in either polar or non-polar solvents. The drying may comprise exposing the particulate substance to a gas, for example air, nitrogen, carbon dioxide or mixtures thereof. The gas may be a dry gas, and may be dried before use. The exposing may comprise passing the gas over or through the particulate substance, and may comprise sucking the gas through the particulate substance. Alternatively the drying may comprise exposing the particulate substance to a partial vacuum. The partial vacuum may have an absolute pressure of less than about 0.5 bar, or less than about 0.2, 0.1, 0.05, 0.01, 0.005 or 0.001 bar, and may have an absolute pressure of about 0.5, 0.4, 0.3, 0.2, 0.1, 0.05, 0.02, 0.01, 0.005, 0.002 or 0.001bar. The drying may be conducted at a temperature below the degradation temperature of the releasable substance, and may therefore depend on the nature of the releasable substance. The drying may be conducted for example at less than about 100°C, or less than about 90, 80, 70, 60, 50, 40, 30 or 20°C, and may be conducted at about 20, 30, 40, 50, 60, 70, 80, 90 or 100°C or at some other suitable temperature.

Releasable substance that has not been incorporated into particles may be recovered from the coalescing liquid and reused if required.

An example of a process for producing the particulate substance is summarized in Figure 1 and may be described as follows:

1. A stable microemulsion is prepared by mixing a surfactant, a non-polar solvent, an aqueous solution containing the releasable substance (e.g. drug) and a catalyst for the sol-gel reaction (e.g. acid, $F^-$).
2. The hydrolysable species (e.g. TMOS: tetramethoxysilane) is then added to the microemulsion and is allowed to slowly diffuse to the water pools (i.e. core of the emulsion droplets) where it undergoes hydrolysis leading to the formation of nuclei, e.g. solid primary particles.

3. After formation of the nuclei, a polar solvent (e.g. acetone), optionally mixed with a non-polar solvent (e.g. cyclohexane), is combined with the mixture to destabilise the emulsion and coalesce the solid nuclei, thus producing larger particles, which precipitate out of the continuous phase. In this step, the solvent(s) may be added to the mixture, or the mixture may be added to the solvent(s).

4. The particles are then washed, and freeze-dried.

The emulsions parameters (surfactant/water ratio, TMOS/water ratio) as well as the nature, the volume and the way the coalescing liquid is added may control the final particle size and distribution.

The growth of solid precursor particles within the droplets of an emulsion is governed by the same mechanisms as in a colloidal suspension: nucleation and growth by either ripening aggregation or coagulation.

As in colloidal suspension, nucleation in water-in-oil emulsions takes place when the concentration of condensable species present in the droplets (the discontinuous phase) exceeds the nucleation threshold [Cn]. The only difference between this and classical colloidal suspension is that, in emulsions, the solution is compartmentalised in small droplets. Thus, the concentration of condensable species may change from water pool to water pool (ie from droplet to droplet). In other words, in emulsions the number of molecules of condensable species increases with their average supersaturation until the average concentration per pool is larger than [Cn].

Based on these considerations, the number of nuclei increases with increasing concentration of condensable species and decreases with the number of droplets. Since the number of molecules of condensable species is proportional to the amount of free water available per micelle, the number of nuclei increases with increasing free water content. In other words the number of nuclei increases with the water to surfactant ratio. (Free water may be contrasted with bound water i.e. the water solvating the surfactant hydrophilic head and which does not participate in the hydrolysis of the hydrolysable species, e.g.silicon alkoxide. The amount of free water depends on the nature of the surfactant (size and nature of the hydrophilic head) and the water to surfactant ratio.)

Another specific characteristic of emulsions is that the emulsion droplets are capable of exchanging their cores during collision. An increase in the rate of inter-micellar exchange can induce a redistribution of the condensable species before the supersaturation reaches the nucleation threshold. Thus increasing the collision of micelles can lead to a decrease in nucleation rate.

As with aqueous colloidal suspensions, growth of solid particles in emulsions follows nucleation. To a first approximation, the water pools (droplets of discontinuous phase) may be viewed as micro-reactors in which particle growth takes place by addition of condensable species to the nuclei. Thus, the larger the number of nuclei, the smaller the nuclei. Similarly, for a fixed number of nuclei, the higher the supersaturation in the water pool, the larger the resulting nuclei.

As mentioned above, emulsions are dynamic systems and the emulsion droplets collide constantly with one another, exchanging the content of their aqueous core in the process. Growth of the droplets may then take place by collision of pools containing solid nuclei or nuclei with under-saturated pools containing only condensable species. In other words, growth can take place by consumption of unsaturated micelles (in a process akin to coalescence). Thus the final particle size of the droplets increases with the number of collisions or micellar exchanges.

Base catalysis of sol-gel reactions promotes both hydrolysis/condensation and, more importantly, dissolution. When combined with micro-emulsions, base catalysed sol-gel chemistry leads to a rapid nucleation due to the high hydrolysis rate. The rapid condensation leads to a rapid consumption of all the condensable species inside the micelles and further growth takes place by ageing and scavenging of other micellar pools. High dissolution rates ensure the production of spherical particles by ripening of the aggregates formed during the collision of droplets containing nuclei (see Figure 26). Thus with reference to Figure 26, once nuclei have formed within the droplets of the emulsion, base catalysis leads to a rapid hydrolysis and growth of particles, whereas acid catalysis does not promote growth. If the emulsion is destabilised in a controlled fashion as described in the present invention, polar channels are formed, leading to colloidal growth to form nanoporous particles in the range of about 30 to 1000nm diameter, or about 50 to 500nm.

Acid catalysis promotes hydrolysis but hinders both condensation and dissolution (except at very low pH i.e. pH $\leq$ 0). Consequently, for the emulsion synthesis of silica particles using acid catalysis, many nuclei are generated but very little growth is observed. In fact, no solid particles were observed in acid catalysed emulsions even after 48h ageing. A condensation catalyst such as fluoride must be introduced to produce solid particles. Even in this case, nuclei remain very small (i.e. about 10 nm) as revealed by direct TEM analysis of the emulsions prior to destabilisation (see Figure 11). At this stage it is likely that the nuclei are soft aggregates of linear polymer. They appear to be solid when observed by TEM due to the drying intrinsic to sample preparation. This suggests that, in contrast to base catalysis, no coalescence takes place between the droplets during inter-micellar collisions. The particles grow to fill the droplet core by consuming slowly all the condensable species present in its pool. This is confirmed by Small Angle Neutron Scattering SANS (results are summarised in Table 1), which shows that the particle size after 5 days ageing remains similar to the initial particle size.

Table-1: SANS data

| | Fitted diameter (nm) | Core scattering length density, $\rho$ ($\times 10^{-6}$ A$^{-2}$) |
|---|---|---|
| Micelle | 9.0 | 0.28 |
| Age 1 day | 9.4 | 0.49 |
| Age 5 days | 10.0 | 0.50 |
| Addition of acetone | 19.0 | 0.98 |

The basis of the present invention is the destabilisation of an emulsion using a polar liquid as a coalescing liquid, or as a component thereof, to induce coalescence of the aqueous emulsion droplets and thus initiate growth and aggregation of the nuclei.

Addition of water (a highly polar solvent) is known to swell emulsion droplets and to produce larger particles. This works up to a certain amount of water after which multiphase domains are formed (e.g. water and emulsion phase). In the case of acetone, SANS experiments reveal that addition of a small quantity of acetone leads to a significant swelling of the droplet cores (see Table 1). This suggests that acetone does induce coalescence of the small droplets into larger droplets. In contrast to water, addition of a larger quantity of acetone, does not lead to the formation of two distinct phases (e.g. water and reverse emulsion). In fact what is observed when the destabilisation is successful is the slow precipitation of particles out of this single-phase (i.e. acetone + cyclohexane + water+ surfactant) system.

The destabilisation of the emulsion system appears to go through the following steps:

- Step 1: when acetone is added, the droplets start to swell.
- Step 2: as more acetone is added, open channels are created between the cores of the droplets forming an associated lamella-like phase or bicontinuous structure. This phenomenon has been observed for NP5/water/heptane solution at high water to surfactant ratio (C-L Chang, HS Fogler, Langmuir 1997, 13, 3295-3307).
- Step 3: Further addition of acetone leads to the swelling of the channels and ultimately the destruction of the emulsion structure, the system reverting to a single-phase of mixed solvents (cyclohexane + water + acetone) where the surfactant is molecularly dispersed as in a true solution. The swelling of the aqueous channel allows the nuclei and other condensable species to react freely, forming particles by classical colloidal growth (i.e. addition of hydrolysed monomer to the nuclei).

It is important to note that this destabilisation process is also a kinetic process with a competition between the emulsion droplet coalescence rate (and the resulting formation of open channels) with the sol-gel reaction rate (i.e. silica production rate).

Using the mechanistic model outlined above, it is possible to rationalise the influence of the different processing parameters on the destabilisation of the emulsion and the final particle morphology.

After addition of the coalescing liquid, four different types of behaviour may be observed:

- The suspension remains clear. No particles are formed and the destabilisation is ineffective.
- The suspension remains initially clear and a gel forms very slowly. The final gel appears as a sponge-like structure, in contrast to colloidal gels, which appear as glued strings of particles. A very slow destabilisation leads to slow aggregation of nuclei of about 10nm diameter in a 3 dimensional sponge like gel structure.
- The suspension forms a gel immediately. The final gel is colloidal, i.e. particles fused together in a 3 dimensional network. The partial destabilisation of the emulsions leads to the formation of the water channels, which induces some particles growth but the destabilisation is not "strong" or rapid enough and the partially grown particles aggregate together.
- The suspension forms submicron-size particles with a mean particle size of between about 30 and 1000nm, or about 50 and 300nm or about 150 and 500nm. The channels have been swollen (or even destroyed forming one single phase) thus releasing the micellar cores and enabling their contents of condensable species to participate in particle growth.

Using these mechanisms it is possible to postulate explanations of the influence of different processing parameters.

Increasing the surfactant concentration is known to increase the stability of micro-emulsions. Correspondingly, for a fixed quantity of coalescing liquid (e.g. acetone), emulsions with increasing surfactant concentrations are more difficult to destabilise and result in slow gelation of nuclei (see pictures c and d in Figure 2).

As expected, an increase in the amount of silicon precursor leads to an increase in particle size. At acid pH the rate of hydrolysis of the alkoxide is high and the rate of condensation is low, which means that the number of monomer molecules is high and the number of nuclei remains low. As the amount of silicon precursor is increased, the number of hydrolysed precursor molecules increases significantly more than the number of new nuclei. When destabilisation occurs this leads to a higher proportion of monomer to nuclei and thus larger particles.

Apart from the case of Triton X-100, which forms particles prior to destabilisation, two surfactants that produced submicron particles were NP5 and NP6, which have intermediate HLB's around 10. An HLB of 10 indicates a strong amphiphile nature (i.e. balanced hydrophilicity and hydrophobicity) and denotes a medium strength molecular interaction between the surfactant polar head and water from the droplet. Such a medium interaction leads at high water content to the coalescence of the droplets and the formation of open channels as surfactant with weaker or stronger interaction with water remain as dispersed droplets. This reinforces the importance of the formation of water channels during destabilisation, to provide a path for the unreacted hydrolysed precursor to migrate from their original micellar "prison" towards nuclei and thus provide materials for particle growth. For HLB<10, no particles were detected suggesting that the addition of acetone does not succeed in destabilising the emulsion. In contrast, for HLB>10, destabilisation is often too fast and the nuclei aggregate to form a gel before growth can occur, although Tween 21 (HLB 13.3) has been found to be usable. Thus an HLB of between about 10 and about 14 may be a suitable guideline for the surfactant. It also appears that surfactants having between about 4 and 6 oxyethylene units in their polar head group may be suitable for use in the invention.

When the concentration of surfactant is increased, a similar trend is observed, with the difference that destabilisation by Brij 30 produces a colloidal gel with fused particles, suggesting a partial destabilisation and coalescence of water droplets. The important reduction in the average particle size obtained with NP6 may be explained by an increase in emulsion stability due to an increase in the surfactant concentration.

TMOS hydrolyses more rapidly than TEOS and consequently for an identical concentration of alkoxide inside a droplet, the TMOS system may reach the nucleation threshold faster, thus leading to production of more nuclei. This larger number of nuclei in the TMOS emulsion system leads after destabilisation to the production of smaller particles (since more nuclei for the same amount of hydrolysed monomer provides for more and smaller particles).

A wide range of organic liquids with different dielectric constants and polarities were used, either by themselves or in combination with cyclohexane, to destabilise an NP5 emulsion and produce submicron particles (see Table 2, which shows the liquids according to their dielectric constant). The effect on the emulsion system and the final morphology differs drastically depending on their polarity and their respective miscibility in both water and cyclohexane.

Table 2: Coalescing liquid classified according to their dielectric constant Experimental parameters: NP-5: 10 mmol/pH 1 $HNO_3$, Water 60 mmol/$F^-$: 0.06 mmol/cyclohexane: 50 mL/TMOS: 6 mmol/ageing 48 hrs/destabilise by 100 mL following solvent and 100 mL cyclohexane.

| N° | Solvent | Dielectric Constant | Phenomena |
|---|---|---|---|
| 1 | Water | 80.10 at 20°C | 2 phases, W/O (cloudy) + W (clear) |
| 2 | DMSO | 46.68 at 20°C | 2 phases, all clear |
| 3 | Acetonitrile | 37.50 at 20°C | 3 phases, W/O + O + O/W |
| 4 | DMF | 36.71 at 25°C | 2 phases, all clear |
| 5 | Methanol | 32.70 at 25°C | 2 phases, all clear |
| 6 | Ethanol | 24.55 at 25°C | 1 phase, clear, no particles settled |
| 7 | Acetone | 20.70 at 25°C | 1 phase, clear, particles settled |
| 8 | 1-Propanol | 20.33 at 25°C | 1 phase, clear, no particles settled |
| 9 | iso-Propanol | 19.92 at 25°C | 1 phase, clear, no particles settled |
| 10 | MEK | 18.51 at 20°C | 1 phase, clear, particles settled slowly |
| 11 | 1-Butanol | 17.51 at 25°C | 1 phase, clear, particles settled slowly |
| 12 | tert-Butanol | 12.47 at 25°C | 1 phase, clear, particles settled slowly |

(continued)

| N° | Solvent | Dielectric Constant | Phenomena |
|---|---|---|---|
| 13 | 1-Pentanol | 15.13 at 25°C | 1 phase, clear, particles settled slowly |
| 14 | 1-Hexanol | 13.03 at 20°C | 1 phase, clear, particles settled slowly |
| 15 | Pyridine | 12.47 at 20°C | 1 phase, clear, particles settled slowly |
| 16 | THF | 7.58 at 25°C | 1 phase, clear, particles settled slowly |
| 17 | Dichloromethane | 8.93 at 25°C | 2 phases, O (clear) + O/W (cloudy) |
| 18 | Chloroform | 4.81 at 20°C | 2 phases, O (clear) + O/W (cloudy) |
| 19 | Benzene | 2.284 at 20°C | 2 phases, O (clear) + O/W (cloudy) |
| 20 | Toluene | 2.38 at 25°C | 2 phases, O (clear) + O/W (cloudy) |
| 21 | Cyclohexane | 2.02 at 20°C | |

They may be classified into three different categories:

- Polar solvents (e.g. acetonitrile, methanol), which are only miscible in water. Addition of such solvents leads to micellar swelling until the system moves out of the W/O phase domain into a two phase region (Water phase :W/O emulsion).
- Non-polar solvents (e.g. Toluene, Benzene, Chloroform), which are only miscible in cyclohexane. Addition of such solvent leads to the contraction of the micelles, but a large quantity results in the production of a diphasic mixture.
- Medium polarity solvents (from ethanol to methyl ethyl ketone), which are miscible in both water and cyclohexane. Addition even in large amounts always leads to the production of one phase system.

As described above, the key to a successful destabilisation is the formation of a single phase system which will allow the content of the droplet core to participate freely in colloidal growth and consequent production of submicron particles. Phenomenologically, this may be done either by a substantial swelling of the polar phase channel and the formation of a bi-continuous phase or simply by destruction of the emulsion into a classical solution. In other words, to produce submicron particles, the addition of the coalescing liquid should form a single phase (or bi-continuous) system. This requires the coalescing liquid to be miscible in both water and cyclohexane.

It is important to stress that this miscibility requirement is necessary but not sufficient. The amount used, as well as the dielectric constant of the destabilising liquid (which is mixed with a non-polar liquid to generate the coalescing liquid) is important for control of the formation of submicron particles. Two destabilising liquids (ethanol and acetone) have been successfully used to produce submicron particles, although seven different liquids (see Table 2) were tested and found to produce a single-phase emulsion after addition. This underlines the fact that the miscibility in both the continuous phase and water, as well as the production of a single phase system after destabilisation, is not sufficient to ensure the production of submicron particles.

The inventors hypothesise that the polarity or dielectric constant of the destabilising liquid plays a key role in the successful destabilisation of the emulsion and production of the submicron particles. Figure 20 reveals that when the polarity of the destabilising agent is lowered by gradually substituting acetone by n-propanol, the morphology of the final product slowly evolves from spherical submicron particles to aggregated submicron particles that gradually degrade into fused string of "pearls" and ultimately to a condensed gel. This evolution suggests that, as the dielectric constant of the mixture is lowered, the destabilising power of the destabilising liquid is also reduced.

Phenomenologically, it appears that the water phase channels formed during the destabilisation are not large enough to permit the content of micelles to flow freely and participate in particle growth. In other words, as the destabilising power decreases, the mobility of the hydrolysed precursor become lower than their condensation rate and thus gelation

takes place rather than particle growth, leading to the gelation of the content of the water channel or the bi-continuous phase. This hypothesis is further confirmed by the very open structure of the gel observed in TEM (Figure 20c and d compared to 19 d, e, and f).

On the other hand, when acetone is replaced by ethanol, the morphology progressively evolves towards dense aggregates of submicron and micron-size particles. This trend may be explained by an increase in the destabilisation speed and a further reduction of the condensation rate by a re-esterification of the hydrolysed silicates in the presence of excess ethanol. This decrease in condensation rate might lead to the production of "soft" non-fully condensed submicron particles that aggregate due to Brownian motion into dense clumps.

Following the concept of "destabilising power" discussed above, it appears that the amount of destabilising power (i.e. a combination of the volume and dielectric constant) needs to be high enough to fully destabilise the emulsion, thus freeing the contents (i.e. hydrolysed alkoxides) of the emulsion droplet to allow them to participate in particle growth.

If insufficient coalescing liquid is introduced, a gel is gradually formed by collision of nuclei. If too much coalescing liquid is used then the system can form a gel (as in figure 17f for ethanol).

As can be seen from Table 3 and Figure 7, as the dielectric constant of the non-polar liquid of the coalescing liquid decreases, the morphology of the final product degrades from well-formed spherical particles in cyclohexane (see figure 6) to aggregated submicron particles in dodecane and decane (Figure 7e and d), fused particles string in octane and particulate and condensed gel in hexane and petroleum ether. Thus, for a constant destabilisation power (i.e. same quantity of acetone), the destabilisation decreases with the decreasing dielectric constant of the destabilising liquid, thus leading gradually (as observed above when replacing acetone by a less polar solvent) to the formation of 3-dimensional network.

Table 3: Influence of different non-polar liquids

| Solvent | Dielectric Constant | Final Product Morphology |
|---|---|---|
| Hexanol | 13.3 | |
| Toluene | 2.38 | Dense aggregate of nuclei |
| Cyclohexane | 2.02 | Submicron particles |
| Dodecane | 2.015 | Heavily agregated submicron particles with large polydispersity |
| Decane | 1.99 | Heavily agregated submicron particles with large polydispersity |
| Octane | 1.95 | Fused particle strings |
| Hexane | 1.89 | Dense aggregate of nuclei |
| Petroleum ether | N/A * | condensed gel |
| * Not applicable because petroleum ether is a mixture of alkanes and the dielectric constant varies depending on their respective volume fractions | | |

It is important to note that the coalescing liquid contained 100 ml of cyclohexane thus shifting the average polarity of the oil phase upon addition.

Figure 25 shows the $N_2$ adsorption isotherm of a sample of submicron particles prepared at pH=1. The key values (surface area and pore volume) are also summarised in Table 4. The peaks in the pore size distribution were determined from Figure 38. The isotherm reveals the presence of two regions of porosity, inside the particles.

Table-4: BET results of sub-micron silica particles

| Sample | Destabilisation conditions | Surface Area ($m^2$ $g^{-1}$) | Microporous Vol. (< 1.7 nm) | Peak in pore size distribution |
|---|---|---|---|---|
| LK-425 | By 100 mL acetone & 100 mL cyclohexane | 510 | 36% | 1.1 nm |
| LK-428 | Neutralise water pool with NaOH, then by 100 mL acetone & 100 mL cyclohexane | 513 | 19% | 1.5 and 2.7 nm |

It is important to note that the surface area of the particles corresponds to the surface of dense silica particles around 6 nm in size. This confirms that surface area is related to the internal surface of the particles and consequently that the submicron particles are highly porous. Furthermore, the proportion of microporous volume decreases with an increase in pH by titration of the droplets prior to destabilisation. It is known that addition of base may promote condensation

inside the droplets, thus increasing the number of solid particles. This will, after destabilisation, increase the proportion of mesopores. This provides an example of control of the particles internal structure and thus release kinetic of the encapsulated molecules.

An additional consequence of the destabilisation of the emulsion and colloidal growth by migration of the content of the core through polar phase channels is the low encapsulation efficiency of the releasable substance, for example a drug (see Figure 21). In the initial emulsion, the drug is compartmentalised inside the water droplets. If nucleation and growth takes place inside the droplet core prior to destabilisation then the drug is encapsulated in the silica. After destabilisation, the contents of non-condensed droplets (i.e. hydrolysed silicon alkoxide + free drug) are diluted in acetone. Although the silicon alkoxide may migrate towards nuclei and contribute to the growth of submicron particles, it is unlikely that much of the drug will get encapsulated at this stage.

In summary, submicron particles may be produced by destabilisation of acid catalysed sol-gel emulsions. The resulting particles may be used to encapsulate and release small molecules in a controlled fashion over extended periods of time (up to 6 months). The internal structure of the submicron silica particles, and thus the release rate, may be controlled by initial sol-gel parameters such as pH. The encapsulation efficiency appears to be dependent on the solubility of drug in destabilising mixture.

The conditions necessary to achieve successful destabilisation appear to be:

- A surfactant with an HLB (Hydrophile/Lipophile Balance) between 10 and 14. This translates into a good balance between the lipophilic and hydrophilic regions of the surfactant, which is required for the formation of bicontinuous phases or liquid crystals, which allows the migration of the hydrolysed alkoxide to the nuclei and participation in the particle growth process. In order to achieve successful destabilisation the surfactant should have medium strength molecular interaction between its polar head and the water pool. This molecular interaction may be characterised by the surfactant footprint, which is calculated by dividing the surface area of the water droplet surface by the aggregation number of the surfactant. A medium interaction corresponds to a surfactant footprint between 1.5 and 10 $nm^2$ per molecule. A suitable surfactant footprint for a surfactant usable in the present invention may be between about 1.5 and about 10nm2/molecule, or between about 1.5 and 5, 2 and 5, 3, and 5, 1.5 and 3, 5 and 10, 2 and 10, 2 and 8, 2 and 6 or 2 and 4 $nm^2$/molecule, and may be about 1.5, 2, 2.5, 3, 3.5, 4, 4.5, 5, 6, 7, 8, 9 or 10 $nm^2$/molecule.
- A coalescing liquid which does not form two phases (e.g. water and water-in-oil emulsion) when added to the emulsion system. The formation of two phases will lead to heterogeneous nucleation and segregation of the particles in different physical phases. In terms of solubility requirement, this means that the coalescing liquid should be miscible in both oil and water phases, that is, it should have the appropriate polarity.
- The "destabilisation power" (i.e. a combination of polarity and volume added) has to sufficiently high to induce channel formation and free flow of droplet core content to participate to the growth of nuclei into a submicron particle. When the destabilisation power decreases, it reduces mobility inside the polar phase channel and gradually leads to an evolution of the final microstructure from discrete submicron particles, to strings of coalesced particles and macroporous condensed gel. This could be viewed as a gradual freezing of the bicontinuous/channel structure.

The process of the present invention is capable of producing sub-micron particles in an acid environment by use of a sol-gel mechanism in a microemulsion. Acid catalysis is important in the production of microporous particles that can encapsulate small molecules such as doxorubicin and release them in a controlled fashion. The present invention uses a novel approach for building sub-micron (i.e. large nano) particles in emulsion. Contrary to earlier work (WO1/62332 Barbé & Bartlett "Controlled release ceramic particles, compositions thereof, processes of preparation and methods of use") in which it was necessary to screen surfactant/solvent couples to obtain an emulsion with the correct droplet size to generate the desired final particle size, the method of the present invention starts with a stable microemulsion having droplets of about 5 to 10nm diameter, and then destabilises the microemulsion to produce particles of between about 30 and 100nm, or about 50 and 500nm or about 100 and 400nm.

The particulate substance of the invention having a drug releasably immobilised therein and/or thereon may be used in the treatment of a condition in a mammal. The mammal may be selected from the group consisting of human, non-human primate, equine, murine, bovine, leporine, ovine, caprine, feline and canine. The mammal may be selected from a human, monkey, ape, horse, cattle, sheep, dog, cat, goat, llama, rabbit and a camel, for example. The condition may be a disease. The condition may be for example cancer, diabetes, hormonal dysfunction, hypertension, pain (for example pain treatable by morphine and/or opiates), or asthma.

Doxorubicin can be encapsulated inside the sub-micron particles and release gradually in PBS (phosphate buffer saline) solution.

The present invention may also be used for controlled release of other substances, such as fluorescent dyes, radiopharmaceuticals, enzymes, catalysts, hormones, biocides and/or other substances. Applications may include diagnostics, radiodiagnostics, radiotherapy, biotechnology, bioreactors, imaging etc.

Examples

The following general experimental procedure was used in the experiments detailed below for preparing nanoparticles containing doxorubicin, with the variations from this general procedure detailed for the individual experiments:

1. Dissolve NP-5 [nonylphenoxypolyethoxyethanol, $C_9H_{19}C_6H_4(OCH_2CH_2)_nOH$, n=5] 4.40 - 8.80g (10 - 20 mmol) in 50 mL cyclohexane;
2. Add 1.08 mL dilute nitric acid, pH1 (equivalent to 60 mmol water) containing 0.06 mmol NaF and 0.1 - 1.0 mg doxorubicin. Stir for 20 minutes to produce a microemulsion;
3. Add 0.911 mL (6 mmol) TMOS (tetramethoxysilane) into the above system;
4. Age by stirring for 24 to 48 hours;
5. Pour the resultant emulsion into a stirred mixture of dry acetone (100 mL) and cyclohexane (100 mL), and stir for 10 minutes, to destabilise the emulsion and coalesce the particles;
6. After sedimentation, separate the solid particles from organic (liquid) phase and wash the particles three times with 50 mL acetone each time. Alternatively the particles may be washed using a procedure described in WO1/62332 (Barbé & Bartlett, "Controlled Release Ceramic Particles, Compositions Thereof, Processes of Preparation and Methods of Use").
7. (Optional) Mix the solid particles with 5 - 10 mL aqueous NaCl solution with a concentration calculated to provide at least 85% of the dry mass of the total solid (i.e. silica and NaCl), then freeze dry the solution to make the final dry product.

This procedure was followed (steps 1 to 6) with the following variations:

***1. NP-5 concentrations effect:***

Particles were synthesized using the following experimental parameters: water (pH1) 60 mmol, $F^-$ 0.06 mmol, TMOS 6 mmol, cyclohexane (at step 1) 50 mL, ageing 48 h and destabilisation with a mixture of 100 ml acetone/100ml of cyclohexane. The surfactant (NP-5) concentration at step 1 was varied from 0.2 mol/L to 0.8 mol/L. TEM micrographs of the resulting particles are shown in Figure 2.
**Result:** The particle size decreased with increasing surfactant concentration. Above 0.4 mol/L, no coalescence was observed.

***2. TMOS concentrations effect:***

Particles were synthesized using the following experimental parameters:

Surfactant NP-5 0.4 mol/L, $F^-$/TMOS molar ratio 0.01, water at pH1, cyclohexane (at step 1) 50 mL, ageing 48 h and destabilisation with a mixture of 100 ml acetone/100ml of cyclohexane.
The TMOS concentration at step 3 was increase from 4 mmol to 12mmol while keeping the water to TMOS molar ratio at 10. TEM micrographs of the resulting particles are shown in Figure 3.

**Result:** The particle size was found to increase with TMOS content. The coalescence/fusing of the particles at [TMOS]=12 mmol is significant.

***3. Surfactant type effect***

***low concentration:***

The following surfactants were used in place of NP-5 at step 1: (a) Brij 30 (HLB=9), (b) NP-5 (HLB=10), (c) NP-6 (HLB=10.9) (d) Triton X-114 (HLB= 12.4) with 1-pentanol 20 mmol (e) NP-9 (HLB=13) with 1-pentanol 20 mmol (f) Triton X-100 (HLB=13.5) with 1-pentanol 20 mmol. Particles were synthesized using the following experimental parameters: Surfactant 0.2 mol/L, water (pH1) 60 mmol, $F^-$ 0.06 mmol, TMOS 6 mmol, in 50 mL cyclohexane (at step 1), ageing 48 h and destabilisation with a mixture of 100 ml acetone/100ml of cyclohexane. TEM micrographs of the resulting particles are shown in Figure 4.
**Result:** The only systems to form submicron sized spheres were those with NP-5 and NP-6. No destabilisation took place in sample (a). The "coalesced droplet" morphology of sample (f) was the results of the unstable nature of the microemulsion used in its synthesis.

***high concentration:***

The same experiments were conducted as at low concentration (above), increasing the surfactant concentration at step 1 to 0.4 mol/L. TEM micrographs of the corresponding particles are shown in Figure 5.
**Result:** The only systems to form submicron spheres at this surfactant concentration were those with NP-5. All the others with the exception of those with NP-6 produced particles of diameter less than 20 nm, suggesting that the micro-emulsion are not destabilised, and the particles do not coalesce, on addition of acetone. The slight growth observed for systems with NP-6 suggests a reduced destabilisation.

***4. Co-surfactant effect:***

Particles were synthesized using the following experimental parameters:

Water (pH1) 60 mmol, $F^-$ 0.06 mmol, TMOS 6 mmol, cyclohexane (at step 1) 50 mL, ageing 48 h and destabilisation with a mixture of 100 ml acetone/100ml of cyclohexane. The surfactant at step 1 was: (a) NP-5 0.2 mol/L; (b) NP-5 0.4 mol/L; (c) NP-5 0.2 mol/L, 1-pentanol 0.2 mol/L.
TEM micrographs of the corresponding particles are shown in Figure 6.

**Result:** No visible improvement was caused by adding a co-surfactant (i.e. surfactant) to the emulsion. Increasing the surfactant concentration (experiment 4(b)) was more effective in narrowing the particle size distribution.

***5. Microemulsion solvent effect***

***destabilised using 100 mL acetone/100 mL various non-polar solvents:***

Particles were synthesized using the following experimental parameters: NP-5 10 mmol (0.2 mol/L), water (pH1) 60 mmol, $F^-$ 0.06 mmol, TMOS 6 mmol, a non-polar solvent (at step 1) 50 mL, ageing 48 h and destabilisation with a mixture of 100 ml acetone/100ml of the non-polar solvent. Several solvents were used as the continuous phase for forming the microemulsions: (a) petroleum benzine, 1-hexanol 20 mmol; (b) hexane, 1-hexanol 20 mmol; (c) octane, 1-hexanol 20 mmol; (d) decane, 1-hexanol 20 mmol; (e) dodecane, 1-hexanol 40 mmol; (f) toluene. Hexanol was added as a cosurfactant in order to obtained stable micro-emulsions. TEM micrographs of the corresponding particles are shown in Figure 7.
**Result:** Some submicron particles were obtained for experiments 6d and 6e, suggesting that a partial controlled destabilisation (ie particle coalescence) was achieved for emulsions synthesized in decane and dodecane.

***destabilised by 50 mL ethanol/100 mL cyclohexane:***

The same emulsions as described in experiment 6 were prepared and destabilised using a 50 mL ethanol/100 mL cyclohexane mixture at step 5. TEM micrographs of the corresponding particles are shown in Figure 8.
**Result:** a) petroleum benzine/1-hexanol 20 mml and b) hexane/1-hexanol 20 mmol systems formed submicron particles. Coalescence of the octane/1-hexanol 20 mmol (experiment c) resulted in a gel and no coalescence took place in decane or dodecane (experiments d and e).

***toluene microemulsions:***

The following surfactants were used in place of NP-5: (a) NP-5, (b) Triton X-114 and (c) NP-9. Particles were synthesized using the following experimental parameters: surfactant 20 mmol (0.2 mol/L), water (pH1) 40 mmol, $F^-$ 0.06 mmol, TMOS 6 mmol, toluene 100 mL (at step 1 in place of cyclohexane), ageing 24 h and destabilisation with a mixture of 100 ml acetone/100ml of cyclohexane. TEM micrographs of the corresponding particles are shown in Figure 9.
**Result:** During the synthesis, silica particles were isolated from the emulsions with time and they eventually formed a gel with average particle sizes between 10 - 50 nm.

***6. Ageing time effect***

Particles where synthesized using the following experimental parameters: NP-5 10 mmol (0.2 mol/L), water (pH1) 60 mmol, $F^-$ 0.06 mmol, TMOS 6 mmol and cyclohexane 50 mL. This emulsion was aged for: (a) 24 h, (b) 48 h, (c) 120 h, (d) 168 h, before being destabilised with a mixture of 100 ml acetone/100ml of cyclohexane. TEM micrographs of the corresponding particles are shown in Figure 10.

**Result:** The ageing time had no significant influence on the final particle size.

***7. Effect of the hydrolysable species:***

Particles were synthesized using the following experimental parameters: NP-5 10 mmol (0.2 mol/L), TMOS or TEOS 6 mmol, water (pH1) 60 mmol, $F^-$ 0.06 mmol, cyclohexane (at step 1) 50 mL, ageing 48 h and destabilisation with a mixture of 100 ml acetone/100ml of cyclohexane. TEM micrographs of the particles before and after destabilisation are shown in Figure 11.

**Result:** A comparison of the particles before and after destabilisation clearly shows that addition of acetone, which on the macroscopic scale results in a crashing of the stable micro-emulsions into a white precipitate, induces on the microscopic scale substantial particle growth. Final particles exhibit a slightly larger particle size when TEOS is used as the hydrolysable species.

***8. Precipitation condition effect:***

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL, ageing 48 h.

The destabilisation of step 5 was then conducted in different ways.

<u>8-1 Addition sequence</u>

(a) by pouring 50 mL acetone into the microemulsion; (b) by pouring the microemulsion into 50 mL acetone; (c) by pouring the microemulsion into 100 mL acetone; (d) by pouring the microemulsion into a mixture of 100 mL acetone and 100 mL cyclohexane. TEM micrographs of the corresponding particles are shown in Figure 12.

**Result:** Although submicron particles are obtained in all cases, pouring the microemulsion into a diluted solution of acetone (experiment 11-1d) appears to have produced the most homogenous and less aggregated samples.

<u>8-2 Quantity of coalescing liquid</u>

by pouring the microemulsion: (a) into a mixture of 50 mL acetone and 50 mL cyclohexane; (b) into a mixture of 100 mL acetone and 100 mL cyclohexane; (c) into a mixture of 150 mL acetone and 150 mL cyclohexane; (d) into a mixture of 200 mL acetone and 200 mL cyclohexane. TEM micrographs of the corresponding particles are shown in Figure 13.

**Result:** Although the destabilisation with a mixture of 50 ml of acetone/50ml of cyclohexane (experiment 11-2a) produced submicron particles, the particles were largely aggregated and fused together. Increasing the volume of the destabilising mixture leads to a lower aggregation but an increase in polydispersity.

<u>8-3. Precipitation condition effect:</u>

Particles were destabilised using a 100 ml acetone/100 ml cyclohexane mixture. The destabilisation (step 5) was performed by pouring the microemulsion in: a) a 1 litre beaker stirred with a rod shape magnetic stir-bar; b) a 250 ml beaker stirred with a cross-shape magnetic stir-bar; c) a 250 ml beaker stirred with a rod shape stir-bar; d) in a 250 ml beaker ultrasonicated for 5 minutes; and e) with NaCl (0.2M) added. In e), 0.135 mL 1 mol/L NaCl solution was added into the microemulsion, which was then shaken until clear, and then destabilised by pouring into a stirred mixture of acetone and cylcohexane in a 250 ml beaker stirred with a rod-shaped stir-bar stirring at moderate stirring speed. TEM micrographs of the corresponding particles are presented in Figure 14.

**Result:** The container volume, magnetic stir-bar shape, ultrasonication, or salt addition had no significant effect on final particle size.

***9. Effect of pH of the emulsion droplets:***

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL, ageing 48 h. The pH of the water droplets was adjusted from 1.5 to 7 by addition of base (1M aqueous NaOH solution), before destabilisation in a mixture of 100 ml acetone/100 ml cyclohexane. TEM micrographs of the corresponding particles are shown in Figure 15.

**Result:** No significant effect of the pH on the final particle morphology was observed.

***10. Influence of the nature and quantity added of the destabilising liquid***

10-1 Acetone

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL and ageing 48 h. The microemulsion was then destabilised at step 5 using a mixture of 100 mL cyclohexane and different amounts of acetone: (a) 10 mL, (b) 20 mL, (c) 50 mL, (d) 100 mL and (e) 200 mL. TEM micrographs of the corresponding particles are shown in Figure 16.
**Result:** In experiments 10-1a and 10-1b (10 or 20 ml acetone), no immediate precipitate was observed, however a gel gradually formed with time. In experiment 10-1c (50 ml acetone), the gel formed immediately. In experiments 10-1d and 10-1d (100 ml or 200ml acetone), spherical submicron particles were formed.

10-2 Ethanol

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL, ageing 48 h. The microemulsion was then destabilised at step 5 using a mixture of 100 mL cyclohexane and different amount of ethanol: (a) 5 mL; (b) 10 mL; (c) 20 mL; (d) 35 mL; (e) 50 mL; (f) 80 mL; (g) 100 mL. TEM micrographs of the corresponding particles are shown in Figure 17.
**Result:** When the quantity of ethanol was 5 or 10 ml (experiments 10-2a bd 10-2b), no immediate precipitate was observed, however a gel gradually formed with time. With 20 ml ethanol (experiment 10-2c) a gel formed immediately after pouring the emulsion into the cyclohexane/ethanol mixture. From 35 ml to 80 ml ethanol (experiments 10-2d to 10-2f), spherical submicron particles were formed although aggregation increased with the amount of ethanol, giving rise to a mixture of particles and gel for the sample destabilised with 80 ml of ethanol (experiment 10-2f). A further increase in the quantity of ethanol (experiment 10-2g) led to gelation.

10-3 Mixture of acetone and ethanol

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL, ageing 48 h. The microemulsion was then destabilised at step 5 using a mixture of: a) 100 mL cyclohexane, 80 mL acetone, 20 mL ethanol; b) 100 mL cyclohexane, 60 mL acetone, 40 mL ethanol; c) 100 mL cyclohexane, 40 mL acetone, 60 mL ethanol; d) 100 mL cyclohexane, 20 mL acetone, 80 mL ethanol. TEM micrographs of the corresponding particles are presented in Figure 18.
**Result:** Submicron particles were formed with a mixture cyclohexane, acetone and ethanol. The aggregation increases with increasing proportion of ethanol, giving rise to a gel structure for more than 20/80 ethanol to acetone volume ratio (experiments 10-3b to 10-3d).

10-4 Other solvents

Particles were synthesized using the following experimental parameters: NP-5 10 mmol (0.2 mol/L), water (pH1) 60 mmol, $F^-$ 0.06 mmol, TMOS 6 mmol, cyclohexane (at step 1) 50 mL, ageing 48 h. The micro emulsion was then destabilised at step 5 using a mixture of 100 mL cyclohexane and different amount of the following solvents:

(a) iso-propanol: 10 mL, 25 mL, 50 mL, 100mL
(b) 1-propanol: 25 mL, 50 mL, 100 mL, 200 mL
(c) Methylethyl ketone: 25 mL, 50 mL, 100 mL, 200 mL
(d) chloroform: 25 mL, 50 mL, 100 mL
(e) toluene: 100 mL
(f) benzene: 100 mL
(g) tetrahydrofuran (THF): 100 mL
(h) dimethylformamide (DMF): 100 mL
(i) pyridine: 100 mL
(j) 1-butanol: 100 mL
(k) acetonitrile: 2 mL, 5 mL, 10 mL, 20 mL
(l) methanol: 2 mL, 5 mL, 10 mL, 20 mL
(m) dimethylsulfoxide (DMSO): 2 mL, 5 mL, 10 mL, 20 mL
(n) tert-butanol: 100 mL

(o) 1-pentanol: 100 mL
(p) 1-hexanol: 100 mL
(q) dichloromethane: 100 mL

TEM micrographs of some of the corresponding particles are shown in Figure 19. All samples in Fig. 19 were obtained by destabilising the microemulsion with a mixture of 100 mL of the appropriate solvent and 100 mL cyclohexane.
**Results:** Destabilisation using isopropanol and n-propanol led to the formation of gel with time. Destabilisation did not take place when acetonitrile or methanol was used: no particles were formed. Chloroform led to the formation of coalesced gel structure, and use of toluene, benzene or THF led to the production of fine gel structures.

10-5 Other mixtures

Particles were synthesized using the following experimental parameters: 5 mmol NP-5, cyclohexane (at step 1) 25 mL, pH=1 $HNO_3$ with water 30 mmol, $F^-$ 0.03 mmol, TMOS 3 mmol and aged 48 hrs. The micro emulsion was then destabilised using a mixture of 50 mL cyclohexane and different amount of the following solvents:

| Destabilizing liquid | Result |
|---|---|
| LNK-752: 40 mL acetone + 10 mL 1-propanol | Submicron particle |
| LNK-753: 30 mL acetone + 20 mL 1-propanol | Particles and gels |
| LNK-754: 20 mL acetone + 30 mL 1-propanol | Gel: Fused particles |
| LNK-755: 10 mL acetone + 40 mL 1-propanol | Gel: condensed |
| LNK-756: 40 mL ethanol + 10 mL 1-propanol | Gel: condensed |
| LNK-757: 30 mL ethanol + 20 mL 1-propanol | Gel: condensed |
| LNK-758: 20 mL ethanol + 30 mL 1-propanol | Gel: condensed |
| LNK-759: 10 mL ethanol + 40 mL 1-propanol | Gel: condensed |
| LNK-760: 40 mL acetone + 10 mL *i*-propanol | Submicron particle |
| LNK-761: 30 mL acetone + 20 mL *i*-propanol | Particles and gels |
| LNK-762: 20 mL acetone + 30 mL *i*-propanol | Gel: Fused particles |
| LNK-763: 10 mL acetone + 40 mL *i*-propanol | Gel: condensed |
| LNK-764: 40 mL ethanol + 10 mL i-propanol | Gel: condensed |
| LNK-765: 30 mL ethanol + 20 mL i-propanol | Gel: condensed |
| LNK-766: 20 mL ethanol + 30 mL i-propanol | Gel: condensed |
| LNK-767: 10 mL ethanol + 40 mL i-propanol | Gel: condensed |
| To illustrate, the morphology of LNK 752 to LNK 755 are represented in Figure 20**.** | |

### *11. Encapsulation and release of doxorubicin*

Figure 21 shows the doxorubicin encapsulation efficiency as a function of the quantity of doxorubicin added to the solution of step 1. About 30% of doxorubicin remained encapsulated the rest was lost during the destabilisation step (due to the high solubility of doxorubicin in acetone) and the washing.
In normal phosphate buffer, doxorubicin decomposes exponentially during the first ten days with half decay ($T_{1/2}$) about 4.5 days, as shown in Figure 22. The decomposition rate becomes slower after 10 days: about 74% of doxorubicin disappears in 2 weeks. Release of doxorubicin from the nanoparticles is summarised in Figures 23 and 24. Figure 23 shows the short-term release behaviour of nanoparticles synthesised using the process of the present invention. The lower curve represents the active (non-denaturated) doxorubicin as a function of time, and shows that a quasi-constant concentration of active doxorubicin can be maintained over 30 days. The higher curves represent the total amount of doxorubicin released (i.e. active + inactive). Figure 24 shows that nanoparticles synthesised using the process of the present invention can release doxorubicin over a period of more than six months and maintain an active concentration in vitro.

## Additional Experiments

### 1) Typical synthesis conditions

A 0.2 M surfactant solution was prepared in 50 mL cyclohexane. 1.08 mL nitric acid 0.1M containing 0.06 mmol NaF was added to the surfactant solution and the resulting mixture was stirred for 20 minutes to produce a microemulsion. 6 mmol TMOS was then added into the above system which was stirred for 48 hours. The emulsion was then poured into a stirring mixture made of 100 mL acetone and 100 mL cyclohexane and was left stirring for 10 more minutes. After sedimentation, the particles were separated from organic phase and washed three times with acetone. The particles were then resuspended in about 10 mL of NaCl aqueous solution, washed further by decantation with chloroform, and finally freeze dried. The particles were found to be distributed homogeneously in NaCl matrix with silica and sodium chloride weight ratio at 85%. The particles could also be used directly in the form of an aqueous suspension after washing prior to freeze-drying. The process is shown schematically in Fig. 27. **2) Influence of the nature of the surfactant:** This is discussed in section 15.

### 3) Destabilisation of ORMOCER/TMOS mixture

The particles were synthesised according to the typical procedure (see Fig. 27) but using a mixture of ORMOSILs and TMOS as the silica precursor. Both were introduced in the micro-emulsion at the same time, 48h prior to destabilisation. The corresponding TEM micrographs are presented in Figures 28 and 29. Only two hybrid systems produced particles (i.e. GTMS and APTMS, Figure 28-d and 29 respectively). The introduction of 25% of GTMS (glycidopropoxy trimethoxy silane) led to strongly aggregated submicron particles and 25% APTMS (aminopropyl trimethoxysilane) led, after destabilisation, to a bimodal distribution of large and small particles. Both system were investigated using lower percentages (5-15%) of ORMOSILs. The corresponding TEM micrographs are presented in Figure 30. They show increasing agglomeration of the submicron particles for increasing amount of GTMS being introduced in the system. In contrast as the amount of APTMS increased the particle size decreases.

### 4) Destabilisation of TEOS/TMOS mixture:

Particles were synthesised according to typical procedure using a TMOS/TEOS mixture as silica precursors in place of a single alkoxide. The corresponding TEM micrographs are presented in Figure 31. No significant difference was observed with the changing proportions of

TMOS/TEOS.

### 5) Destabilisation of multiple emulsions system :

Two microemulsions were prepared according to the typical synthesis conditions. Emulsion-A contains dye-A and emulsion-B contains dye-b. Immediately before destabilisation, the two emulsions were briefly mixed and stirred for 10 minutes. (See Fig. 32). Destabilisation by the acetone/cyclohexane mixture produced submicron silica particles. The resulting powders were characterised using UV/Vis DRIFT (Diffuse-Reflectance Infrared Fourier Transform) spectoscopy (see Fig. 33). Powders containing the individual dyes were also synthesized and analysed to provide a comparison.

### 6) Destabilisation of an emulsion containing both nanoparticles synthesised using base catalysis (i.e. seeds) and monomer hydrolysed in acid media

The silica nanoparticles produced using base catalysis were synthesised as follows. NP-9 (6 mmol), 1-pentanol (6 mmol) and cyclohexane (30 mL) were mixed together. 0.648 mL of aqueous ammonia $NH_4OH$ (1.333M) representing an equivalent 36 mmol water was combined to the previous solution to form a micro-emulsion. TEOS (1.2 mmol) was then added into the microemulsion and the system was aged for 48 hours. An acidic aqueous phase was then added to the micro-emulsion now containing 50 nm silica particles and the system was further stirred at 60 °C for 5 hours. NP-9 (12 mmol), 1-pentanol (12 mmol) and cyclohexane (60 mL) were then added, followed by addition of silicon alkoxide. The mixture was further aged for 48 hours and then destabilised using a mixture of acetone and cyclohexane. The corresponding TEM image are shown in Figure 34. A summary of the synthetic procedure is given in Fig. 35.

The original seed synthesised in base may also be made of hybrid materials (i.e. using a mixture of ORMOSILs and TMOS) A typical synthesis procedure for these seeds involved mixing of NP-5 (5 mmol) and cyclohexane (25 mL) and 0.54 mL 1.333M $NH_4OH$ followed by the addition of TMOS or a mixed silica precursor and ageing for 24 hours. The corresponding TEM micrographs of the seed particles are shown in Figure 36 (first column). 0.54 mL of 1.5 M nitric acid

with $F^-$ 0.03 mmol was then added to the mixture and the system was further stirred at 60 °C for 5 hours. NP-5 (5 mmol) and cyclohexane (25 mL) were added, followed by the addition of 3 mmol of TMOS. The system was further aged 24 hours prior to destabilisation with a mixture of acetone and cyclohexane. The TEM micrographs of resulting particles are presented in the right column of Figure 36.

**7) Destabilisation of a two emulsions system in which one contains nanoparticles (synthesized in base) and the other one contains oligomers (synthesized in acid):**

The synthetic procedure follows the scheme in Fig. 32, with Emulsion 1 using a base catalysis and Emulsion 2 using acid catalysis.

- Emulsion-I was prepared by mixing NP-9 (6 mmol), 1-pentanol (6 mmol), cyclohexane (30 mL) and 0.648 mL of 1.333M $NH_4OH$ (36 mmol equivalent of water). TEOS (1.2 mmol) was then added into microemulsion and the system was aged for 48 hours. TEM of particles is shown in Figure 33a.
- Emulsion-II was prepared by mixing NP-5 (6 mmol) and cyclohexane (30 mL) and 0.648 mL of pH 1 $HNO_3$ with $F^-$ 0.036 mmol. TMOS (3.6 mmol) was then added into the microemulsion and the resulting mixture was aged for 48 hours.

The two emulsions were then mixed and destabilised using a mixture of 100 mL of cyclohexane and 100 mL of different polar solvents. The TEM micrographs of the corresponding particles are presented in Figure 37.

**8) The pore size distribution of submicron silica particles synthesised by destabilisation of micro-emulsion**

The nitrogen adsorption-desorption isotherms for two samples synthesised at two different pH's have been discussed above. In Figures 38 (LK-425, synthesised at pH 1 and LK-428, synthesised at pH 7, neutralised before destabilisation), the corresponding pore size distribution are presented. These were calculated using a Density Functional Theory (DFT) model with cylindrical shaped pores (P.Webb, C. Orr in "Analytical Methods in Fine Particles Technology", pp81-87, Micromeretics Corporation Norcross GA,USA, 1997).

**9) Influence of the sonication during destabilisation on the particle size distribution**

The particle size distribution was measured by dynamic light scattering (DLS) using a Malvern HPPS instrument. The silica particle suspension was sonicated for 10 minutes in water bath. 0.5 mL of the resulting suspension was diluted by 5 mL of pH 9 NaOH solution and filtered through a 0.8 μm filter into a glass cuvette. Measurements were conducted at 25 °C. The resulting particle size distribution is shown in Figure 39. The particle size is bimodal with a small peak centred around 38 nm and a larger peak centred around 255 nm. When the solution was sonicated using an ultrasound probe with 50% duty cycle and 2 sec cycle time (Sonicator-Ultrosonic Processor, Heat System-Ultrasonics Inc.) during the destabilisation step, the small peak at 38 nm disappeared (Figure 40), giving rise to a monomodal size distribution centred on an average size of 220 nm.

**10) Encapsulation of doxorubicin in silica particles synthesized at different pH**

Silica nanoparticles containing doxorubicin were prepared according to the following procedure. 20 mmol of NP5 was mixed with 100 mL of cyclohexane, 2.16 mL of $HNO_3$ at pH=1, 0.12 mmol of $F^-$ and 1.5 mg of doxorubicin (Doxorubicin-HCl purchased from Australian Pharmaceutical Ingredients Pty. Ltd). 12 mmol of TMOS was then added and the micro-emulsion was aged for 3 days. Then 0.432 mL of NaOH 0.5 M was added to bring the water pools to pH=7.

After the addition of the base, the samples were stirred for 2 more hours prior to destabilisation with 200 mL acetone and 150 mL cyclohexane. After settling of the particles at the bottom of the beaker they were washed with 100 mL acetone three times. Then the particles were resuspended in a solution composed of 4.80 g of NaCl dissolved in 20 mL of deionised water. The suspension was washed three times by decantation using 100 mL of chloroform each times and freeze-dried.

**11) Characterisation of the doxorubicin release from the silica particles using HPLC**

All solvents used were HPLC grade and filtered through a 0.45 μm filter prior to degassing by sonication. The HPLC system consisted of a Waters 1525 binary HPLC pump, in combination with a Waters 717 plus autosampler, a Waters 2487 Dual λ absorbance detector (480 nm), and a Waters 2475 multi λ fluorescence detector (excitation λ: 363 nm, emission λ: 550 nm). The HPLC column used was a Waters Atlantic™ dC-18, 5 μm, 4.6 mm × 150 mm column,

with Waters Atlantic™ dC-18 guard column attached. The mobile phase consisted of 1% acetic acid in water, and acetonitrile, with a gradient of 5% to 95% acetonitrile over 20 minutes and a flow rate of 0.8 mL/min. Doxorubicin concentrations were determined by integration of the area under the curve and comparison to that of standard doxorubicin samples at 0.4, 0.8, 2, 4, and 10 μg/mL. The minimum acceptable $R^2$ for the standard curve was 0.9. Processing of both absorbance and fluorescence HPLC spectra was performed using Waters Breeze™ software.

*11.1) Release at pH < 4*

The release of doxorubicin was studied at pH < 4 (pH ~3.4) as the compound is most stable at this pH. Release experiments were performed using doxorubicin doped nanoparticles (1 g). The particles were suspended in 1% acetic acid solution in Milli-Q water (30 mL). Samples were incubated at 37 °C with stirring. Aliquots (100 μL) were taken after centrifugation at 5000 rpm for 5 minutes. Samples were analysed by HPLC as previously described.

Figure 41 shows the release profile over a 9 day period. The sample synthesized at pH=1 showed an increase in doxorubicin release between an initial burst and day 2. Subsequent to day 2, both systems examined show a slow decrease in doxorubicin concentration. This decrease is due to the slow degradation of doxorubicin in aqueous conditions. Figure 42 shows a comparison of the release profile of a sample synthesised at pH=3 (diamond symbols) versus the formation of degradation product (square symbols). It may be seen from this graph that the decrease in concentration of doxorubicin is very similar to the amount of degradation product detected in the HPLC trace, with a change in concentration of both being approx 0.2 μg/mL between day 2 and day 9. Analysis subsequent to day 9 was not included in the data reported, as release was masked by increased amounts of degradation, and precipitation of an orange coloured product was evident in the spiked sample. The precipitate is attributed to the limited solubility of both doxorubicin and the degradation products in aqueous solutions.

*11.2) Release at pH=7.4*

Studies were undertaken of the release of doxorubicin at physiological pH (7.4). Release studies were performed in 0.02 M phosphate buffered saline (PBS) at 37 °C according to the same procedure used at pH < 4. Figure 43 shows the release curves obtained at pH 7.4. In the sample synthesised at pH=1, there was a visible increase in doxorubicin concentration in the first 24 to 48 hours. After 48 hours, a significant decrease in concentration is seen. Analysis of results from day 4 was difficult, as degradation products began overlapping with the peak of doxorubicin at retention time 11.7-11.8 min. Beyond day 5 no analysis was possible, as the peaks associated with degradation products began to swamp the peak of doxorubicin making it impossible to discern.

The decomposition rate of doxorubicin at physiological pH (pH 7.4) versus its decomposition rate at pH < 4 makes analysis of the system difficult. Figure 44 shows a comparison of the decomposition of a known concentration of doxorubicin at both pH 7.4 and pH < 4. The increased decomposition at physiological pH is clearly evident, making study of this system difficult, as the degradation products have a retention time similar to that of doxorubicin itself.

*11.3) Cumulative Release*

To overcome this problem an alternative experimental procedure was developed. As opposed the previous methods where an aliquot of the liquid was isolated from the total volume, the new procedure involves complete removal of the supernatant for each time point. Doxorubicin doped nanoparticles (35 mg) were suspended in PBS buffer (1 mL) and shaken at room temperature. At the required time points (1 hour, 1, 2, 6 and 9 days) the samples were centrifuged (12,000 rpm, 30 minutes) and the supernatant removed. The particles were resuspended in fresh PBS buffer (1 mL) and agitation continued. The removed supernatant was then analysed using HPLC according the method described above. Figure 45 shows the total quantity of doxorubicin released from the microparticles between time points. The table shows that upon initial exposure to water, 0.9 mg of doxorubicin was released from 35 mg of doped particles. After 24 hours, a further 0.7 μg was released. Between days 2 to 3, and 3 to 6, approximately 0.3 μg and 0.2 μg of were released respectively. Figure 46 shows the cumulative release of doxorubicin from the nanoparticles, showing that even up to 9 days, doxorubicin was released, and apart from the initial burst of approx 0.8 μg, the release was continual over the time period shown.

12) Encapsulation of camptothecin

Camptothecin comes in two forms: 1) the lactone form with a high anti-cancer efficacy but which is sparingly soluble in water and 2) the carboxylate form which is highly soluble in water but which is clinically inactive. The transformation from one form to the other is pH dependent. When pH is greater than 4, the lactone form is transformed into the hydroxy acid (carboxylate) form, and below pH 4 the reverse reaction occurs.

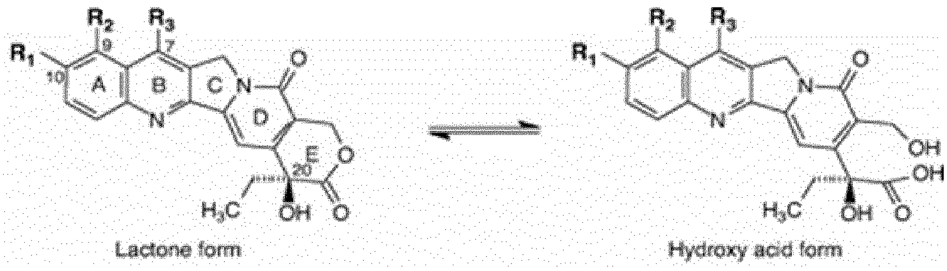

Lactone form    Hydroxy acid form

| | $R_1$ | $R_2$ | $R_3$ |
|---|---|---|---|
| Camptotecin | H | H | H |
| Topotecan | OH | $CH_2N(CH_3)_2$ | H |
| 9-Aminocamptotecin | H | $NH_2$ | H |
| Irinotecin | 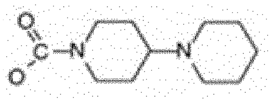 | H | $CH_2CH_3$ |
| SN-38 | OH | H | $CH_2CH_3$ |

The present invention relates to production of a practical drug delivery system. In order to achieve a high therapeutic index, it is desirable to encapsulate the hydrophobic lactone form of camptothecin. However the present invention is designed to encapsulate only hydrophilic molecules. To overcome this limitation the inventors designed the following alternative encapsulation procedure:

Camptothecin was dissolved in 0.1 mol/L sodium hydroxide solution with concentration 2 mg/mL. 1.08 mL of the above solution was added into 10 mmol NP-5 mixed with 50 mL cyclohexane to produce a microemulsion with camptothecin in the carboxylate form located in the water pools. A second microemulsion was produced using the typical synthesis process described in paragraph 1 and Fig. 27. After ageing 48 hours, the two emulsions were mixed, and subsequently stirred for 10 minutes. The mixed emulsions were then destabilised by a mixture of 100 mL cyclohexane and 100 mL acetone. When the resulting submicron particles settled to the bottom of the flask, they were washed 4 times using 50 mL cyclohexane and 50 mL acetone. Then 50 mL of $HNO_3$ 0.5 M or 0.1 M solution containing NaCl (such that the weight ratio between $SiO_2$ and NaCl was 15:85) was mixed with silica particles and the mixture stirred for 30 minutes. This acidification was designed to transform the encapsulated carboxylate form of the camptothecin into the lactone form inside the pores of the silica submicron particles. The resulting suspension was freeze-dried to produce a light yellow product suggesting the presence of camptothecin in its lactone form.

**13) Characterisation of the camptothecin release**

The release of camptothecin was analysed using HPLC. The HPLC system consisted of a Waters 1525 binary HPLC pump, in combination with a Waters 717 plus autosampler, and a Waters 2475 multi $\lambda$ fluorescence detector (excitation $\lambda$: 363 nm, emission $\lambda$: 550 nm). The HPLC column used was a Waters Atlantic™ dC-18, 5 $\mu$m, 4.6 mm $\times$ 150 mm column, with Waters Atlantic™ dC-18 guard column attached. The mobile phase was isocratic and consisted of 70% 0.075 M ammonium acetate buffer (pH 6.4) and 30 % acetonitrile to which tertiary butyl ammonium phosphate (TBAP) was added to a final concentration of 5 m*M*. The flow rate used was 0.8 mL/min. Camptothecin concentrations were determined by integration of the area under the curve and comparison to that of standard Camptothecin samples at 0.4, 0.8, 2, 4, and 10 $\mu$g/mL. The minimum acceptable $R^2$ for the standard curve was 0.9. Processing of HPLC spectra was performed using Waters Breeze™ software.

*Cumulative release*

The initial release studies of Camptothecin were performed using a cumulative release approach. Samples of Camptothecin encapsulated nanoparticles (1 g) were suspended in HPLC mobile phase with no TBAP added (30 mL), and stirred continuously. As the time points required the samples were centrifuged and an aliquot (100 $\mu$L) of supernatant isolated. The supernatant was analysed using HPLC to determine the concentration of Camptothecin. Figure 47 shows

the cumulative release of Camptothecin.

From the results shown in Figure 47, it can be seen that the total amount of Camptothecin released is approximately 3 μg, sustained over a period of up to 3 days. It is suggested that the amount of Camptothecin released from the particles is limited by the solubility of Camptothecin in aqueous conditions. It is known to that Camtothesin is only sparingly soluble in water.

*Release with aqueous phase replacement*

With the knowledge that Camptothecin is only sparingly soluble in water, an experiment was conducted in which the aqueous phase was replaced at each time point, to prevent the saturation of Camptothecin caused by its limited solubility. Samples of Camptothecin (33 mg) were suspended in the HPLC mobile phase with no TBAP added. At the time periods required the particles were pelleted by centrifugation, and the supernatant completely removed and analysed for Camptothecin concentration using HPLC. The particles were then resuspended in fresh mobile phase (1 mL) and stirring continued for the required time.

Figure 48 shows the cumulative quantity of Camptothecin released from the particles. The totally quantity released from the particles in this experiment is almost double the quantity release in the experiment detailed above. This indicates that the release of Camptothecin is dependent on its solubility in the liquid medium.

**14) Encapsulation of other molecules**

The following molecules have been encapsulated into silica particles using the acid destabilisation process: orange II (O-II); rhodamine-B (R-B); rhodamine-6G (R-6G); methyl-violet (M-V); copper (II) phthalocyanine-tetrasulfonic acid tetrasodium salt (CuPC); (tris(2,2 - bipyridyl) dichlororuthenium(II) hexahydrate (Rubpy); rhodamine-B isothiocyanate (RBITC). The encapsulation efficiency of each material may depend on one or more of:

1. the polarity of the material i.e. the solubility in water, cyclohexane, acetone, and chloroform;
2. its interaction with a silica matrix;
3. its molecular size ;
4. the existence of hydrophilic functional group such as hydroxyl which may form hydrogen bonding with silica surface;
5. other processing parameters such as the amount of actives, the volume of each solvents, etc.

The active material may be lost at two stages of the process: during destabilisation, due to dissolution in the acetone, and during washing of the particles. The later may be minimised by using a non-polar solvent for washing particles containing hydrophilic molecules.

**15) Alternative surfactants**

The syntheses were conducted with several alternative surfactants. The corresponding TEM micrographs are presented in Figure 49. Submicron particles were produced for the Tween surfactants but not for AOT (HLB 10-15), suggesting that the particles both the HLB and the presence of PEG unit plays a role in the successful destabilisation of the emulsion. Because the Tween surfactants produced unstable emulsions, TEMs of the particles prior to destabilisation were also recorded and are presented in the first column of Figure 49.

**Discussion:**

**1) Destabilisation of ORMOSIL/TMOS mixture**

The incorporation of ORMOSIL into the particle structure is important because, in addition to providing alternative hybrid matrices, it offers a possible way to increase the encapsulation efficiency by changing the pore size and internal structure of particles as well as providing chemical anchors for the molecules to encapsulate (e.g. amino groups from APTMS which can react with carboxylate groups in a dopant). However, as shown in Figure 29 and Figure 30, two ORMOSIL systems (GTES and APTMS) have been found to produce submicron particles and these had relatively low molar fractions (15% or less).

The two successful systems have a hydrophilic organic group attached to the silicon: amino propyl and glycidoxypropyl. In acid, the epoxy ring of the glycidoxypropyl group is hydrolysed and opens up according to the reaction:

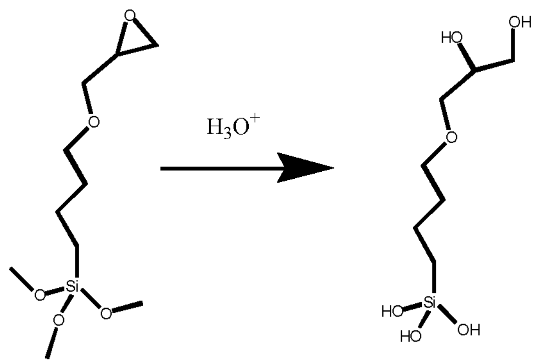

The fact that the alkyl and aryl substituted alkoxides (Methyl (MTMS), Phenyl (PTMS), Octyl (OTES)) did not produce any particles suggests that the organic group stabilises the emulsion (perhaps acting as a co-surfactant), thus preventing its destabilisation. The gel obtained using CHEETES suggests that the cyclohexenyl ethyl ligand decreases the stability of the emulsion, which results in a very rapid and uncontrolled destabilisation and the production of a dense gel.

The amount of organically modified silane influences the final morphology of the particles. Figure 30 shows that increasing the amount of GTMS leads to increasing necking and aggregation between the particles after destabilisation. A possible explanation for this is that the increasing numbers of opened glycidyl rings act as potential condensation points between particles, leading to an increased aggregation.

For APTMS, the particle size decreases with increasing amount of APTMS introduced. A possible explanation for this trend relates to the catalytic effect of the amino group on the sol-gel condensation, which leads to a faster nucleation (i.e. production of more nuclei) and hence less growth and smaller particles.

**2) Destabilisation of TEOS/TMOS mixture:**

As expected there is no significant difference for morphology for particles synthesised with pure TMOS/TEOS or with a mixture thereof During acid catalysed hydrolysis, whether starting from TEOS or TMOS, the same hydrolysed silica oligomers are produced.

**3) Destabilisation of a two emulsions system (i.e. 2 dyes)**

Several active materials may be encapsulated inside silica particles produced by the present process. The actives may be introduced either by mixing them initially inside the water pool followed by the addition of the silicon precursor, or by mixing several emulsion systems in which different active molecules in which different active molecules have been incorporated. The destabilisation of both systems leads to the production of submicron particles containing the different dyes although it is not certain at what the scale the dyes are dispersed. It is not known whether the destabilisation of a multiple emulsion leads to a molecular mixing and homogeneous distribution of the actives inside the submicron particles or whether the submicron particles contain nano-domains of concentration corresponding to the initial water pools of each emulsion prior to destabilisation.

**4) Destabilisation of an emulsion containing both seeds and monomers.**

The results in Figure 34 shows that 50 nm seeds synthesized in base may be successfully embedded inside micron- (and submicron) size particles using acid destabilisation. The acid catalysed hydrolysis of TMOS provides small oligomeric silica units which acts as glue between the based catalysed seeds during the droplet coalescence triggered by the introduction of acetone. This variation of the process is important as it may enable synthesis of particles with high drug loading using the base catalysed process and their transformation into microporous submicron particles, thus providing them with sustained release characteristics and overcoming the rapid diffusion characteristic of based synthesised particles.

Figure 36 shows that this concept can be expanded to hybrid materials seeds, although not all ORMOSIL particles produce submicron particles after destabilisation.

**5) Destabilisation of a two emulsions system one containing nanoparticles (made in Base) and the other one containing monomers (Acid)**

An alternative way to produce composite submicron particles containing mesoporous seeds dispersed inside a micorporous acid catalysed silica matrix is to mix two different emulsions, one containing the acid catalysed species and the other one the base catalysed particles, rapidly before destabilisation. As shown in Figure 37, this was not successful and a bimodal distribution was produced, with the initial seed particles entrapped into a colloidal gel of much smaller particles. This may be explained by the fact that, when the two emulsions are mixed together, exchange of the micellar cores leads to a rapid increase of the pH of the acid pool, which leads to a rapid condensation of the oligomers in these pools (acid-base synthesis) and the precipitation of small nanoparticles that rapidly gel.

**6) Introduction of ultrasonication during destabilization to decrease polydispersity**

Figure 40 shows that performing the destabilisation under ultrasonication is capable of providing a single mode, narrower and slightly smaller particles distribution. This contrasts with earlier findings which suggested that the method of addition did not have any influence over the particle size and morphology.

Two different factors are important to note: the previous analyses were performed using TEM, which is a poor method for analysing particle size distribution; and the ultrasound source used in the earlier experiment was a bath rather than the ultra-sound horn that that was used in the present experiment, which is much more powerful.

**7) Encapsulation and release of Doxorubicin in particles synthesized at different pH.**

Figure 38 illustrates how, by adjusting the internal pH during the synthesis, it is possible to change the pore size distribution and thus the release rate of the active. To demonstrate this, the inventors encapsulated doxorubicin at different pH and studied the release over several days at two pHs, 3.4 and 7.4, using HPLC (see figure 41 and 42). The interpretation of the results is rendered difficult by the rapid decomposition of doxorubicin during the release experiments. Nevertheless Figure 41 suggests that the release of the doxorubicin samples synthesised in acid differed from that synthesised at pH=7. The concentration of the doxorubicin monotonically decreased when encapsulated at pH=7 as it gradually increased for two to three days in the acid samples before decreasing after. The decrease in concentration appears to be due to the degradation of the doxorubicin with time, as shown in Figure 44. Furthermore, as shown in Figure 42, the generation of degradation product corresponds to the observed decrease in doxorubicin concentration. All this suggest that, when encapsulated at pH=7, the doxorubicin is released quasi-instantaneously and slowly degrades afterwards, as when it is encapsulated in more acidic pH, it releases more gradually. This is further confirmed by Figure 46 which shows an extended release over 6 days.

**8) Encapsulation of camptothecin**

Camptothecin was encapsulated and released both in the lactone and carboxylate form. Although, due to the HPLC procedure used, it is not possible to determine accurately the exact proportion of the two forms of the drug encapsulated inside the particles, a substantial proportion of the encapsulated campthothecin was in the lactone form.

The comparison between the quantity released in Figure 47 and Figure 48, shows that one of the factors limiting the release of campthothecin is its poor solubility in water, which led to a larger amount of campthothecin released when the supernatant water was changed at regular intervals (Figure 48). In this case after a steep initial release over two days a slower release took place over the next 4 days, showing the potential for sustained release of camptothecin for therapeutic applications.

**9) Alternative surfactants**

Tween 61 (HLB 9.6) and Tween 81 (HLB 10) produced unstable emulsions that generated submicron particles prior to destabilisation. Those submicron particles were of similar size to those obtained after acid destabilisation. The inventors hypothesize that in this case the submicron particle formation takes place prior to destabilisation. In the case of Tween 21 (HLB 13.3) a relatively stable emulsion formed (i.e. approximately transparent as compared to opaque for Tweens 61 and 81). After reacting for 3 days, the particles were centrifuged at 12000rpm for 20 min to recover a relatively small yield of product (relative to the normal yield after destabilisation). By TEM this product appeared to be large irregular aggregates and contained some gels. Only a few particles appeared spherical. The inventors hypothesize that when starting with a stable micro-emulsion, acid destabilisation is necessary to obtain submicron particles.

The inventors hypothesize that the number of oxyethylene units in the surfactant molecule may play an important role in controlling the production of submicron particles. In those surfactants which provided an acceptable product when

used in the process of the invention, the number of PEG is relatively small: 4 for Tween 21, 5 for NP5 and 6 for NP6 compared to 9 for NP9 and 7.5 for Triton X-114 (the latter two surfactants having been found unsuitable for use in the invention). Furthermore the number of oxyethylene units, (which form the polar head of the surfactant molecule) may influence the interaction between the polar head group of the surfactant and water which may control the coalescence process.

Although the number of oxyethylene units in the surfactant molecule plays a critical role in controlling the production of submicron particles, it is not sufficient to ensure proper destabilisation of the emulsion. This is exemplified by Brij 30, which possesses 4 oxyethylene units units but forms stable emulsions that do not produce submicron particles after destabilisation. Moreover, as mention earlier, in order to achieve successful destabilisation, the surfactant should have medium strength molecular interaction between its polar head and the water pool. This molecular interaction may be characterised by the surfactant footprint (A), which can be calculated by dividing the surface area of the water droplet surface ($\Pi * d^2$, where d is the water pool diameter) by the surfactant aggregation number (N).

$$A= (\Pi * d^2)/N$$

Using values from the literature, the footprint was calculated for a range of the surfactant that we used. The results are listed below. A medium interaction corresponds to a footprint between 1.5 and 10 $nm^2$ per molecule.

| | **Brij 30** | **NP-5** | **Triton X-100** | **AOT** |
|---|---|---|---|---|
| Aggregation Number | R=6.4: 150<br>R=1.61: 45<br>R=12.86: 362 | R=6: 210 | 140 | R=5: 60<br>R=10: 130 |
| Reverse micelle diameter | 3 nm (R=1.34)<br>6 nm (R=13.4) | R=6: 10 nm<br>(SANS result of ANSTO) 13 nm | 46.5 nm (R=5.5) | R=5: 4.8 nm<br>R=10: 6.6 nm |
| Foot print ($nm^2$/molecule) | **0.628** (R=1.61)<br>**0.312** (R=12.86) | **1.50** (10 nm size)<br>**2.55** (13 nm size) | **48.5** | **1.20** (R=5)<br>**1.05** (R=10) |
| Reference: | M. Vasilescu, et al. Adv. Colloid Interface Sci. 89-90, 169-194 (2001). | K. Osseo-Asare, et at., Colloids and Surfaces, 50, 321-339 (1990). Chia-Lu Chang, et al. Langmuir, 13, 3295-3307 (1997). | Robert J. Robson, et al. J. Phys. Chem. 1977; 81(11); 1075-1078. D. M. Zhu, et al. J. Phys. Chem., 1992,96,2382-2385. | F.J. Arriagada, K. Osseo-Asare J. Colloid Interface Sci. 170,8-17 (1995). |
| R: [water]/[surfactant] mol ratio | | | | |

It appears that surfactants with HLB between about 9 and about 14 and having between about 4 and 6 oxyethylene units and having a foot print between 1 and 5 $nm^2$ per molecules may be suitable for use in the present invention.

## Claims

1. A process for making a microporous particulate substance comprising a plurality of particles having a releasable substance releasably immobilised therein and/or thereon, said process comprising:

   - providing a precursor emulsion comprising droplets dispersed in a continuous liquid phase, wherein at least some of the droplets comprise a condensable species; and
   - forming nuclei from the condensable species within the droplets such that at least some of the droplets comprise the condensable species or an at least partial condensate thereof;

   so as to provide an emulsion comprising droplets, said droplets comprising a releasable substance and being dispersed in a continuous liquid phase, wherein at least some of the droplets comprise the nuclei; and

   - at least partly destabilising the droplets to form a plurality of particles of the microporous particulate substance,

said plurality of particles having said releasable substance releasably immobilised therein and/or thereon,

wherein the particles of the particulate substance are between 30 and 1000nm in diameter, said particles having micropores of less than 1nm diameter, together with mesopores of between 1 and 50nm.

**2.** The process of claim 1 wherein the step of at least partly destabilising the droplets comprises at least partly coalescing the droplets.

**3.** The process of claim 1 wherein the step of at least partially destabilising the droplets comprises combining a coalescing liquid and the emulsion.

**4.** The process of claim 3 wherein the coalescing liquid comprises a destabilising liquid and a non-polar liquid.

**5.** The process of claim 4 wherein the destabilising liquid is acetone or ethanol or a mixture of acetone and ethanol.

**6.** The process of claim 1 wherein at least some of the droplets comprise a condensable species.

**7.** The process of claim 6 wherein the condensable species is derived from a tetralkoxysilane, a trialkoxysilane or a mixture thereof, or comprises a silicate, a polysilicate or a mixture thereof.

**8.** The process of claim 1 comprising the steps of:

- providing a second emulsion comprising droplets dispersed in a continuous liquid phase, wherein at least some of the droplets comprise nuclei, said droplets comprising a second releasable substance; and
- combining the emulsion and the second emulsion;

said steps being conducted prior to the step of at least partially destabilising the droplets, wherein the step of at least partially destabilising comprises at least partially destabilising the droplets of the emulsion and of the second emulsion, said process making the particulate substance, said particulate substance comprising particles comprising the releasable substance and the second releasable substance.

**9.** The process of claim 1 wherein the releasable substance is a drug.

**10.** The process of claim 9 wherein the drug is an anticancer drug.

**11.** The process of claim 1 wherein the emulsion is a microemulsion.

**12.** The process of claim 1 wherein the emulsion is a water in oil emulsion.

**13.** The process of claim 1 wherein the step of forming the nuclei comprises ageing the precursor emulsion for sufficient time for formation of the nuclei from the condensable species.

**14.** The process of claim 1 wherein the process of providing the precursor emulsion comprises the steps of:

- providing an emulsion comprising emulsion droplets dispersed in a continuous liquid phase,
- adding a hydrolysable species to the emulsion; and
- at least partially hydrolysing the hydrolysable species within the emulsion droplets to form the condensable species.

**15.** The process of claim 14 wherein the hydrolysable species comprises a tetralkoxysilane.

**16.** The process of claim 15 wherein the hydrolysable species is tetramethoxysilane or tetraethoxysilane.

**17.** The process of claim 1 wherein the process of providing the emulsion comprises the steps of:

- providing a basic emulsion, said emulsion comprising emulsion droplets dispersed in a continuous liquid phase,
- adding a first hydrolysable species to the emulsion;
- at least partially hydrolysing the first hydrolysable species within the emulsion droplets to form the condensable

species;
- acidifying the emulsion to form an acidified emulsion; and
- adding a second hydrolysable species to the acidified emulsion to form the emulsion.

**18.** The process of claim 17 additionally comprising adding a surfactant and a solvent to the acidified emulsion.

**19.** The process of claim 17 additionally comprising ageing the acidified emulsion for sufficient time to at least partially hydrolyse the second hydrolysable species.

**20.** The process of claim 1 wherein the droplets are acidic.

**21.** The process of claim 1 wherein the nuclei are porous.

**22.** A microporous particulate substance comprising a plurality of particles having a releasable substance releasably immobilised therein and/or theron wherein the particles of the particulate substance are between 30 and 1000nm in diameter, said particles having micropores of less than 1nm diameter, together with mesopores of between 1 and 50nm.

**Patentansprüche**

**1.** Verfahren zur Herstellung einer mikroporösen, teilchenförmigen Substanz, umfassend eine Vielzahl von Teilchen, die eine freisetzbare Substanz in freisetzbarer Weise darin und/oder darauf immobilisiert haben, wobei das Verfahren umfasst:

- Vorsehen einer Vorläuferemulsion, umfassend Teilchen, dispergiert in einer kontinuierlichen flüssigen Phase, wobei mindestens einige der Tröpfchen eine kondensierbare Spezies umfassen; und
- Bilden von Nuklei aus der kondensierbaren Spezies innerhalb der Tröpfchen, sodass mindestens einige der Tröpfchen die kondensierbare Spezies oder ein mindestens teilweises Kondensat davon umfassen;

um so eine Tröpfchen umfassende Emulsion vorzusehen, wobei die Tröpfchen eine freisetzbare Substanz umfassen und in einer kontinuierlichen flüssigen Phase dispergiert sind, wobei mindestens einige der Tröpfchen die Nuklei umfassen; und

- mindestens teilweises Destabilisieren der Tröpfchen zur Bildung einer Vielzahl von Teilchen der mikroporösen, teilchenförmigen Substanz, wobei die Vielzahl der Teilchen die freisetzbare Substanz in freisetzbarer Weise darin und/oder darauf immobilisiert haben,

wobei die Teilchen der teilchenförmigen Substanz einen Durchmesser zwischen 30 und 1000 nm aufweisen, wobei die Teilchen Mikroporen von weniger als 1 nm Durchmesser, zusammen mit Mesoporen zwischen 1 und 50 nm aufweisen.

**2.** Verfahren nach Anspruch 1, wobei der Schritt des mindestens teilweisen Destabilisierens der Tröpfchen mindestens teilweises Koaleszieren der Tröpfchen umfasst.

**3.** Verfahren nach Anspruch 1, wobei der Schritt des mindestens teilweisen Destabilisierens der Tröpfchen das Kombinieren einer koaleszierenden Flüssigkeit und der Emulsion umfasst.

**4.** Verfahren nach Anspruch 3, wobei die koaleszierende Flüssigkeit eine destabilisierende Flüssigkeit und eine nichtpolare Flüssigkeit umfasst.

**5.** Verfahren nach Anspruch 4, wobei die destabilisierende Flüssigkeit Aceton oder Ethanol oder eine Mischung aus Aceton und Ethanol ist.

**6.** Verfahren nach Anspruch 1, wobei mindestens einige der Tröpfchen eine kondensierbare Spezies umfassen.

**7.** Verfahren nach Anspruch 6, wobei die kondensierbare Spezies aus einem Tetralkoxysilan, einem Trialkoxysilan oder einer Mischung davon abgeleitet ist oder ein Silikat, ein Polysilikat oder eine Mischung davon umfasst.

**8.** Verfahren nach Anspruch 1, umfassend die Schritte:

- Vorsehen einer zweiten Emulsion, umfassend Tröpfchen, dispergiert in einer kontinuierlichen flüssigen Phase, wobei mindestens einige der Tröpfchen Nuklei umfassen, wobei die Tröpfchen eine zweite freisetzbare Substanz umfassen; und
- Kombinieren der Emulsion und der zweiten Emulsion;

wobei die Schritte vor dem Schritt des mindestens teilweisen Destabilisierens der Tröpfchen durchgeführt werden, wobei der Schritt des mindestens teilweisen Destabilisierens das mindestens teilweise Destabilisieren der Tröpfchen der Emulsion und der zweiten Emulsion umfasst, wobei das Verfahren die teilchenförmige Substanz erzeugt, wobei die teilchenförmige Substanz Teilchen umfasst, umfassend die freisetzbare Substanz und die zweite freisetzbare Substanz.

**9.** Verfahren nach Anspruch 1, wobei die freisetzbare Substanz ein Arzneimittel ist.

**10.** Verfahren nach Anspruch 9, wobei das Arzneimittel ein Antikrebs-Arzneimittel ist.

**11.** Verfahren nach Anspruch 1, wobei die Emulsion eine Mikroemulsion ist.

**12.** Verfahren nach Anspruch 1, wobei die Emulsion eine Wasser-in-Öl-Emulsion ist.

**13.** Verfahren nach Anspruch 1, wobei der Schritt des Bildens der Nuklei das Altern der Vorläuferemulsion über eine ausreichende Zeit umfasst, zur Bildung der Nuklei aus der kondensierbaren Spezies.

**14.** Verfahren nach Anspruch 1, wobei das Verfahren des Vorsehens der Vorläuferemulsion die Schritte umfasst:

- Vorsehen einer Emulsion, umfassend Emulsionströpfchen, dispergiert in einer kontinuierlichen flüssigen Phase,
- Zugeben einer hdydrolysierbaren Spezies zu der Emulsion; und
- mindestens teilweises Hydrolysieren der hydrolysierbaren Spezies innerhalb der Emulsionströpfchen zur Bildung der kondensierbaren Spezies.

**15.** Verfahren nach Anspruch 14, wobei die hydrolysierbare Spezies ein Tetralkoxysilan umfasst.

**16.** Verfahren nach Anspruch 15, wobei die hydrolysierbare Spezies Tetramethoxysilan oder Tetraethoxysilan ist.

**17.** Verfahren nach Anspruch 1, wobei das Verfahren des Vorsehens der Emulsion die Schritte umfasst:

- Vorsehen einer basischen Emulsion, wobei die Emulsion Emulsionströpfchen, dispergiert in einer kontinuierlichen flüssigen Phase umfasst,
- Zugeben einer ersten hydrolysierbaren Spezies zu der Emulsion;
- mindestens teilweises Hydrolysieren der ersten hydrolisierbaren Spezies innerhalb der Emulsionströpfchen zur Bildung der kondensierbaren Spezies;
- Ansäuern der Emulsion zur Bildung einer angesäuerten Emulsion; und
- Zugeben einer zweiten hydrolysierbaren Spezies zu der angesäuerten Emulsion zur Bildung der Emulsion.

**18.** Verfahren nach Anspruch 17, zusätzlich umfassend das Zugeben eines Tensids und eines Lösungsmittels zu der angesäuerten Emulsion.

**19.** Verfahren nach Anspruch 17, zusätzlich umfassend das Altern der angesäuerten Emulsion über eine ausreichende Zeit, um mindestens teilweise die zweite hydrolysierbare Spezies zu hydrolysieren.

**20.** Verfahren nach Anspruch 1, wobei die Tröpfchen sauer sind.

**21.** Verfahren nach Anspruch 1, wobei die Nuklei porös sind.

**22.** Mikroporöse, teilchenförmige Substanz, umfassend eine Vielzahl von Teilchen mit einer freisetzbaren Substanz, die freisetzbar darin und/oder darauf immobilisiert ist, wobei die Teilchen der teilchenförmigen Substanz einen

Durchmesser zwischen 30 und 1000 nm aufweisen, wobei die Teilchen Mikroporen von weniger als 1 nm Durchmesser, zusammen mit Mesoporen zwischen 1 und 50 nm aufweisen.

## Revendications

1. Procédé de préparation d'une substance particulaire microporeuse comprenant une pluralité de particules présentant une substance libérable immobilisée dans celles-ci et/ou sur celles-ci de manière à pouvoir être libérée, ledit procédé comprenant :

   - l'apport d'une émulsion précurseur comprenant des gouttelettes dispersées dans une phase liquide continue, au moins certaines des gouttelettes comprenant une espèce condensable ; et
   - la formation de noyaux à partir de l'espèce condensable à l'intérieur des gouttelettes de manière à ce qu'au moins certaines des gouttelettes comprennent l'espèce condensable ou un condensé au moins partiel de celle-ci ;

   de manière à obtenir une émulsion comprenant des gouttelettes, lesdites gouttelettes comprenant une substance libérable et étant dispersées dans une phase liquide continue, au moins certaines des gouttelettes comprenant les noyaux ; et

   - la déstabilisation au moins partielle des gouttelettes pour former une pluralité de particules de la substance particulaire microporeuse, ladite pluralité de particules présentant ladite substance libérable immobilisée dans celles-ci et/ou sur celles-ci de manière à pouvoir être libérée,

   dans lequel les particules de la substance particulaire ont un diamètre entre 30 et 1 000 nm, lesdites particules comportant des micropores d'un diamètre inférieur à 1 nm, conjointement avec des mésopores ayant une taille située entre 1 et 50 nm.

2. Procédé selon la revendication 1 dans lequel l'étape de déstabilisation au moins partielle des gouttelettes comprend la coalescence au moins partielle des gouttelettes.

3. Procédé selon la revendication 1 dans lequel l'étape de déstabilisation au moins partielle des gouttelettes comprend la combinaison d'un liquide coalescent et de l'émulsion.

4. Procédé selon la revendication 3 dans lequel le liquide coalescent comprend un liquide déstabilisant et un liquide non polaire.

5. Procédé selon la revendication 4 dans lequel le liquide déstabilisant est l'acétone ou l'éthanol ou un mélange d'acétone et d'éthanol.

6. Procédé selon la revendication 1 dans lequel au moins certaines des gouttelettes comprennent une espèce condensable.

7. Procédé selon la revendication 6 dans lequel l'espèce condensable est dérivée d'un tétralcoxysilane, d'un trialcoxysilane ou un mélange de ceux-ci, ou comprend un silicate, un polysilicate ou un mélange de ceux-ci.

8. Procédé selon la revendication 1 comprenant les étapes suivantes :

   - l'apport d'une seconde émulsion comprenant des gouttelettes dispersées dans une phase liquide continue, au moins certaines des gouttelettes comprenant des noyaux, lesdites gouttelettes comprenant une seconde substance libérable ; et
   - la combinaison de l'émulsion et de la seconde émulsion ;

   lesdites étapes étant réalisées avant l'étape de déstabilisation au moins partielle des gouttelettes, l'étape de déstabilisation au moins partielle comprenant la déstabilisation au moins partielle des gouttelettes de l'émulsion et de la seconde émulsion, ledit procédé de préparation de la substance particulaire, ladite substance particulaire comprenant des particules comprenant la substance libérable et la seconde substance libérable.

**9.** Procédé selon la revendication 1 dans lequel la substance libérable est un médicament.

**10.** Procédé selon la revendication 9 dans lequel le médicament est un médicament anticancéreux.

**11.** Procédé selon la revendication 1 dans lequel l'émulsion est une microémulsion.

**12.** Procédé selon la revendication 1 dans lequel l'émulsion est une émulsion eau dans l'huile.

**13.** Procédé selon la revendication 1 dans lequel l'étape de formation des noyaux comprend le vieillissement de l'émulsion précurseur pendant une durée suffisante pour permettre la formation des noyaux à partir de l'espèce condensable.

**14.** Procédé selon la revendication 1 dans lequel le procédé d'apport de l'émulsion précurseur comprend les étapes suivantes :

- l'apport d'une émulsion comprenant des gouttelettes d'émulsion dispersées dans une phase liquide continue,
- l'ajout d'une espèce hydrolysable à l'émulsion ; et
- l'hydrolyse au moins partielle de l'espèce hydrolysable dans les gouttelettes d'émulsion pour former l'espèce condensable.

**15.** Procédé selon la revendication 14 dans lequel l'espèce hydrolysable comprend un tétralcoxysilane.

**16.** Procédé selon la revendication 15 dans lequel l'espèce hydrolysable est le tétraméthoxysilane ou le tétraéthoxysilane.

**17.** Procédé selon la revendication 1 dans lequel le procédé d'apport de l'émulsion comprend les étapes suivantes :

- l'apport d'une émulsion basique, ladite émulsion comprenant des gouttelettes d'émulsion dispersées dans une phase liquide continue,
- l'ajout d'une première espèce hydrolysable à l'émulsion ;
- l'hydrolyse au moins partielle de la première espèce hydrolysable dans les gouttelettes d'émulsion pour former l'espèce condensable ;
- l'acidification de l'émulsion pour former une émulsion acidifiée ; et
- l'ajout d'une seconde espèce hydrolysable à l'émulsion acidifiée pour former l'émulsion.

**18.** Procédé selon la revendication 17 comprenant en outre l'ajout d'un tensioactif et d'un solvant à l'émulsion acidifiée.

**19.** Procédé selon la revendication 17 comprenant en outre le vieillissement de l'émulsion acidifiée pendant une durée suffisante pour au moins partiellement hydrolyser la seconde espèce hydrolysable.

**20.** Procédé selon la revendication 1 dans lequel les gouttelettes sont acides.

**21.** Procédé selon la revendication 1 dans lequel les noyaux sont poreux.

**22.** Substance particulaire microporeuse comprenant une pluralité de particules présentant une substance libérable immobilisée dans celles-ci et/ou sur celles-ci de manière à pouvoir être libérée dans laquelle les particules de la substance particulaire ont un diamètre entre 30 et 1 000 nm, lesdites particules comportant des micropores d'un diamètre inférieur à 1 nm, conjointement avec des mésopores ayant une taille située entre 1 et 50 nm.

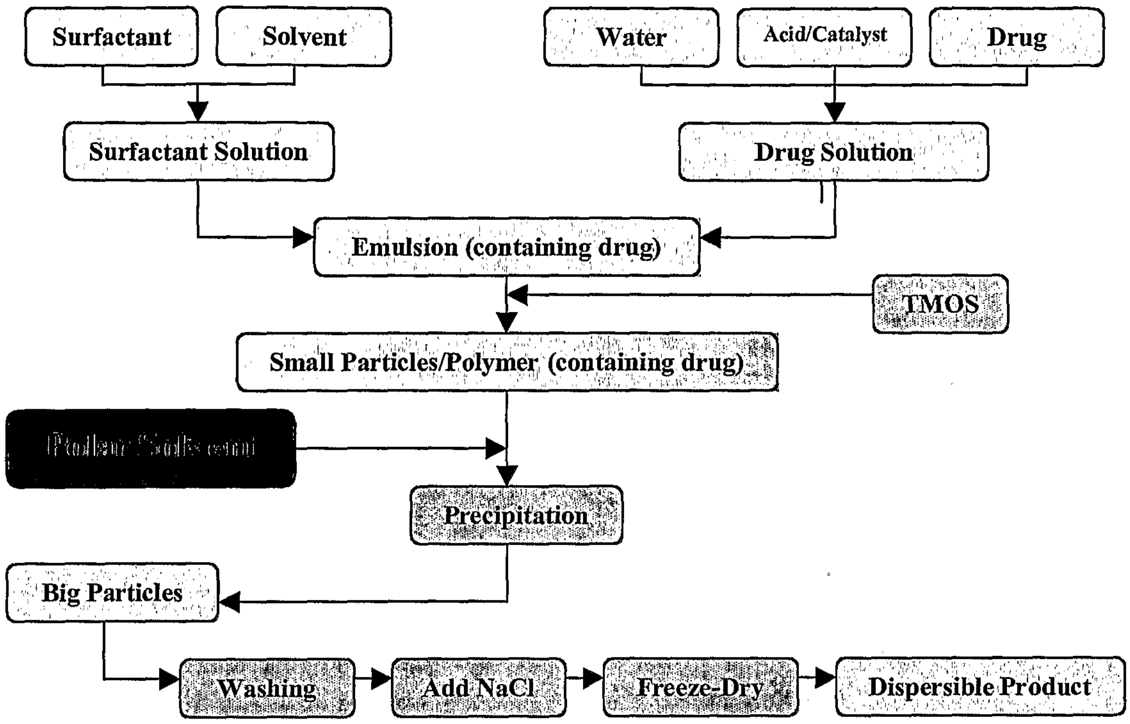
Surfactant
Solvent
Water
Acid/Catalyst
Drug
Surfactant Solution
Drug Solution
Emulsion (containing drug)
TMOS
Small Particles/Polymer (containing drug)
Polar Solvent
Precipitation
Big Particles
Washing
Add NaCl
Freeze-Dry
Dispersible Product

Fig. 1

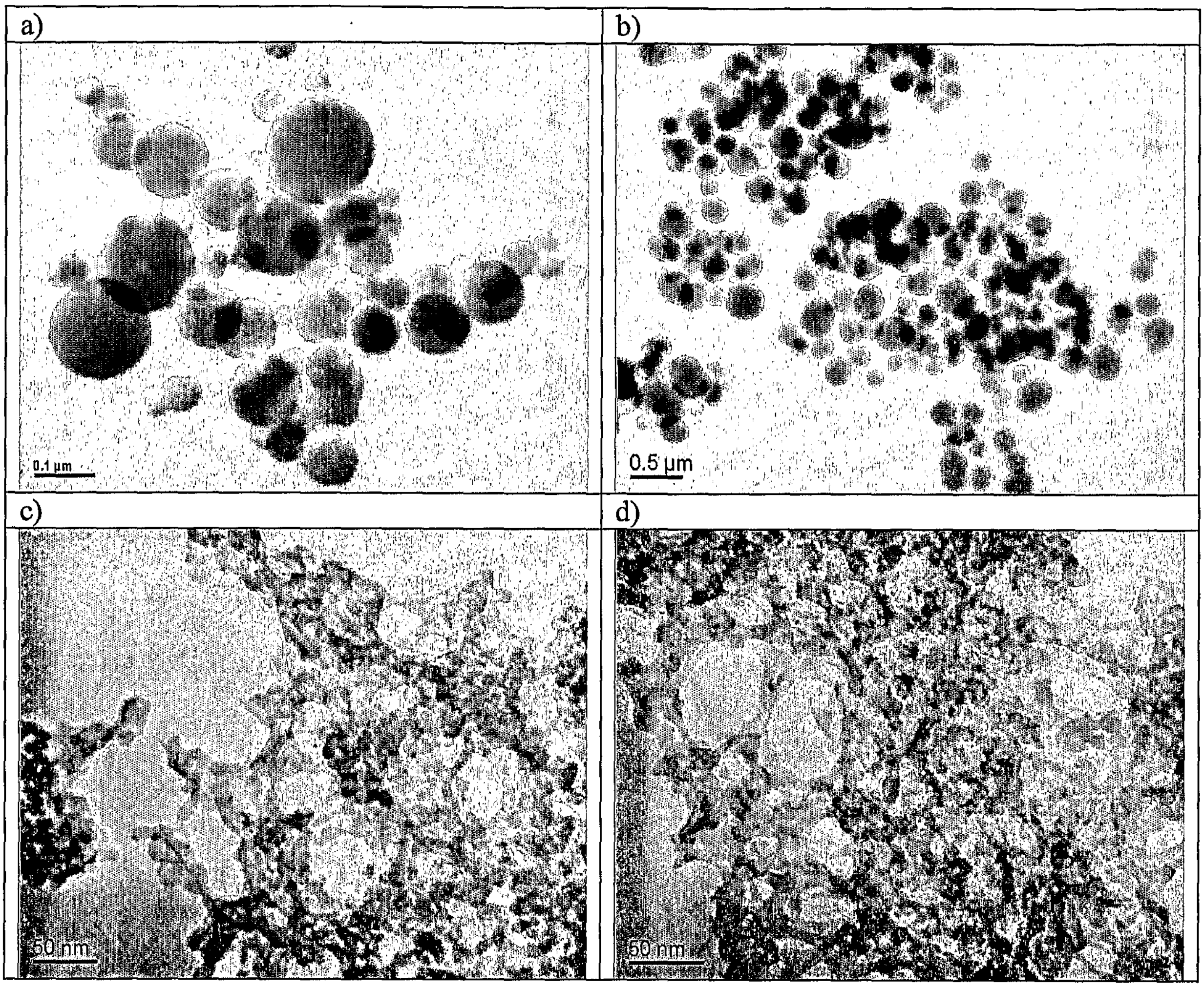


Fig. 2

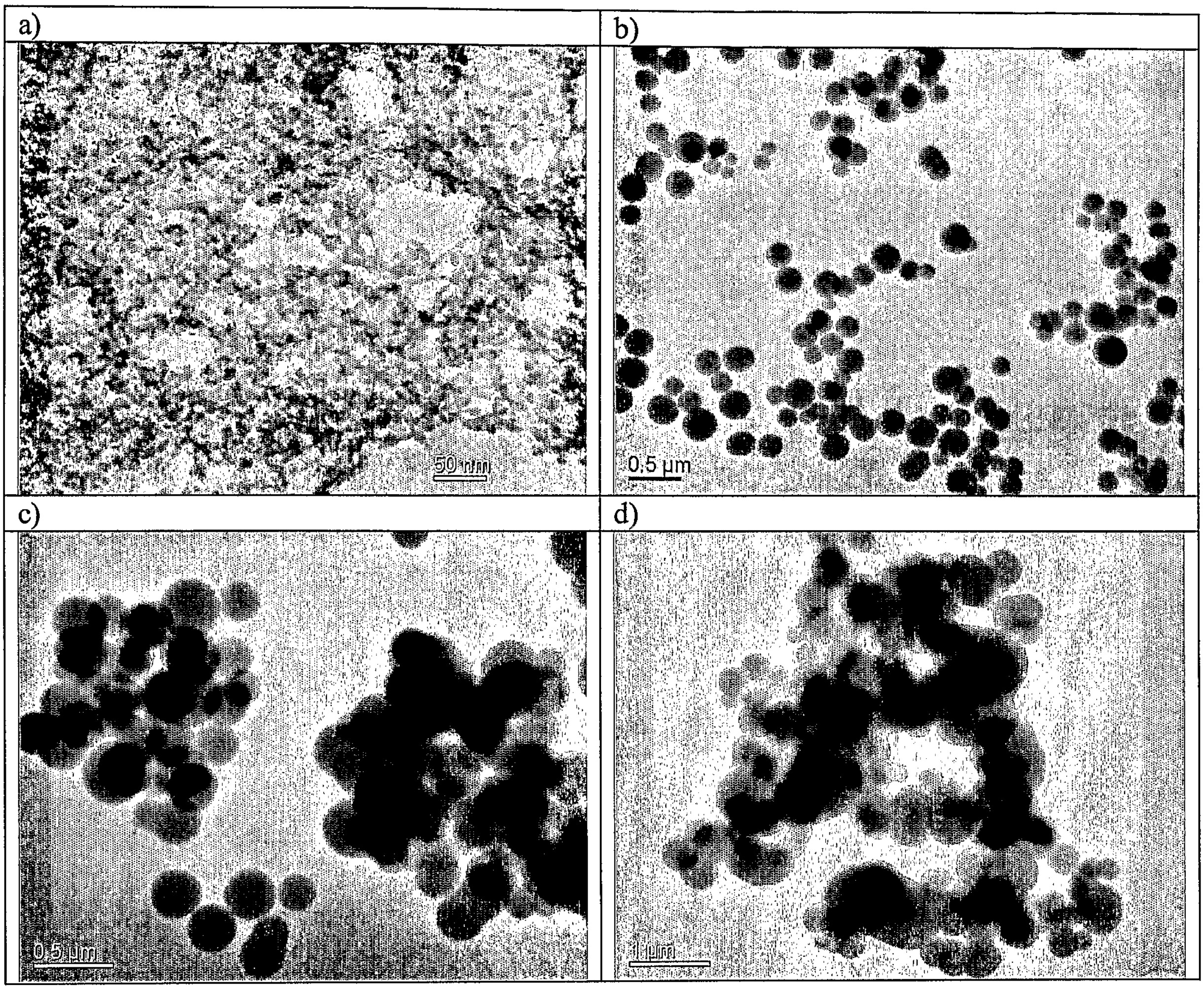


Fig. 3

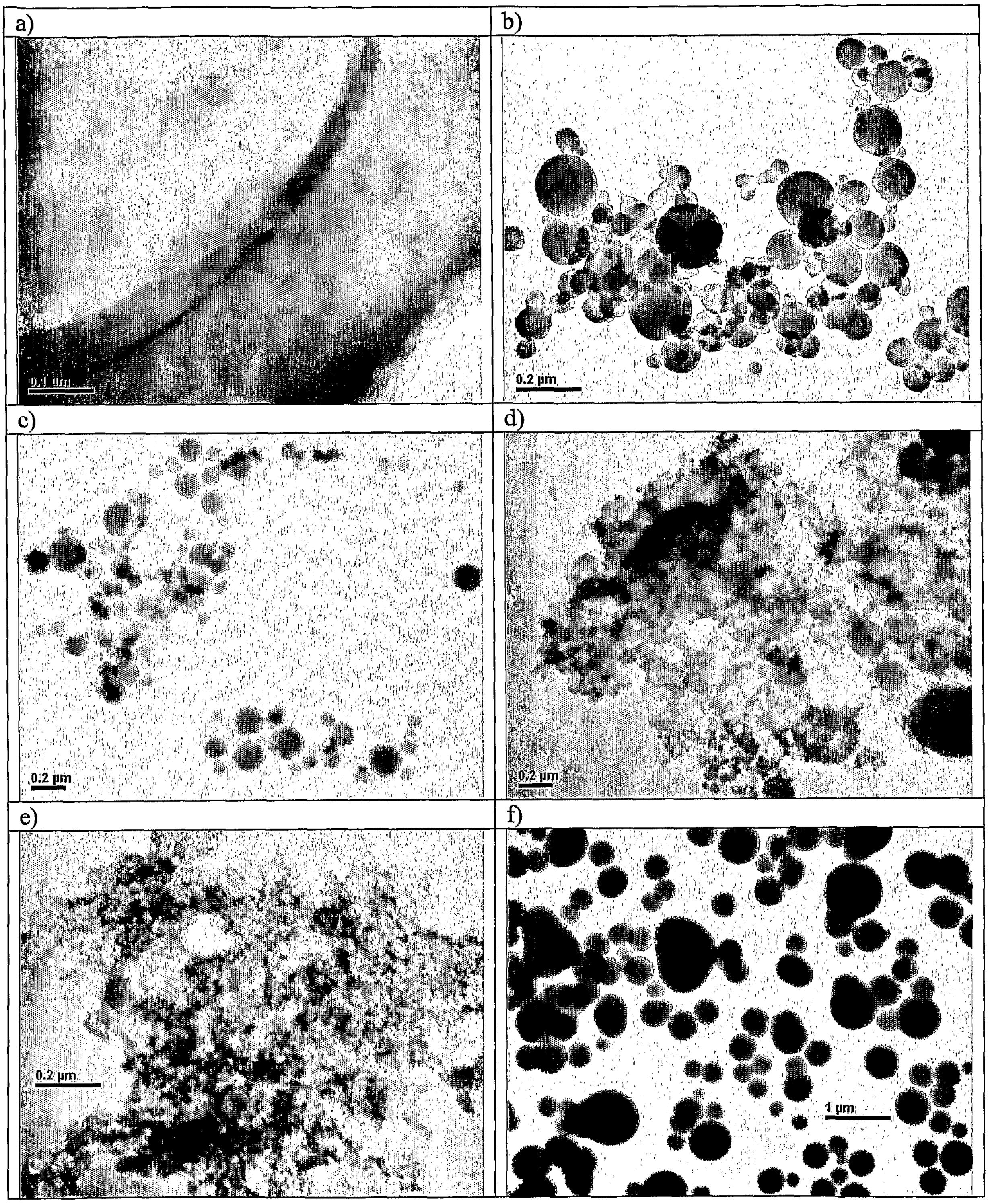


Fig. 4

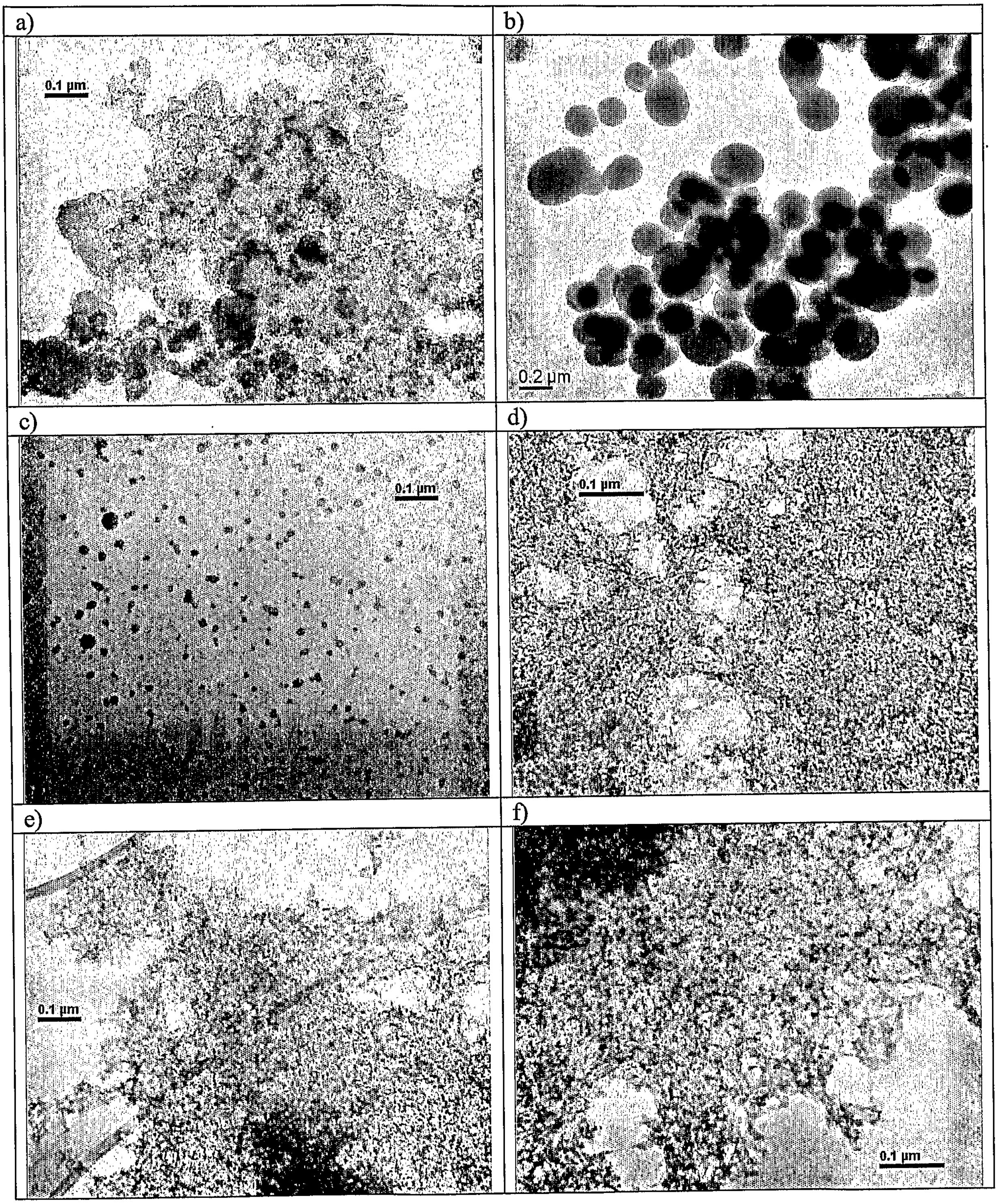


Fig. 5

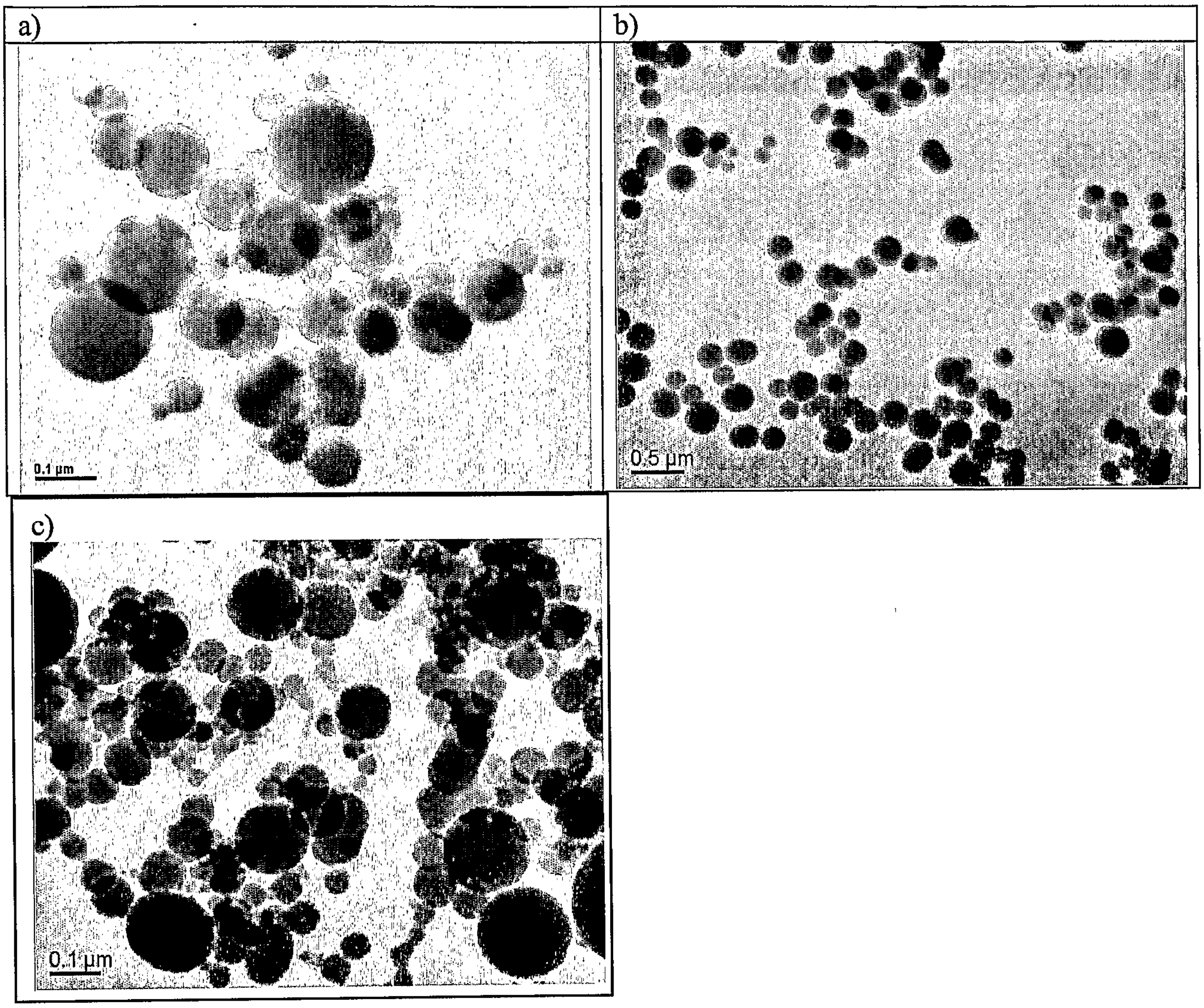


Fig. 6

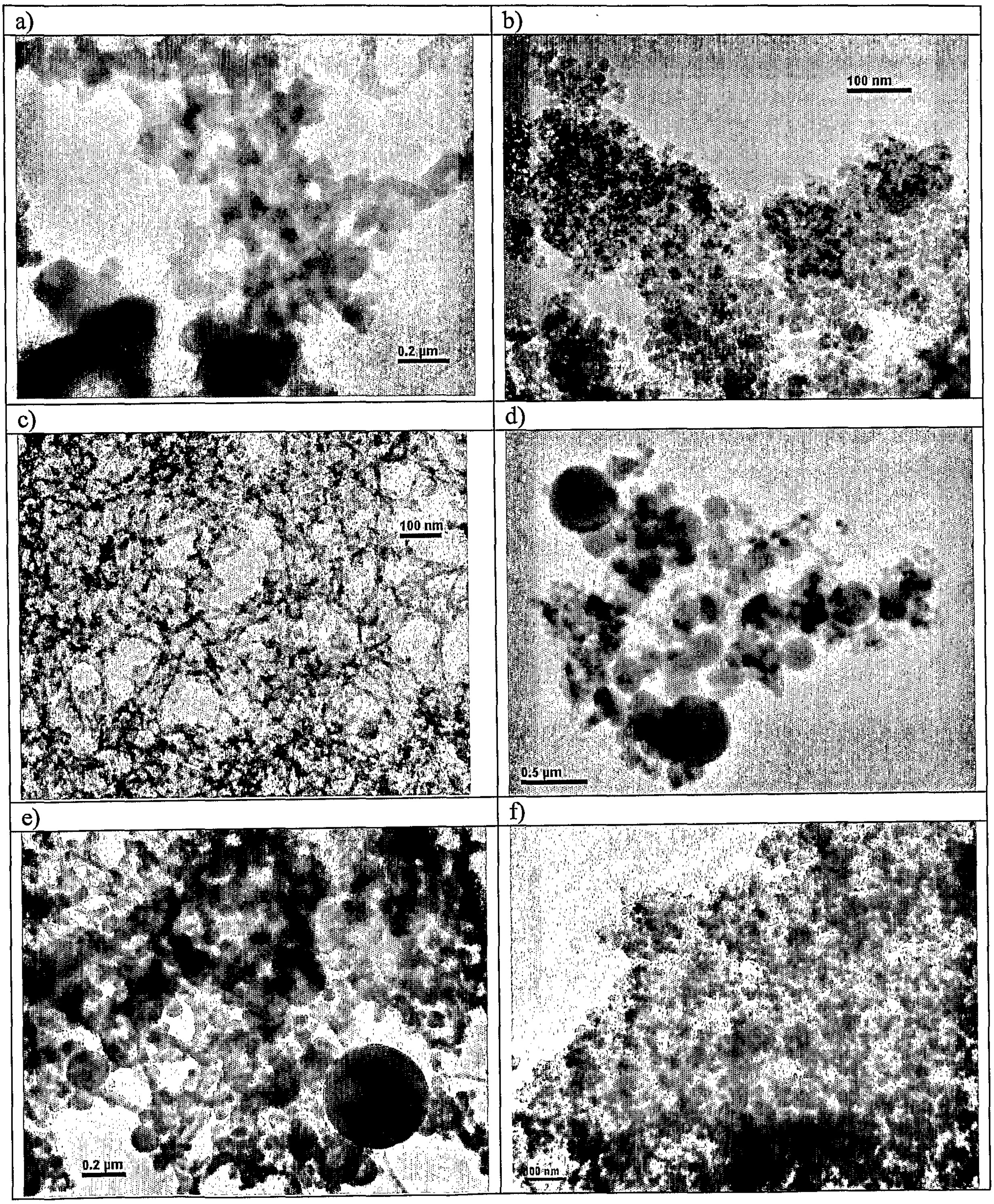


Fig. 7

| a) | b) |
|---|---|
| 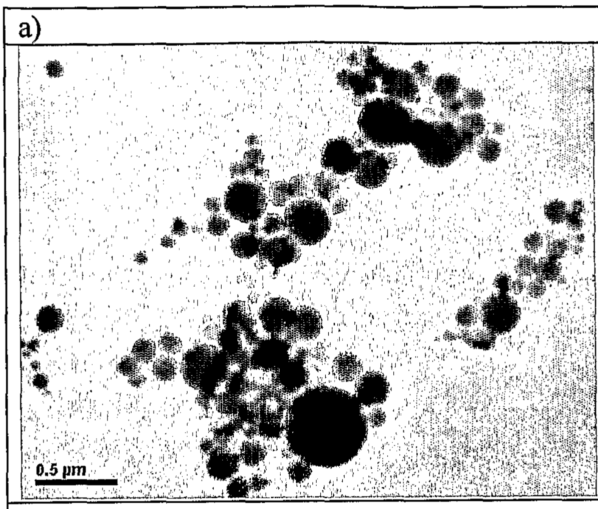  | 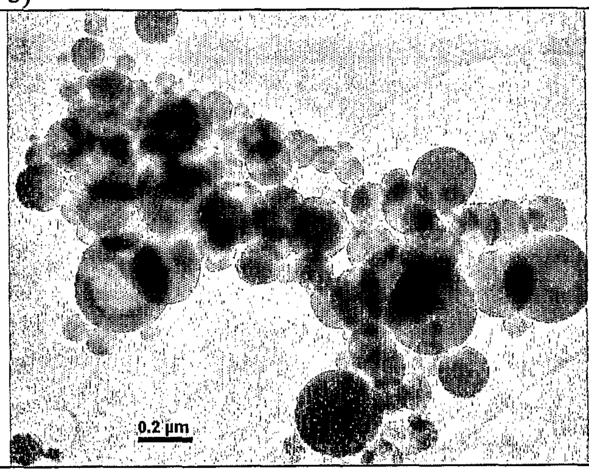  |
| c) | d) |
| Form gel | Form gel |
| e) | f) |
| Form gel | Form gel |

**Fig. 8**

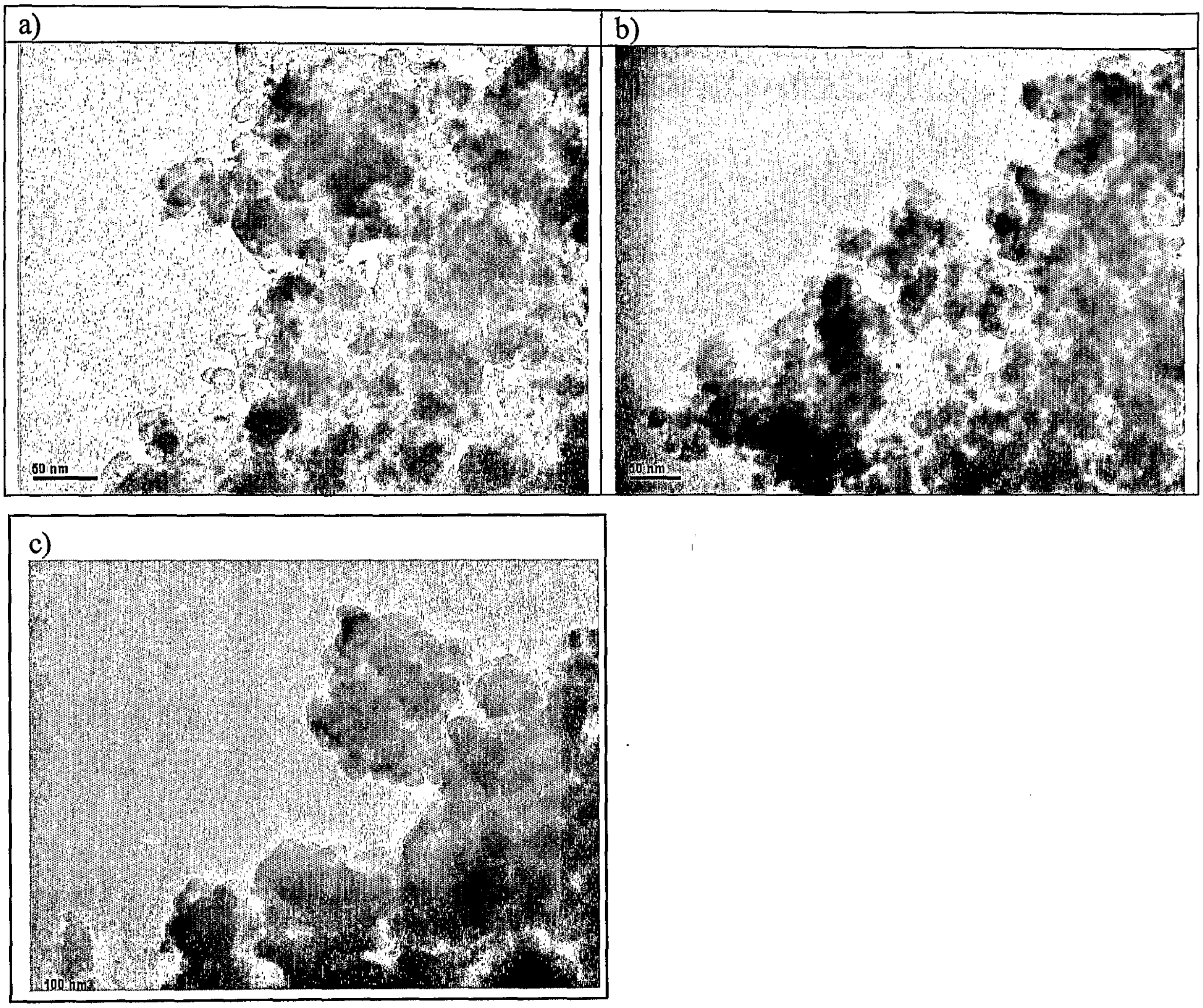


Fig. 9

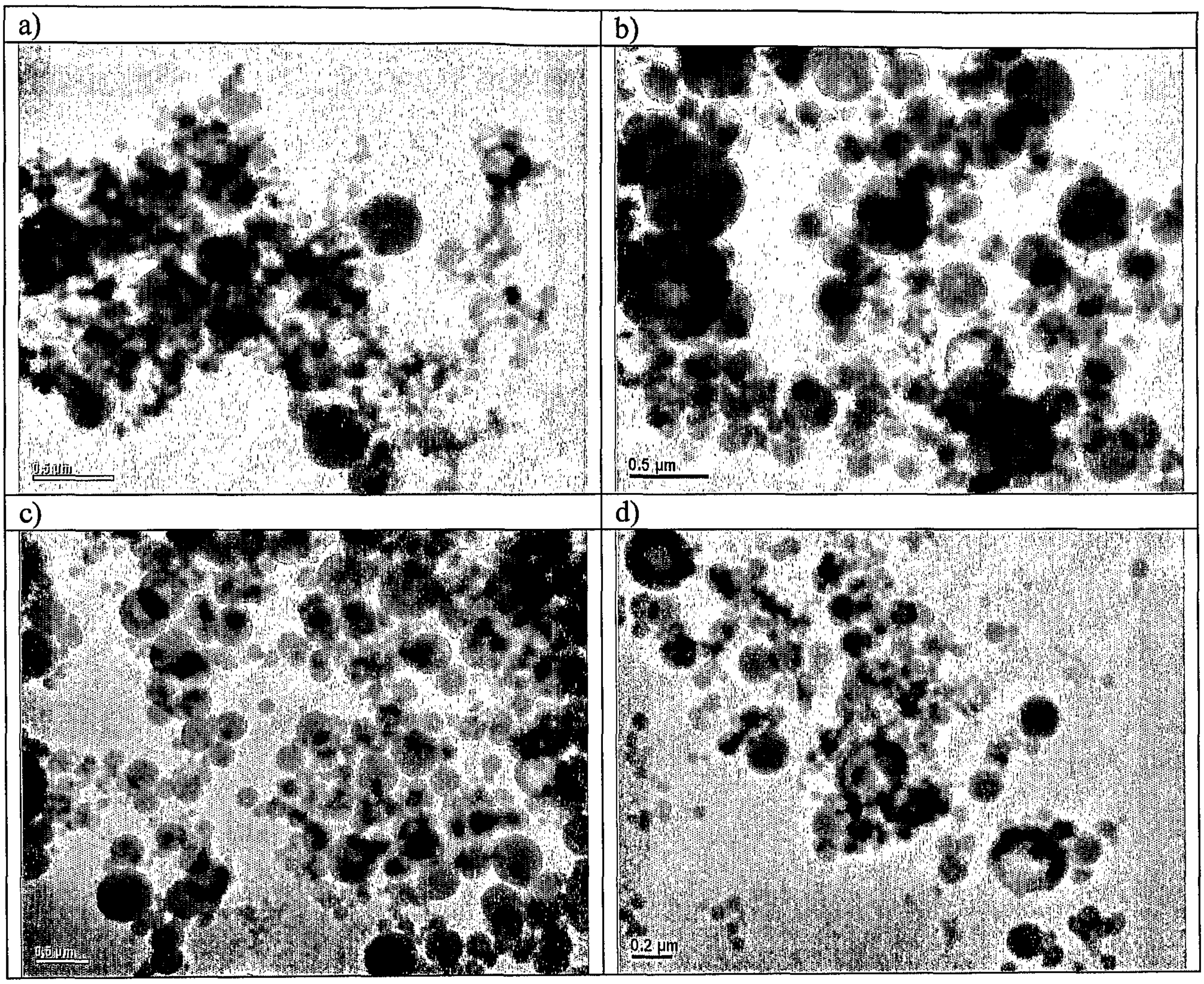


Fig. 10

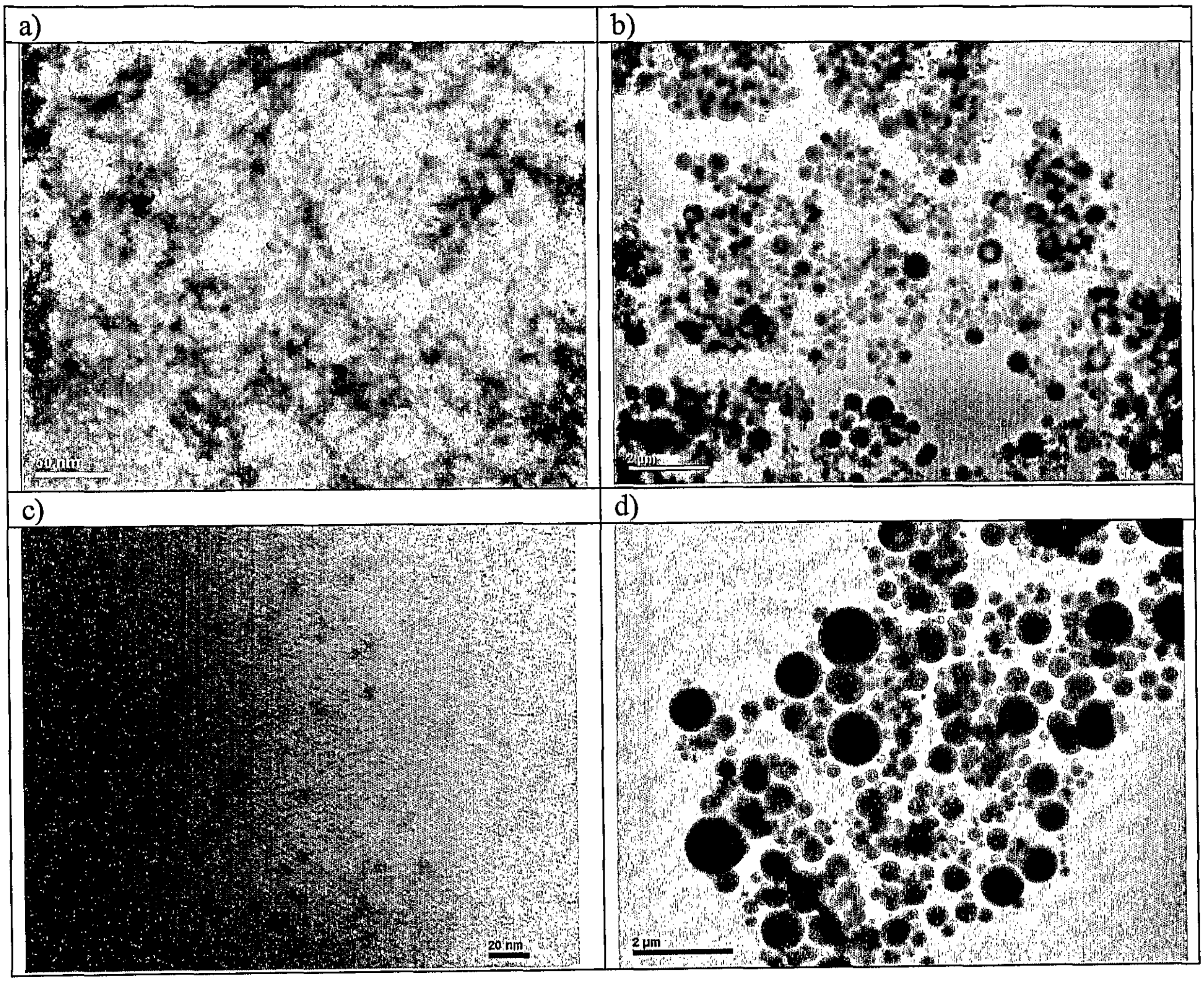


Fig. 11

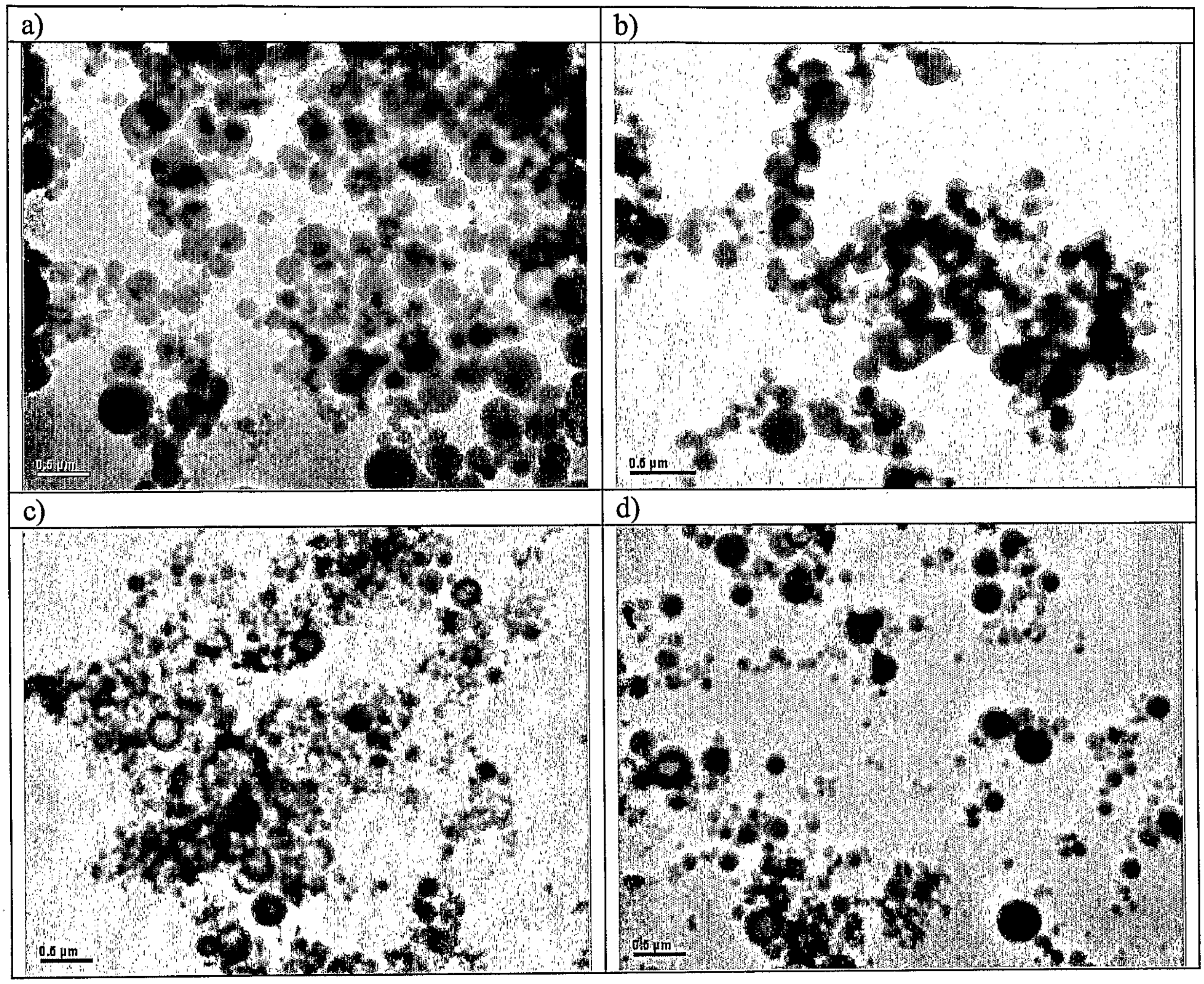


Fig. 12

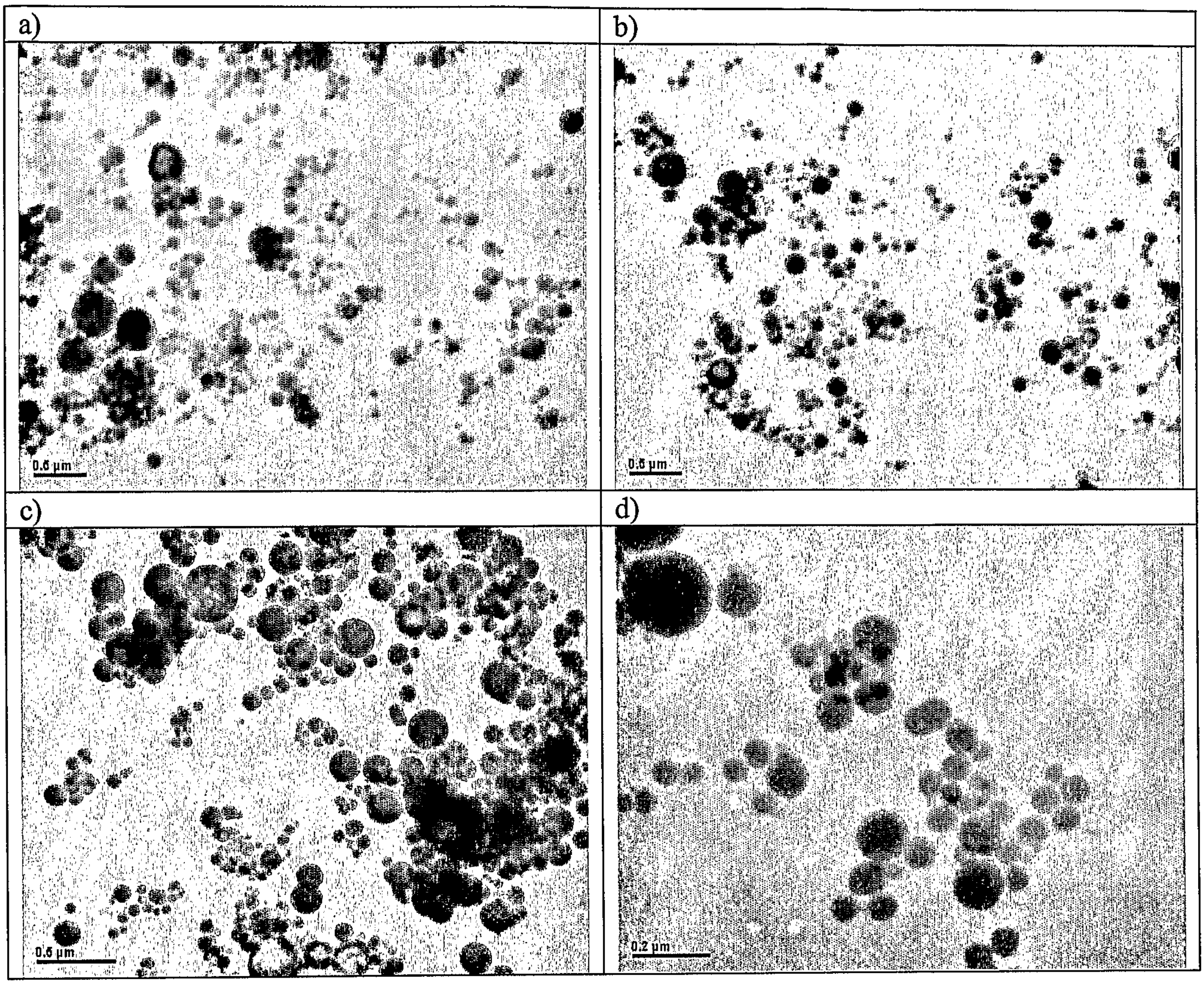


**Fig. 13**

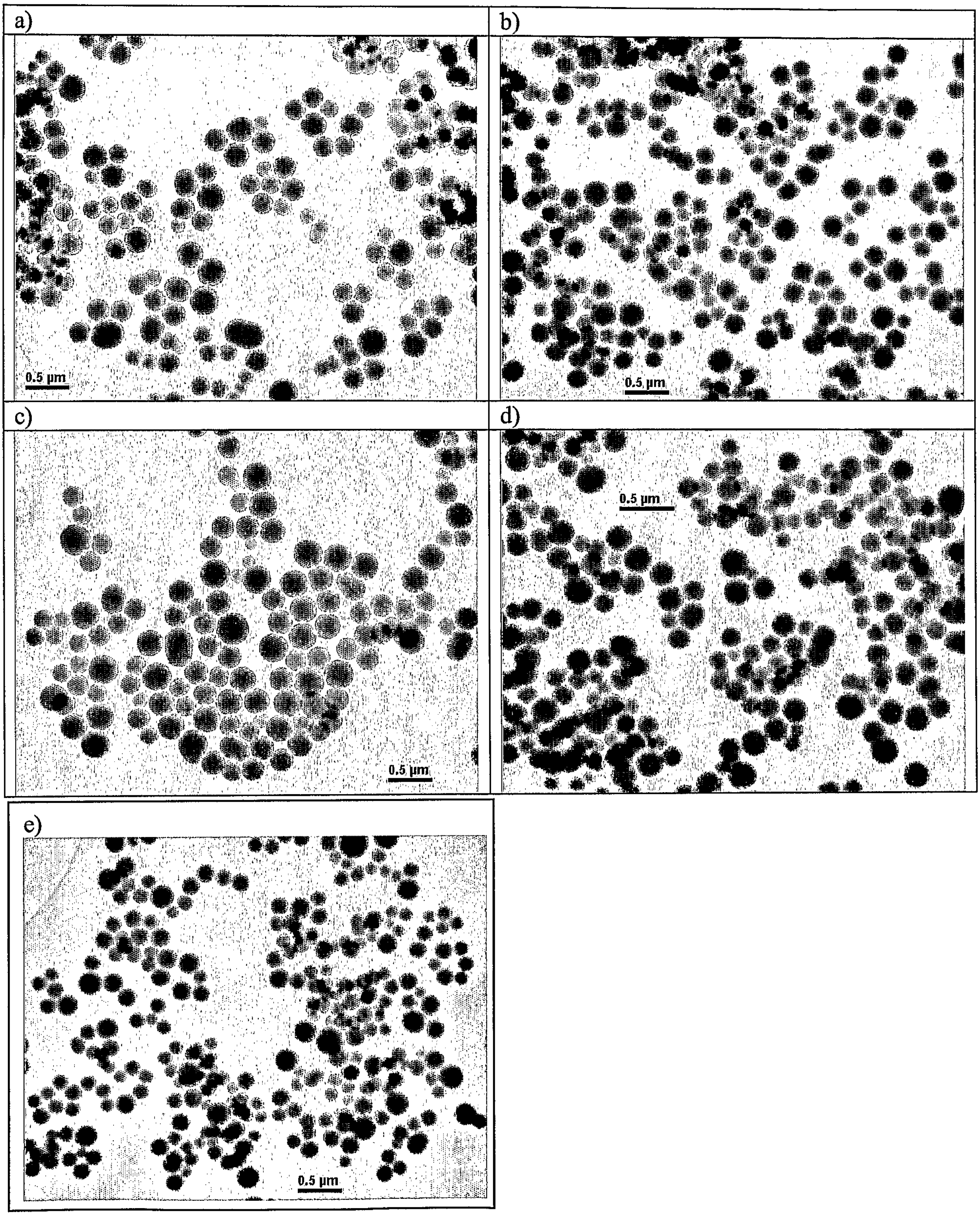


Fig. 14

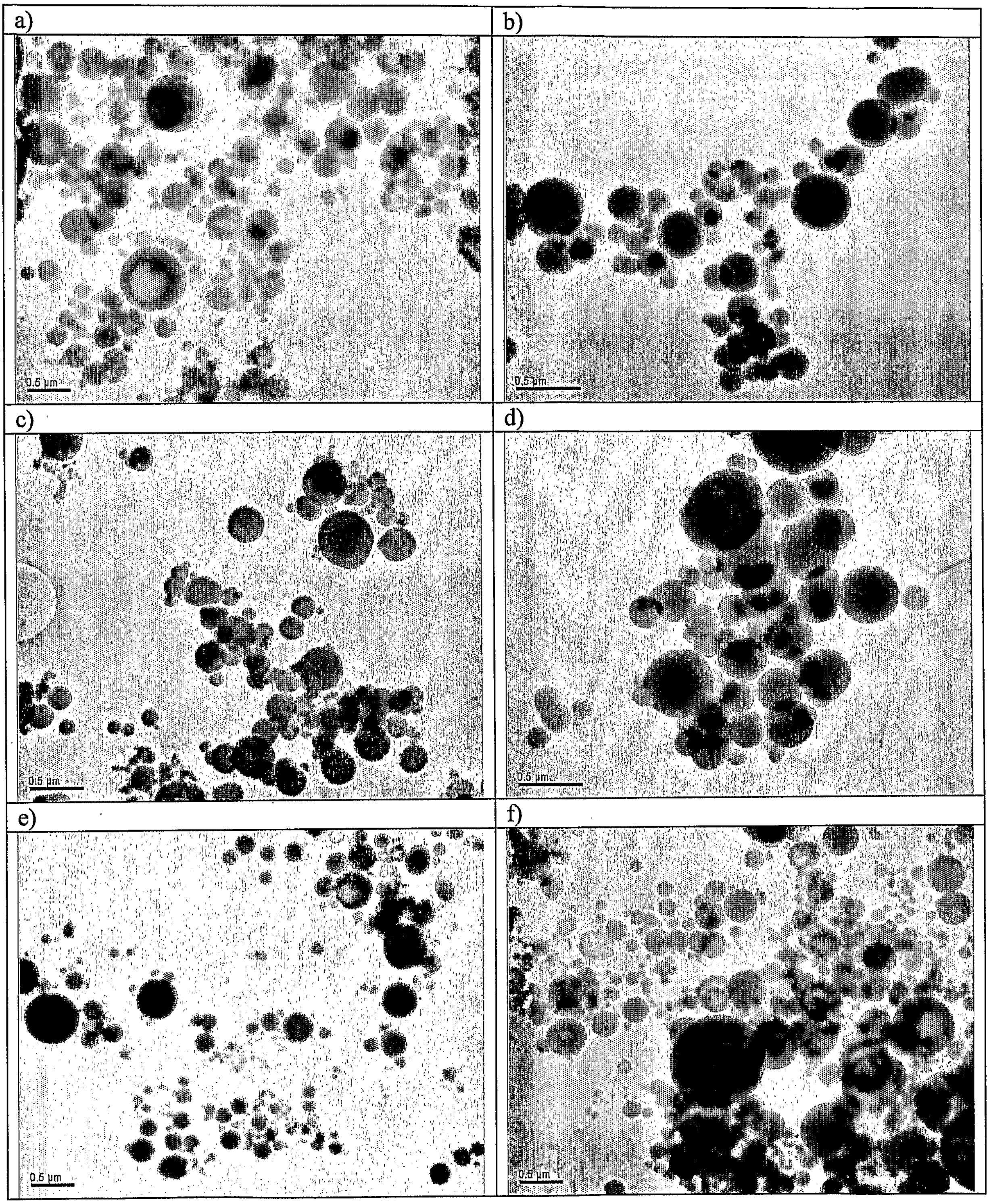


Fig. 15

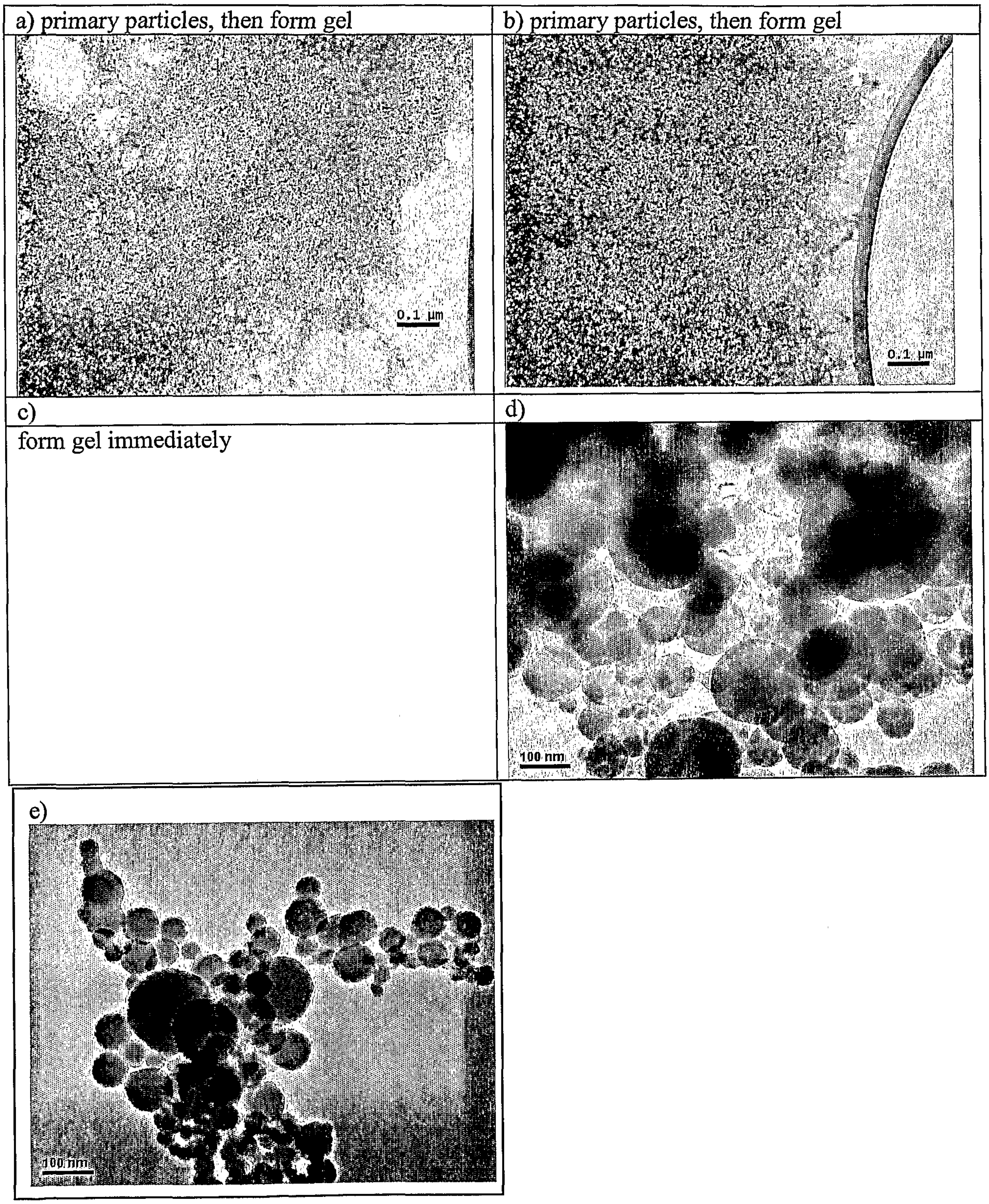
a) primary particles, then form gel
0.1 µm
b) primary particles, then form gel
0.1 µm
c)
form gel immediately
d)
100 nm
e)
100 nm

Fig. 16

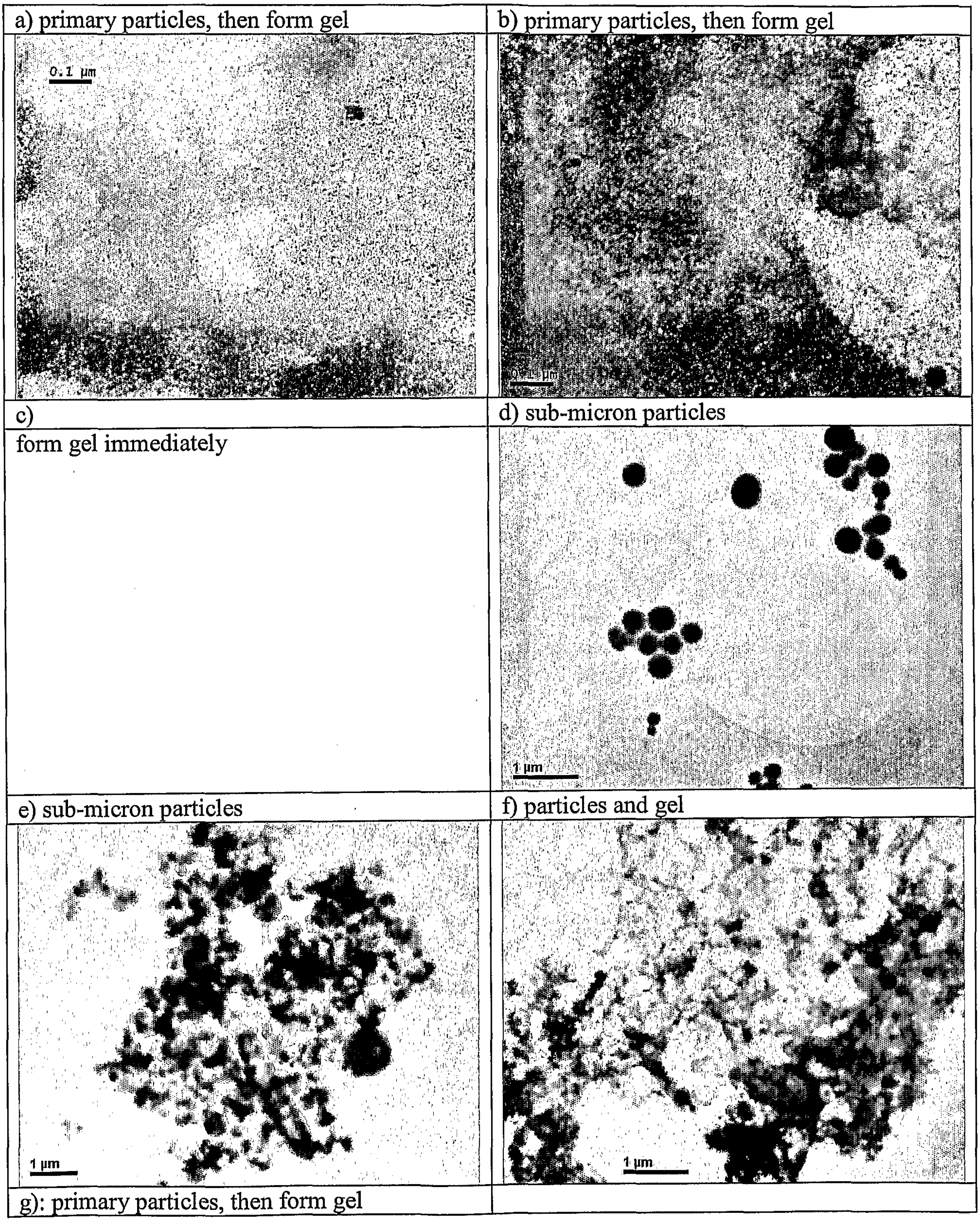
a) primary particles, then form gel
0.1 µm
b) primary particles, then form gel
0.1 µm
c)
form gel immediately
d) sub-micron particles
1 µm
e) sub-micron particles
1 µm
f) particles and gel
1 µm
g): primary particles, then form gel

Fig. 17

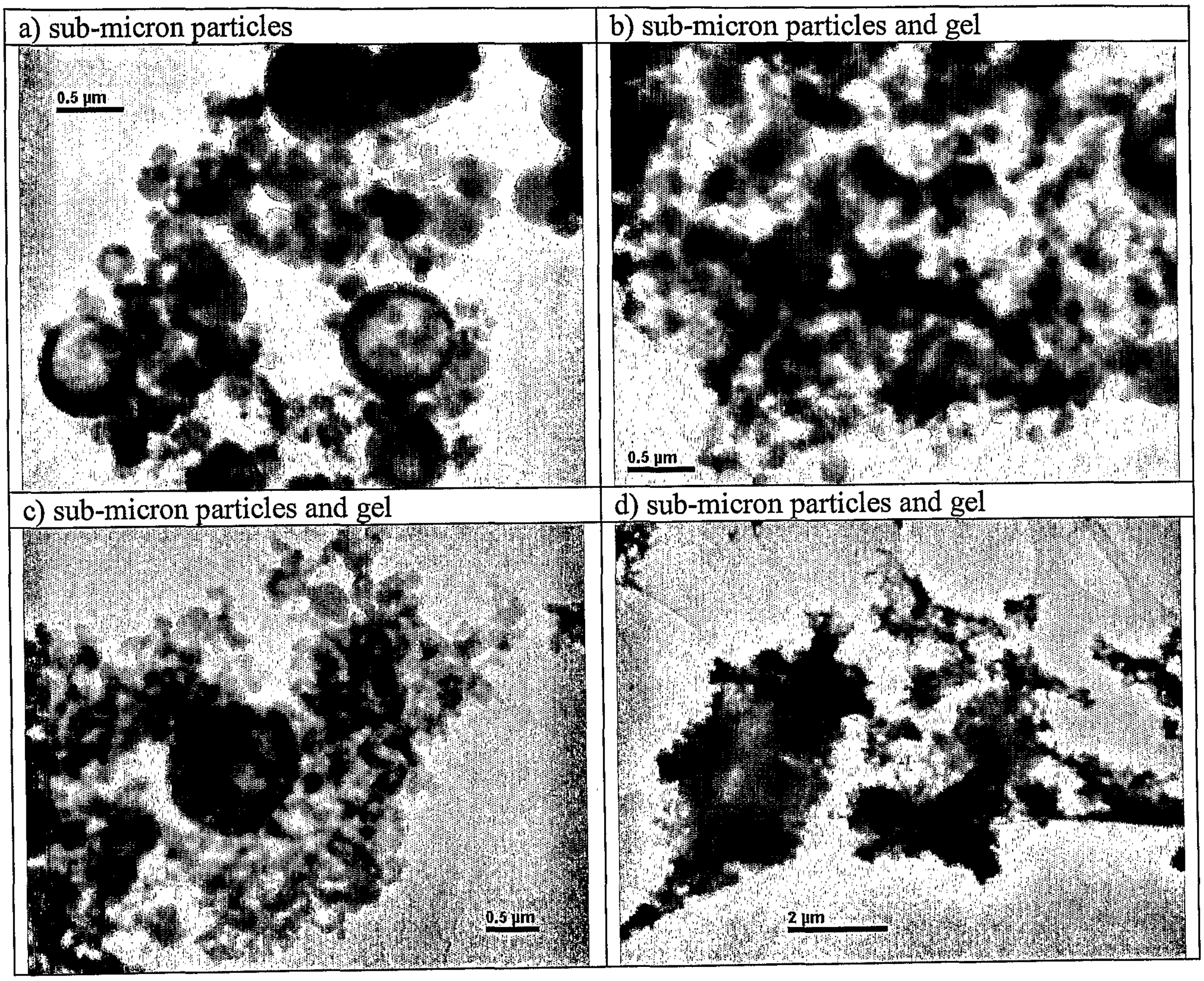
a) sub-micron particles
0.5 µm
b) sub-micron particles and gel
0.5 µm
c) sub-micron particles and gel
0.5 µm
d) sub-micron particles and gel
2 µm

**Fig. 18**

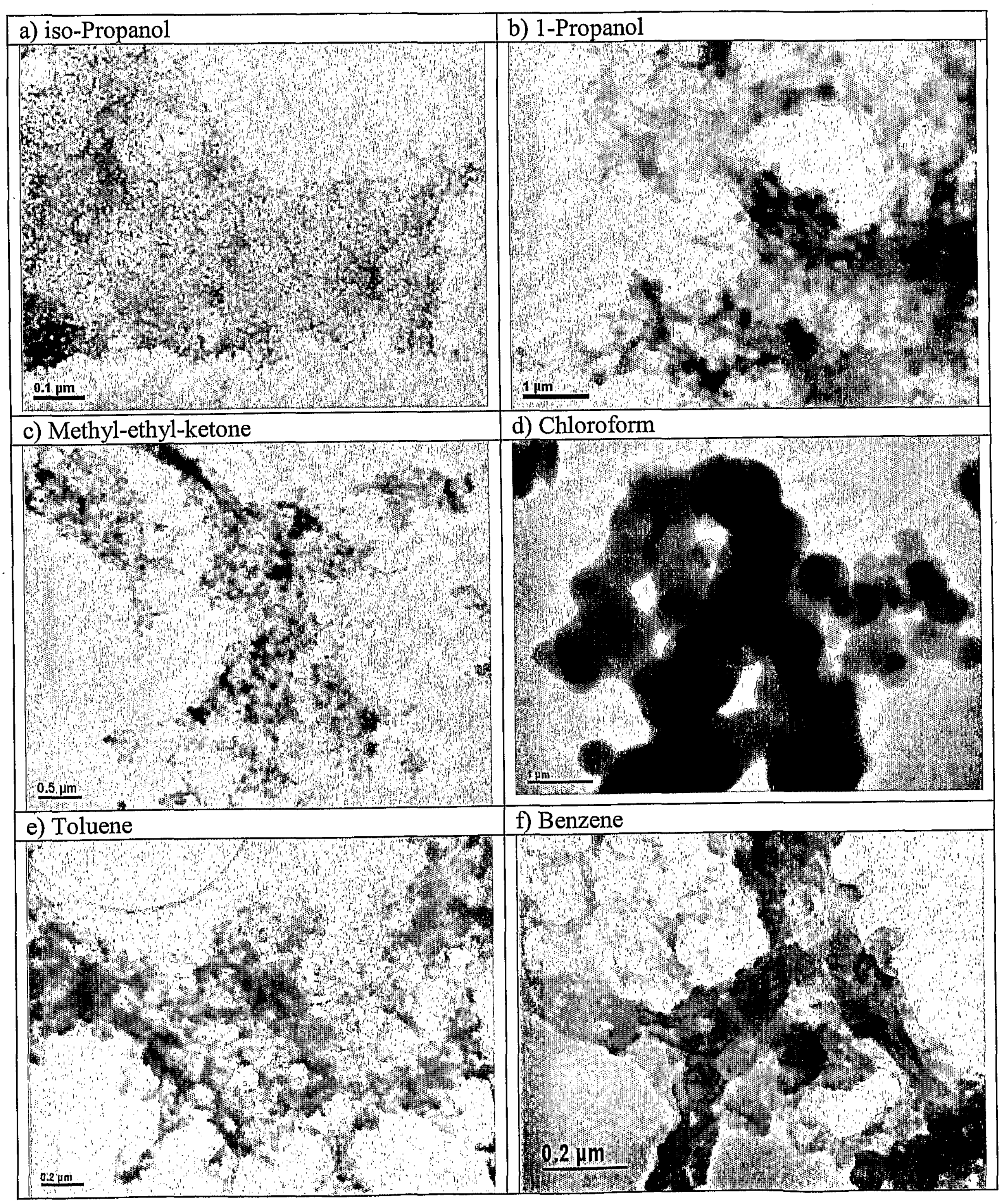
a) iso-Propanol
0.1 µm
b) 1-Propanol
1 µm
c) Methyl-ethyl-ketone
0.5 µm
d) Chloroform
e) Toluene
0.2 µm
f) Benzene
0.2 µm

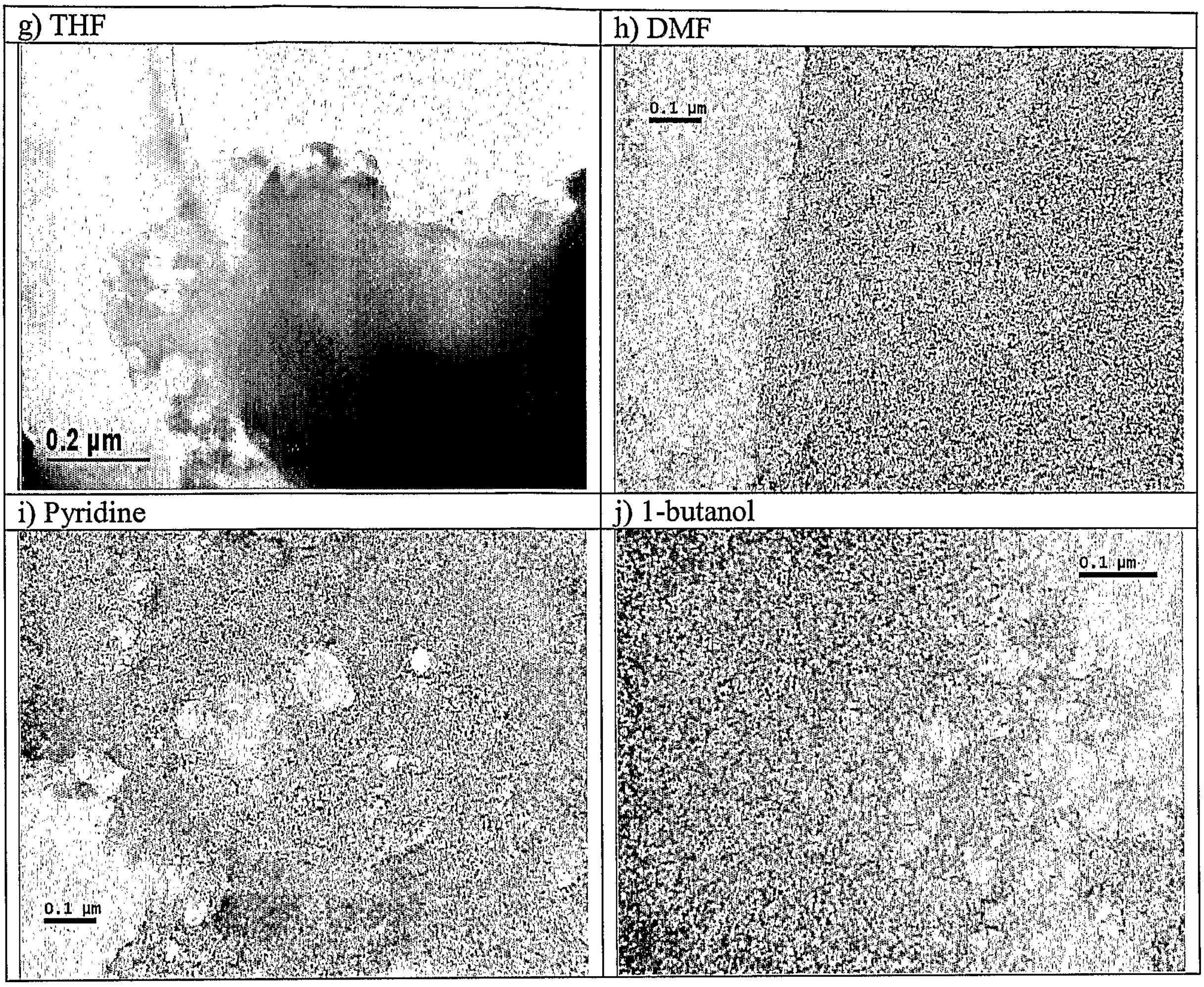


Fig. 19

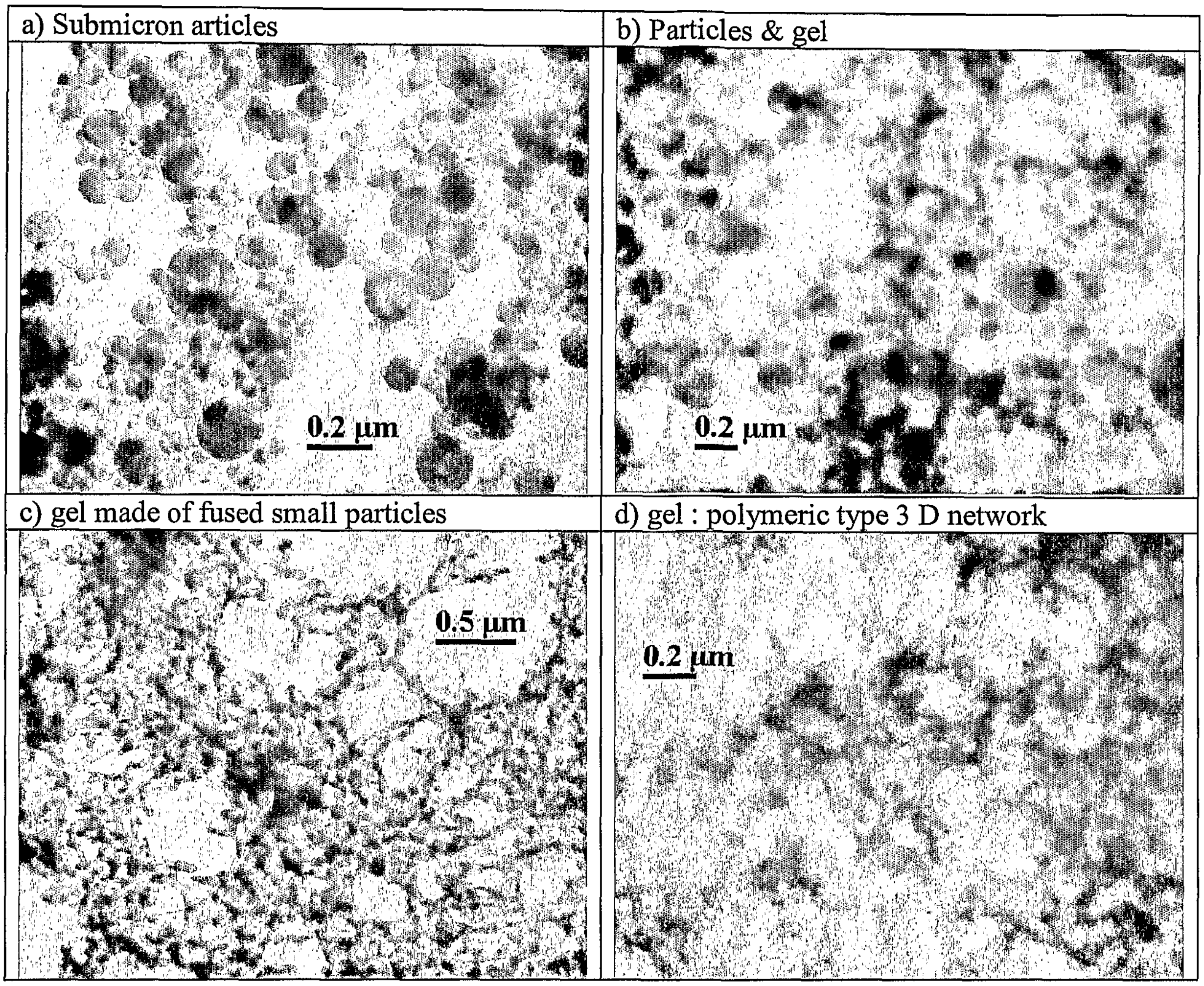
a) Submicron articles
0.2 µm
b) Particles & gel
0.2 µm
c) gel made of fused small particles
0.5 µm
d) gel : polymeric type 3 D network
0.2 µm

Fig. 20

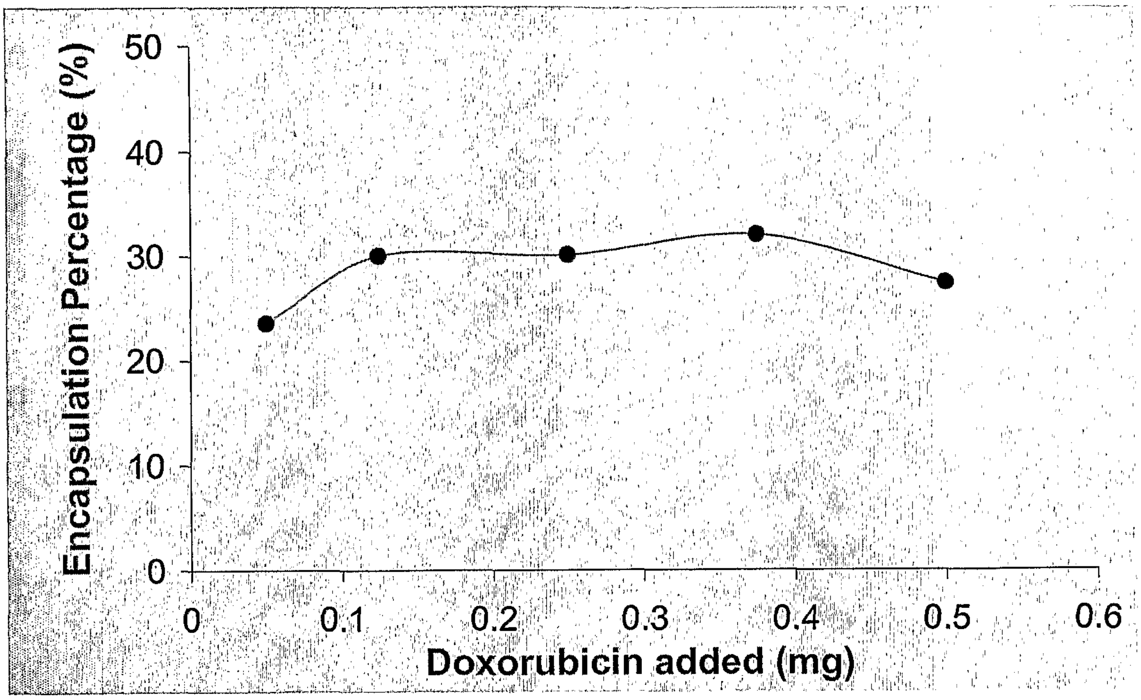
Encapsulation Percentage (%)
50
40
30
20
10
0
0
0.1
0.2
0.3
0.4
0.5
0.6
Doxorubicin added (mg)

Fig. 21

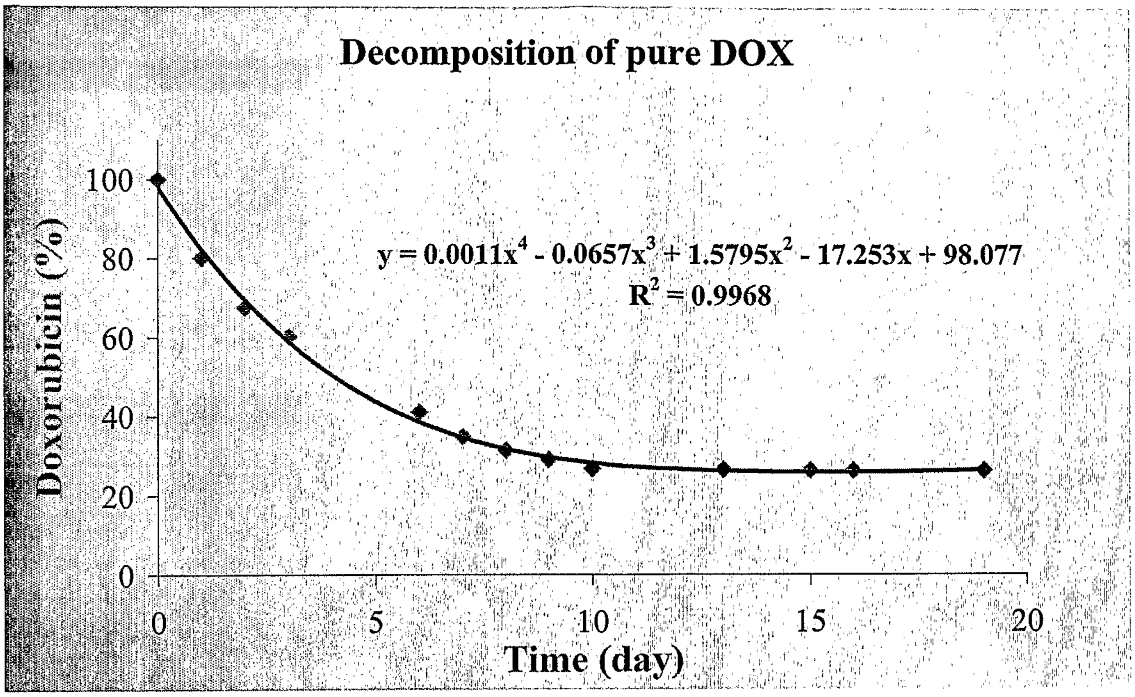
Decomposition of pure DOX
Doxorubicin (%)
100
80
60
40
20
0
y = 0.0011x^4 - 0.0657x^3 + 1.5795x^2 - 17.253x + 98.077
R^2 = 0.9968
0
5
10
15
20
Time (day)

Fig. 22

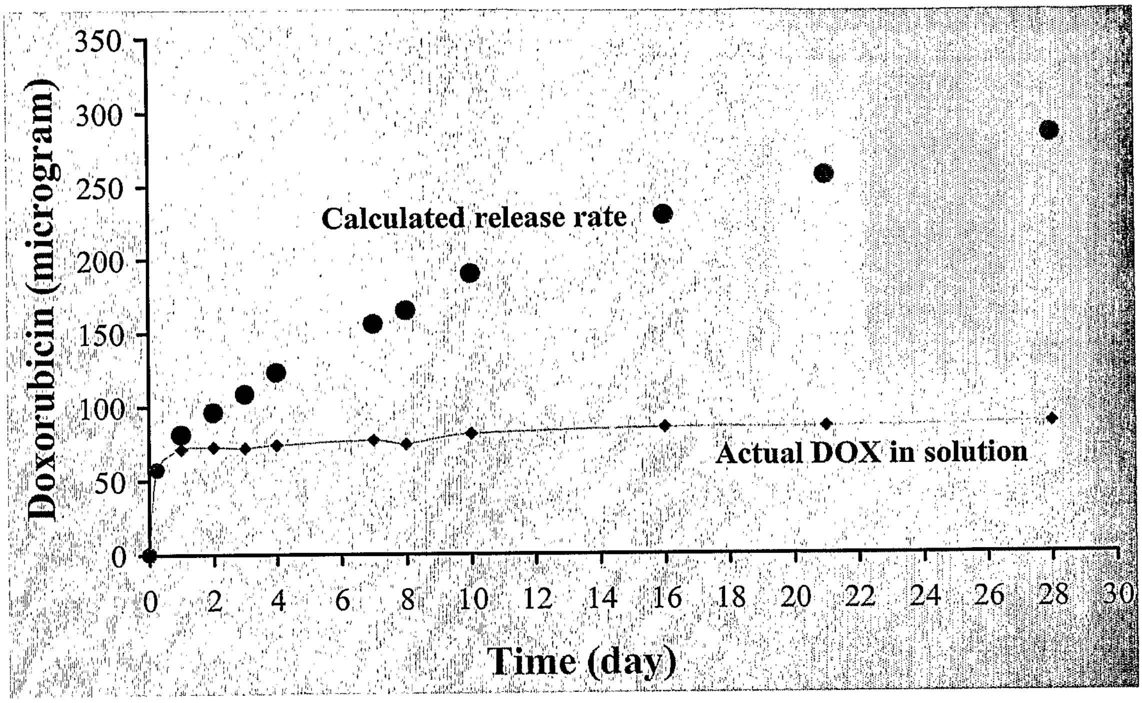

Doxorubicin (microgram)
350
300
250
200
150
100
50
0
Calculated release rate
Actual DOX in solution
0 2 4 6 8 10 12 14 16 18 20 22 24 26 28 30
Time (day)


Fig. 23

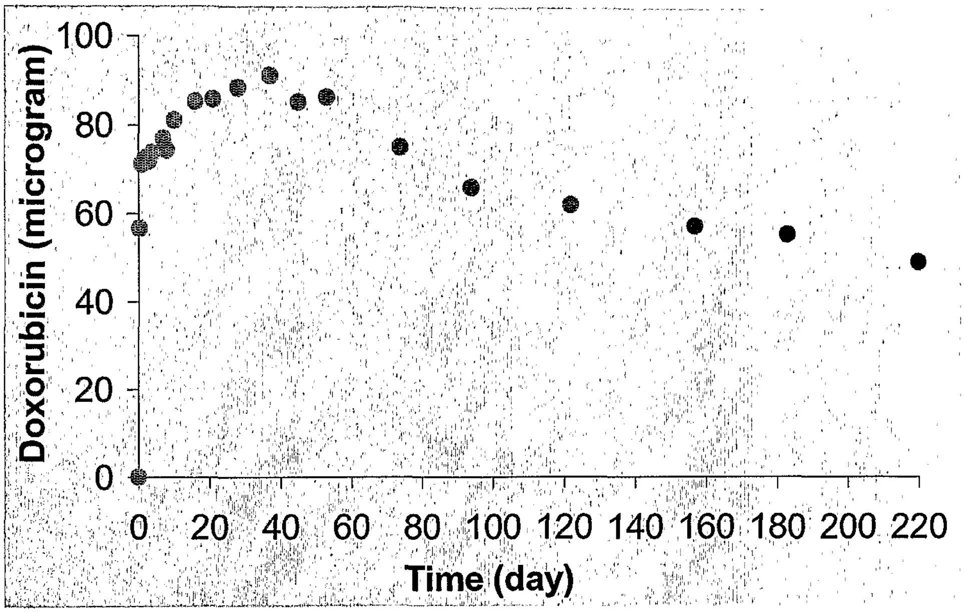
Doxorubicin (microgram)
100
80
60
40
20
0
0
20
40
60
80
100
120
140
160
180
200
220
Time (day)

Fig. 24

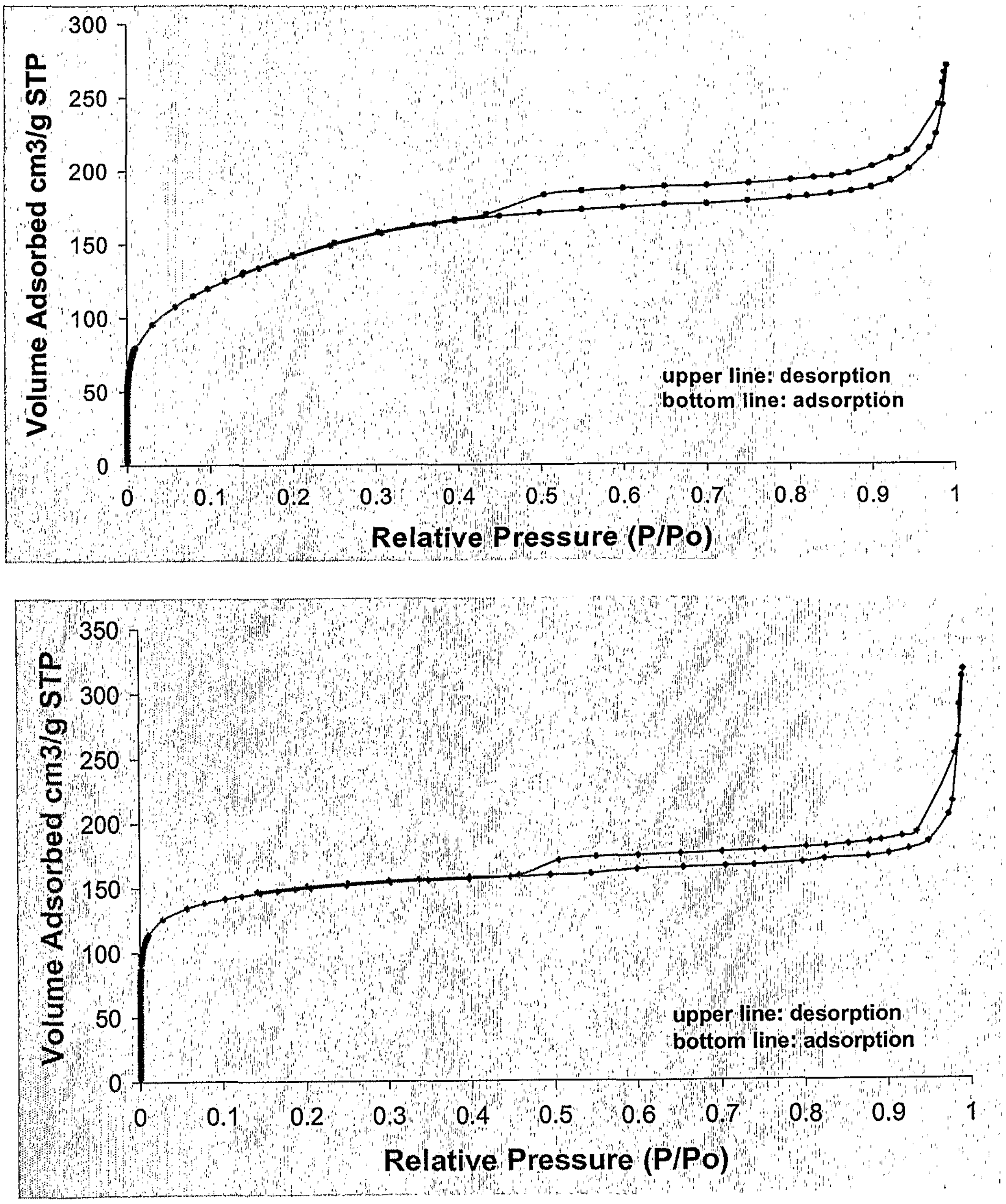
300
250
200
150
100
50
0
Volume Adsorbed cm3/g STP
0
0.1
0.2
0.3
0.4
0.5
0.6
0.7
0.8
0.9
1
Relative Pressure (P/Po)
upper line: desorption
bottom line: adsorption
350
300
250
200
150
100
50
0
Volume Adsorbed cm3/g STP
0
0.1
0.2
0.3
0.4
0.5
0.6
0.7
0.8
0.9
1
Relative Pressure (P/Po)
upper line: desorption
bottom line: adsorption

Fig. 25

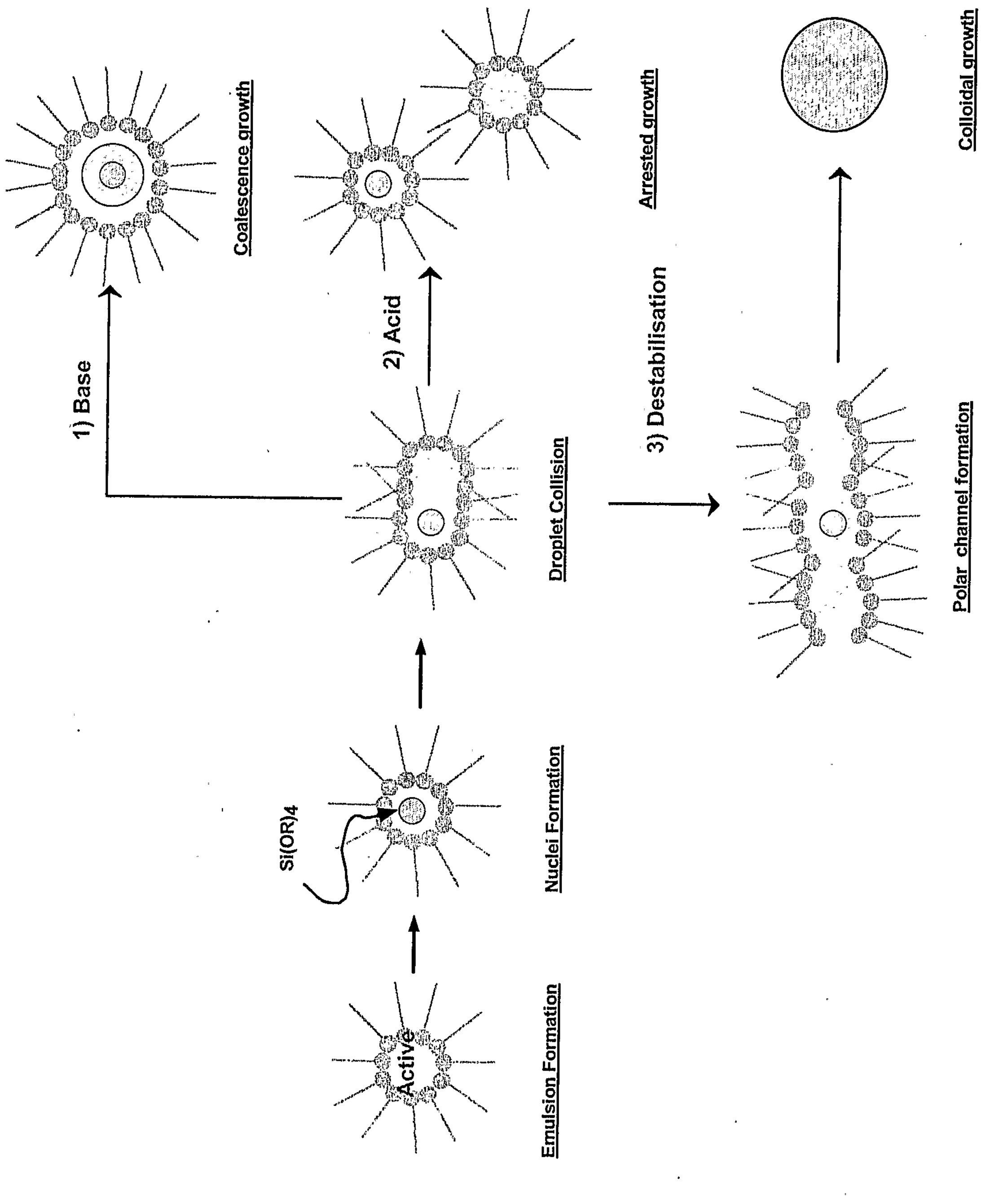
Emulsion Formation
Active
Nuclei Formation
Si(OR)4
Droplet Collision
1) Base
Coalescence growth
2) Acid
Arrested growth
3) Destabilisation
Polar channel formation
Colloidal growth

Fig. 26

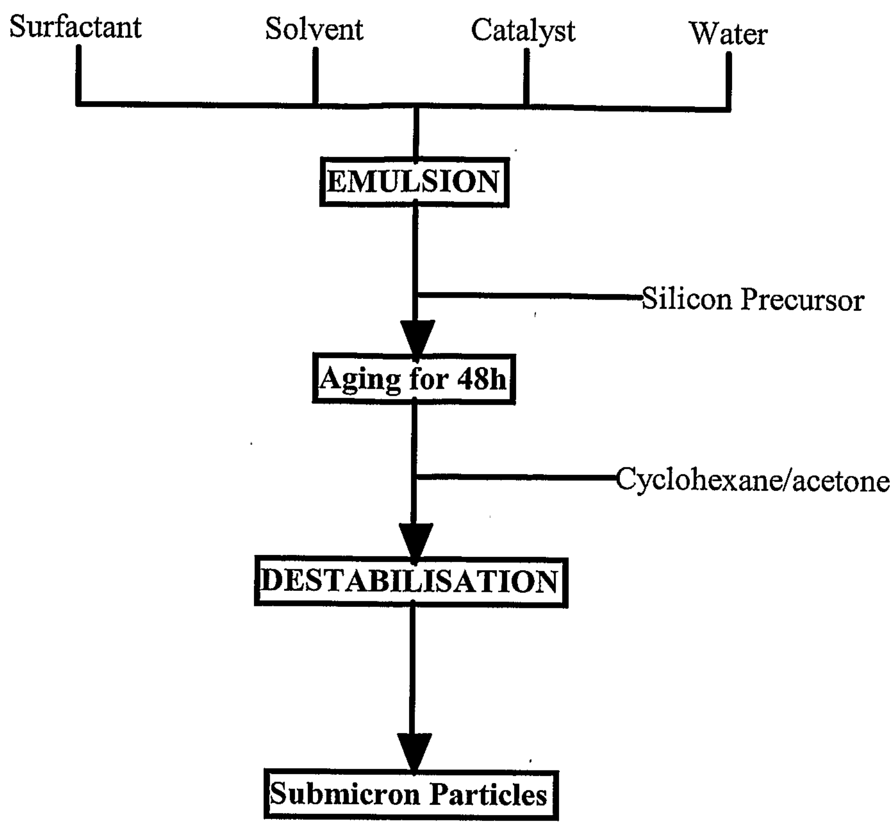
Surfactant
Solvent
Catalyst
Water
EMULSION
Silicon Precursor
Aging for 48h
Cyclohexane/acetone
DESTABILISATION
Submicron Particles

**Fig. 27**

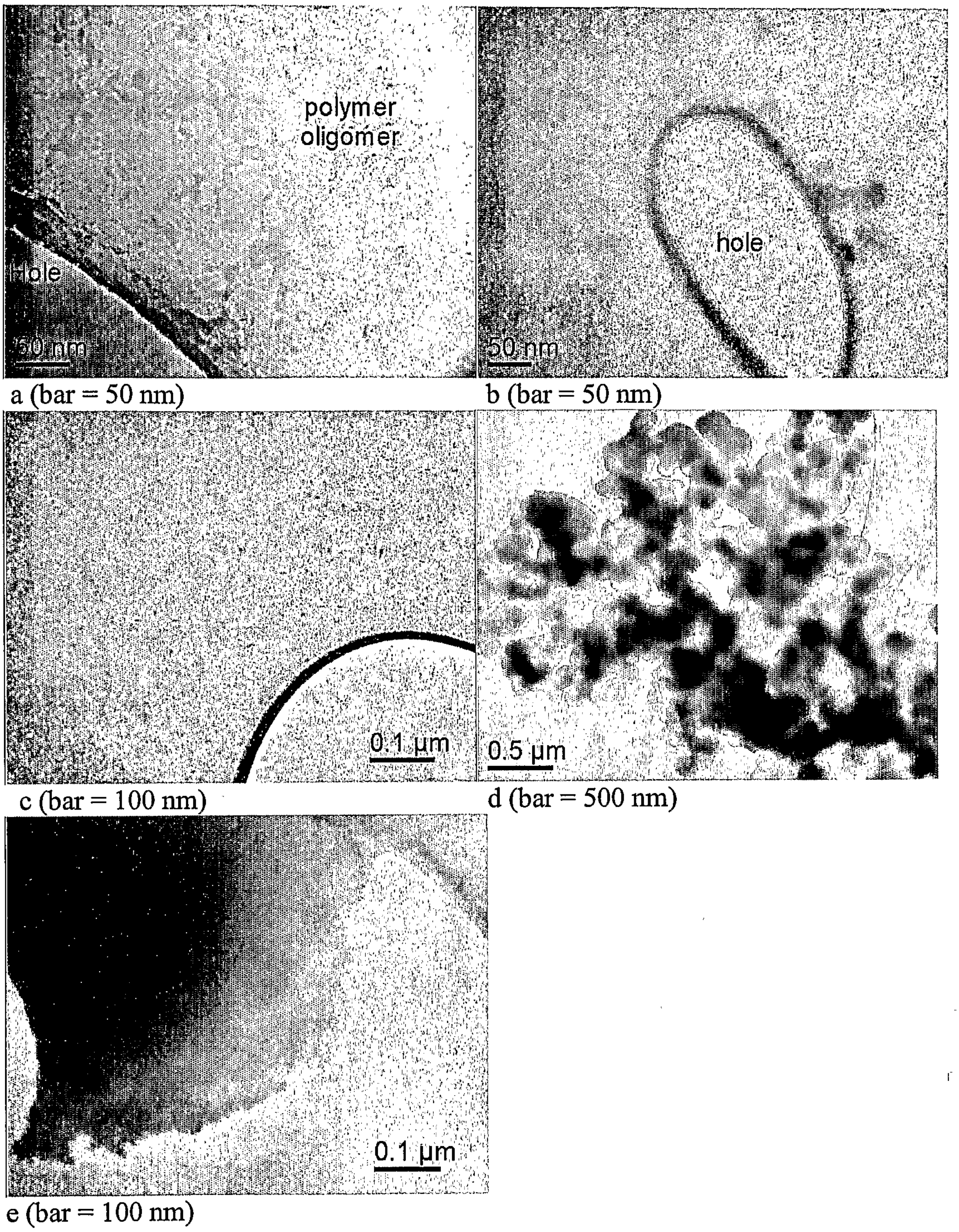


a (bar = 50 nm)
b (bar = 50 nm)
c (bar = 100 nm)
d (bar = 500 nm)
e (bar = 100 nm)

**Fig. 28**

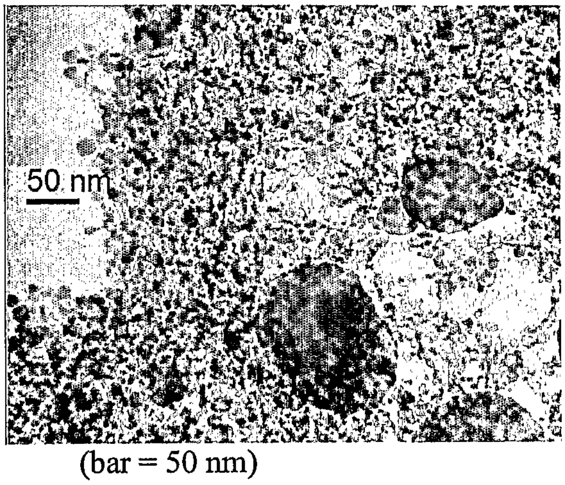


(bar = 50 nm)

Fig. 29

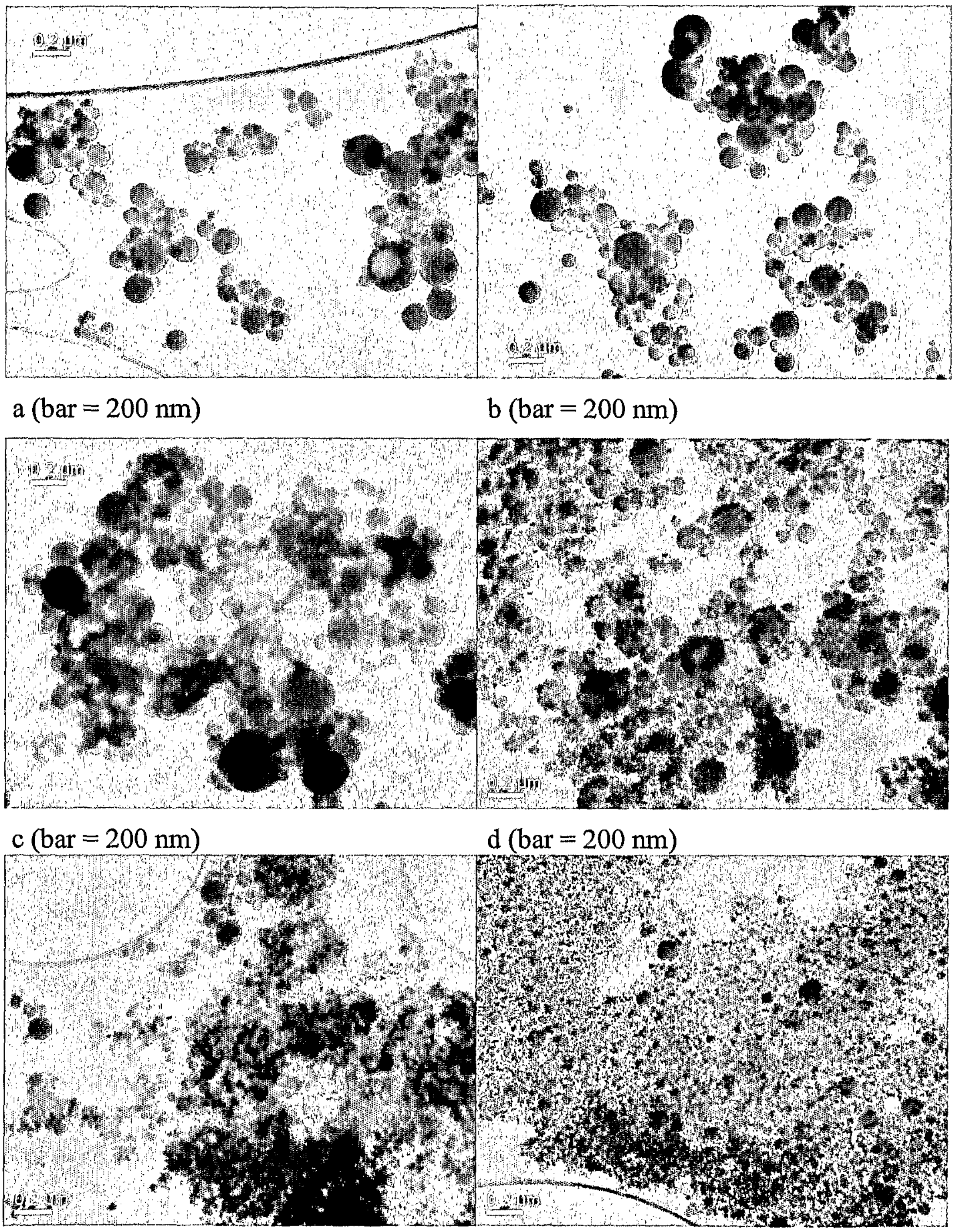

a (bar = 200 nm) b (bar = 200 nm)

c (bar = 200 nm) d (bar = 200 nm)

e (bar = 200 nm) f (bar = 200 nm)

**Fig. 30**

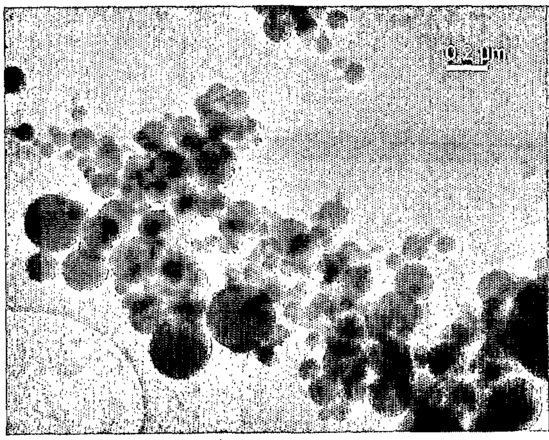


a (bar = 200 nm)

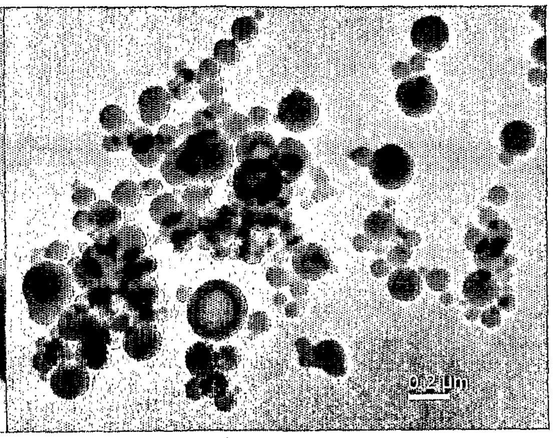


b (bar = 200 nm)

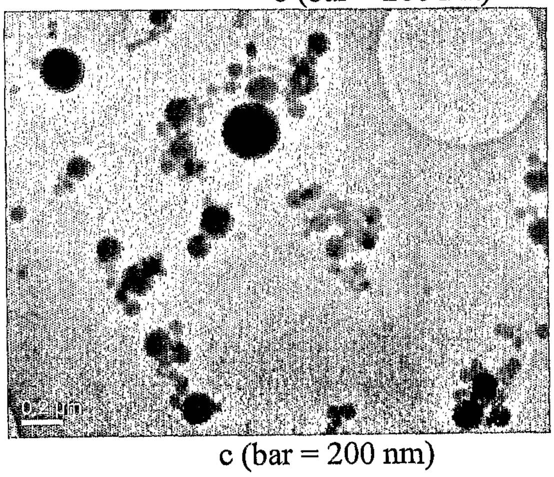


c (bar = 200 nm)

**Fig. 31**

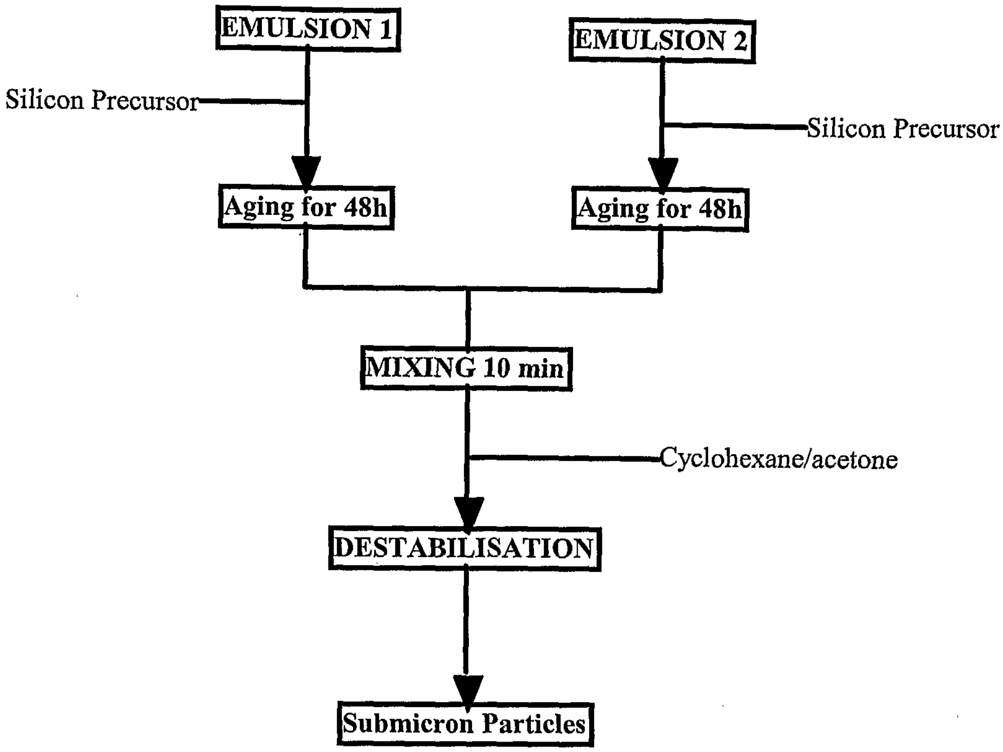
EMULSION 1
EMULSION 2
Silicon Precursor
Silicon Precursor
Aging for 48h
Aging for 48h
MIXING 10 min
Cyclohexane/acetone
DESTABILISATION
Submicron Particles

Fig. 32

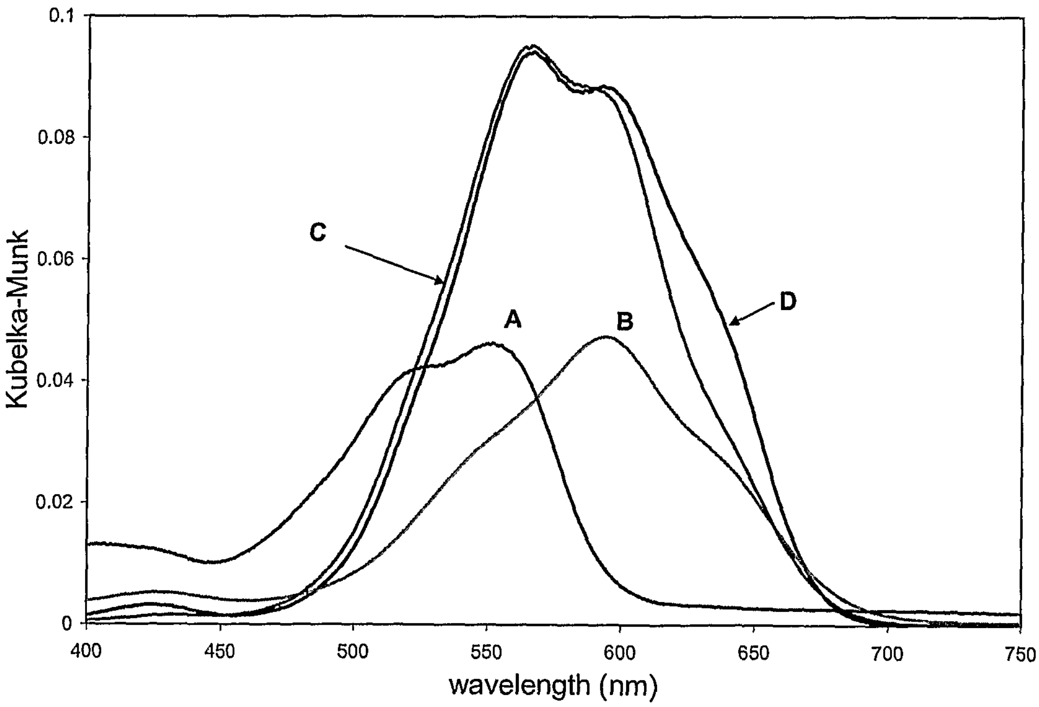
0.1
0.08
0.06
0.04
0.02
0
Kubelka-Munk
C
A
B
D
400
450
500
550
600
650
700
750
wavelength (nm)

Fig. 33

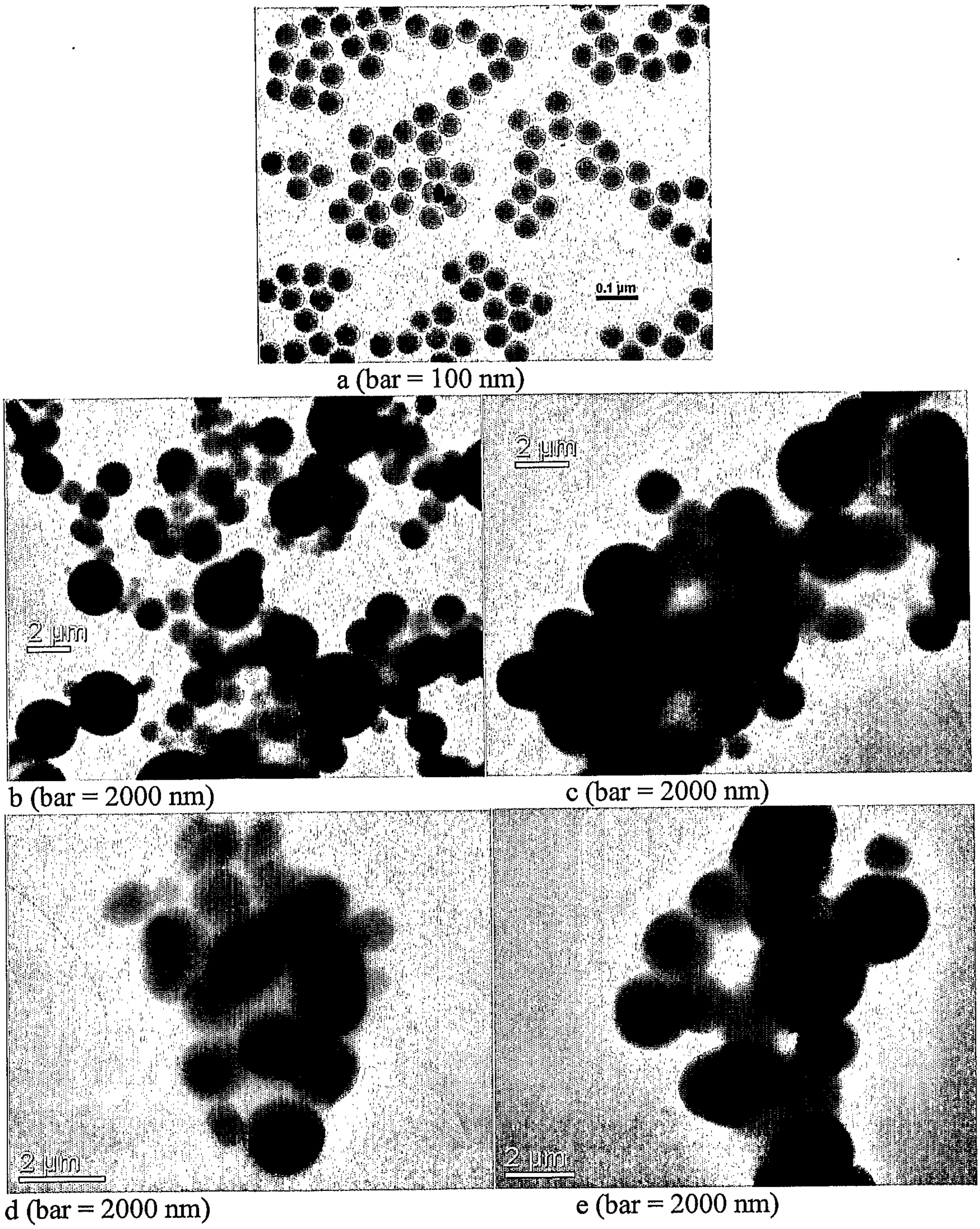


a (bar = 100 nm)

b (bar = 2000 nm)

c (bar = 2000 nm)

d (bar = 2000 nm)

e (bar = 2000 nm)

**Fig. 34**

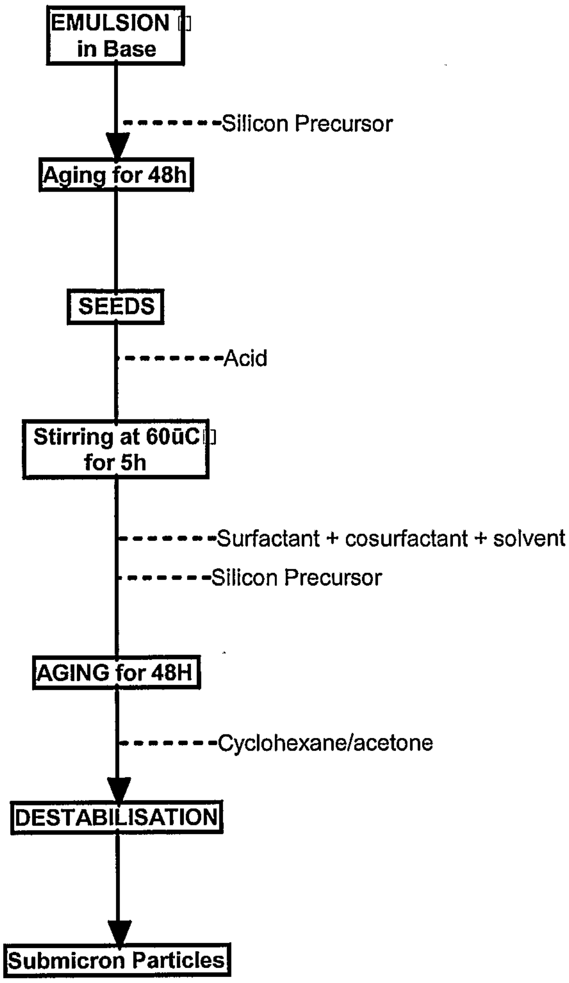
EMULSION
in Base
Silicon Precursor
Aging for 48h
SEEDS
Acid
Stirring at 60ūC
for 5h
Surfactant + cosurfactant + solvent
Silicon Precursor
AGING for 48H
Cyclohexane/acetone
DESTABILISATION
Submicron Particles

Fig. 35

| Seeds (Bar 50 nm) | Destabilised Particles (Bar 2 µm) |
|---|---|
| a) | |
| 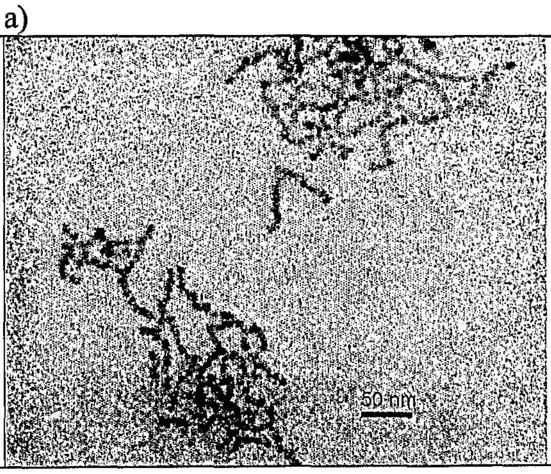 | 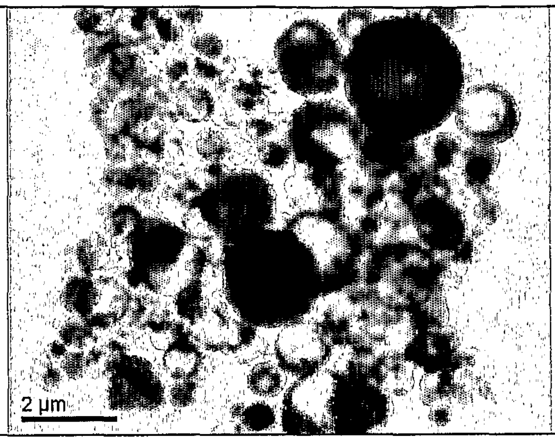 |
| b | |
| 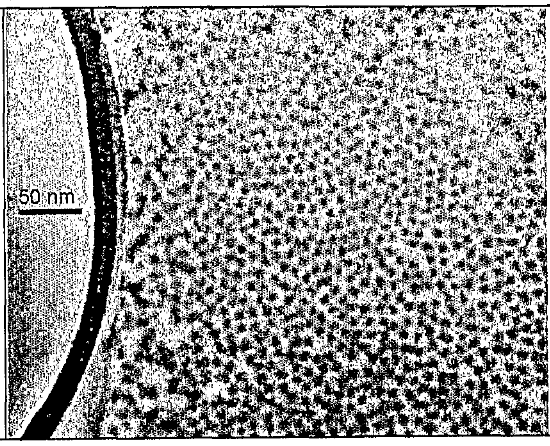 | 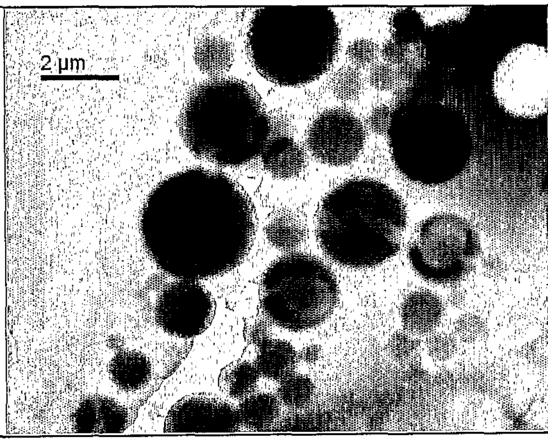 |
| c) | |
| 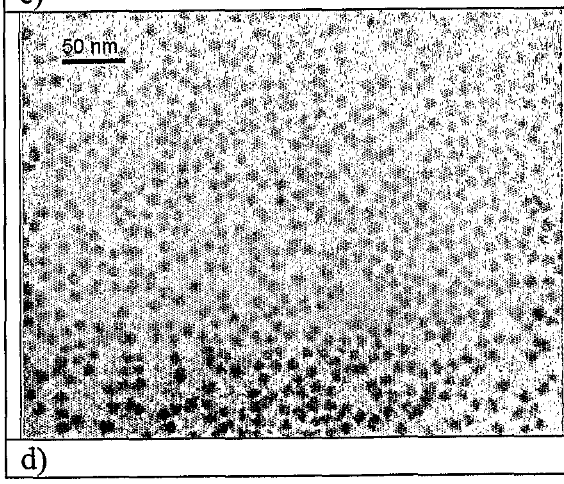 | 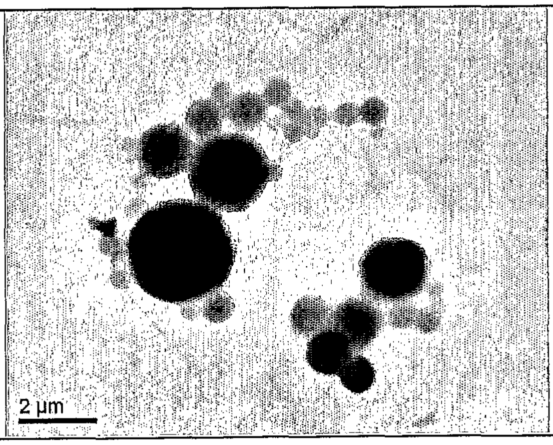 |
| d) | |

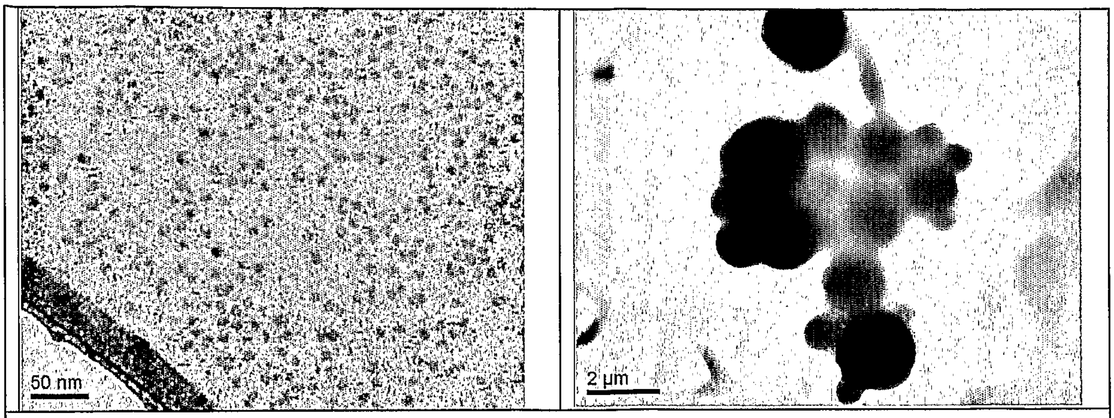


e)

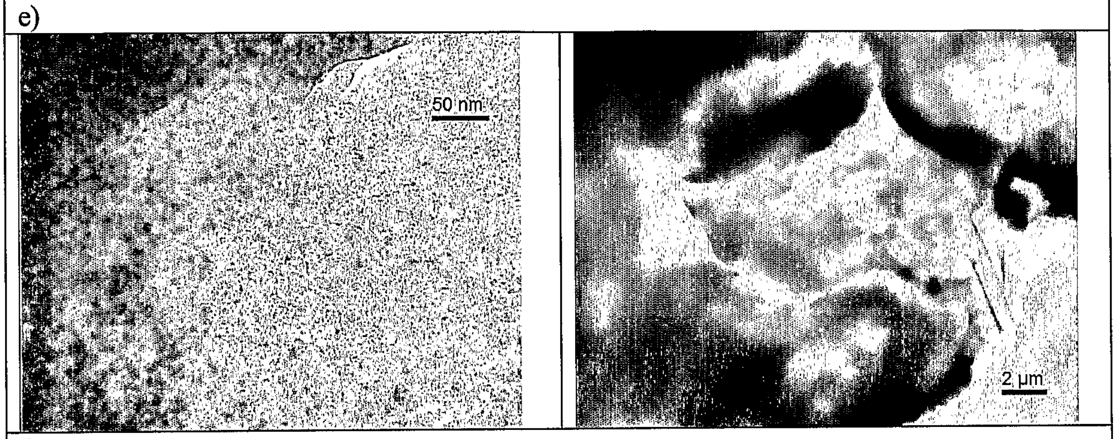


f)

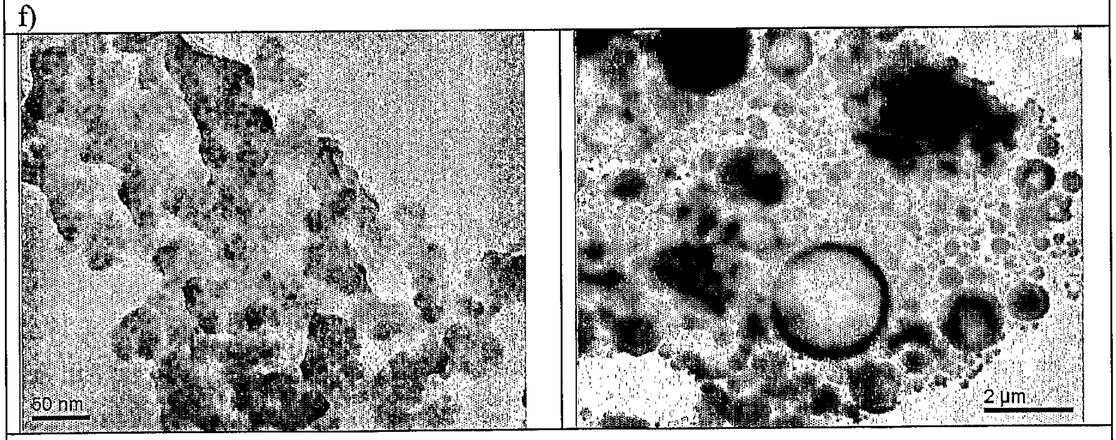


g)

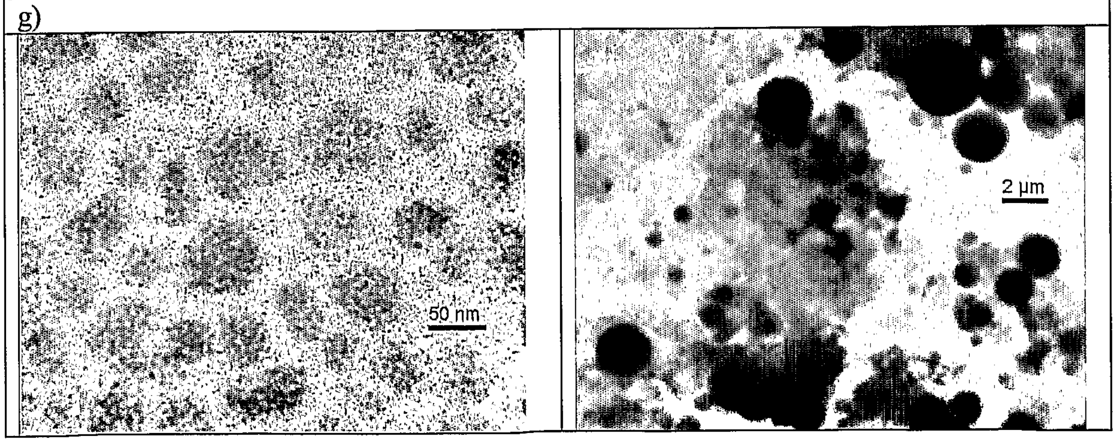

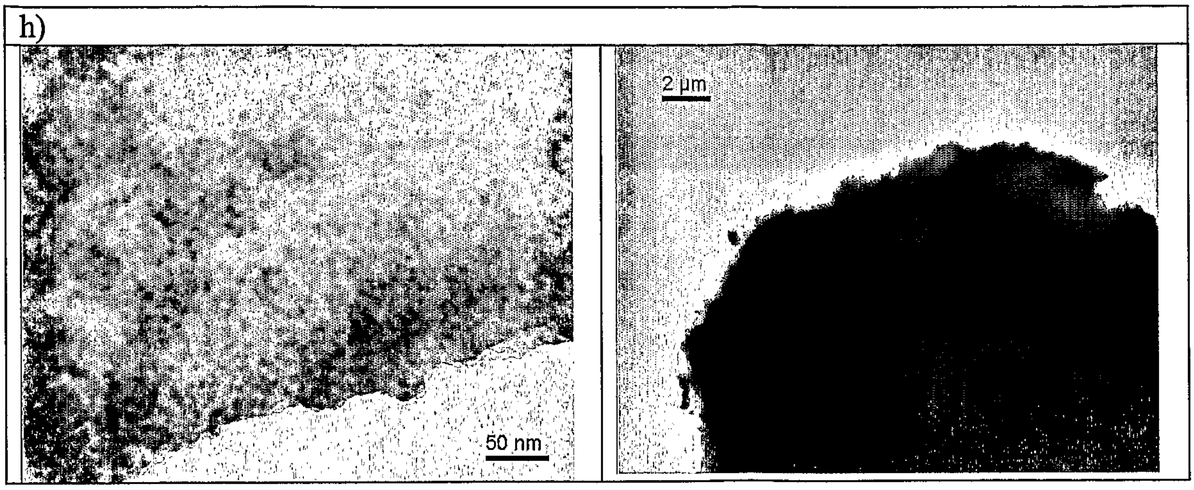


Fig. 36

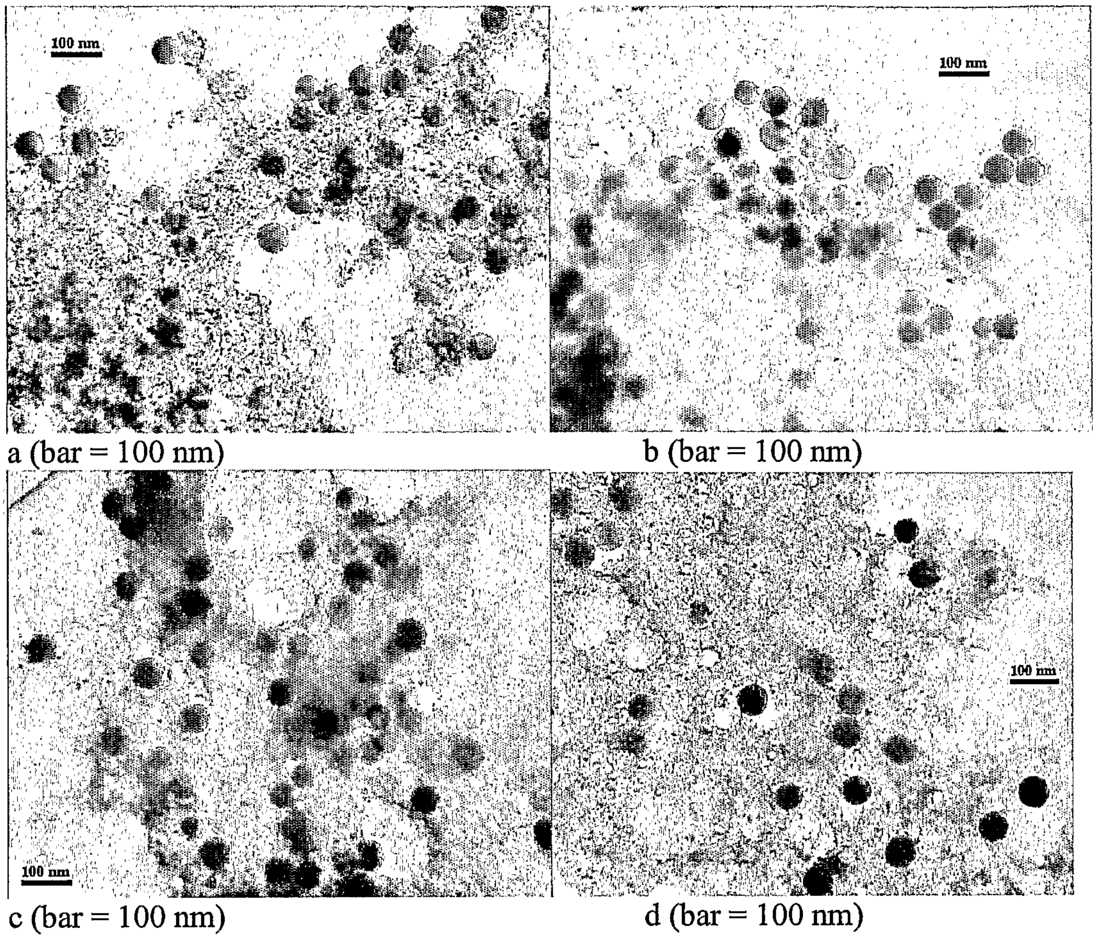


a (bar = 100 nm)

b (bar = 100 nm)

c (bar = 100 nm)

d (bar = 100 nm)

**Fig. 37**

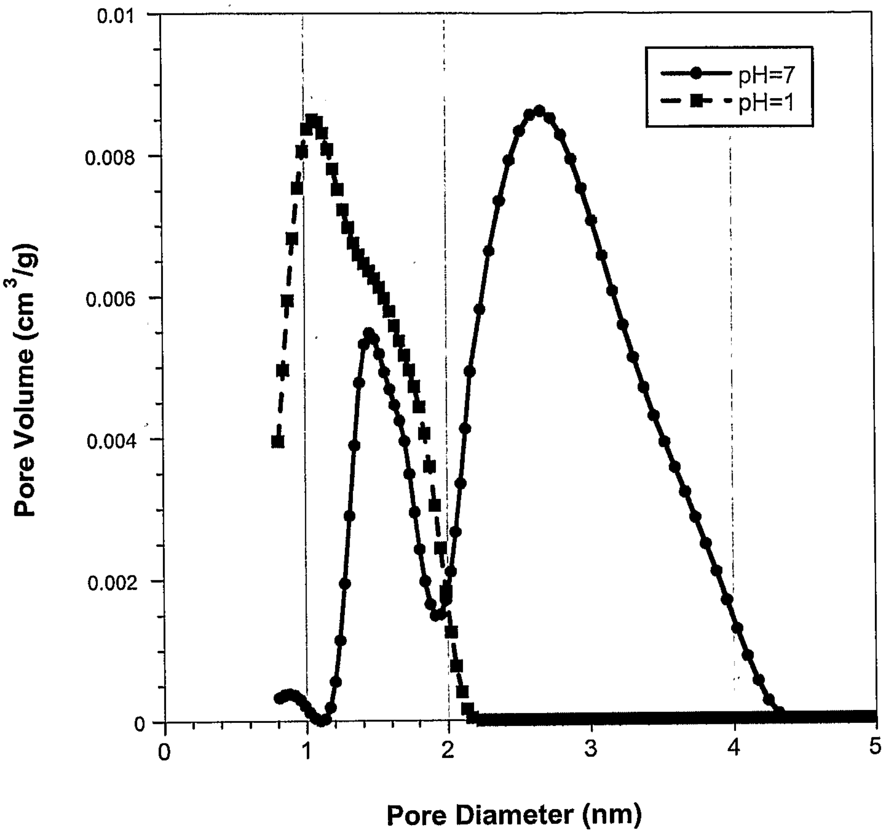
0.01
0.008
0.006
0.004
0.002
0
Pore Volume (cm$^3$/g)
0
1
2
3
4
5
Pore Diameter (nm)
pH=7
pH=1

Fig. 38

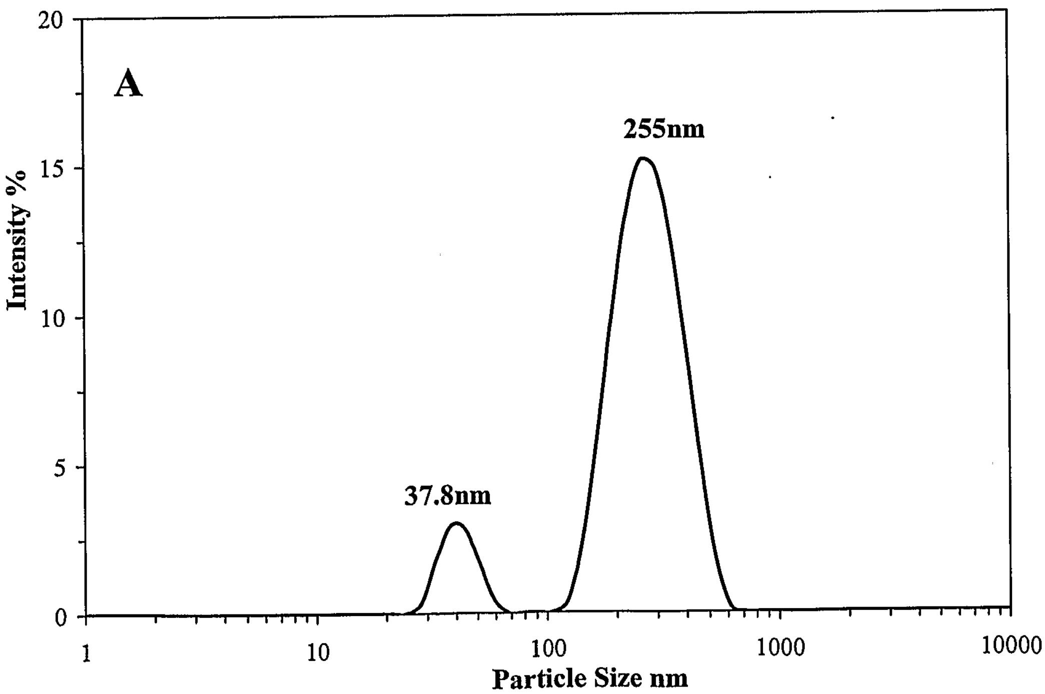
20
15
10
5
0
Intensity %
A
255nm
37.8nm
1
10
100
1000
10000
Particle Size nm

Fig. 39

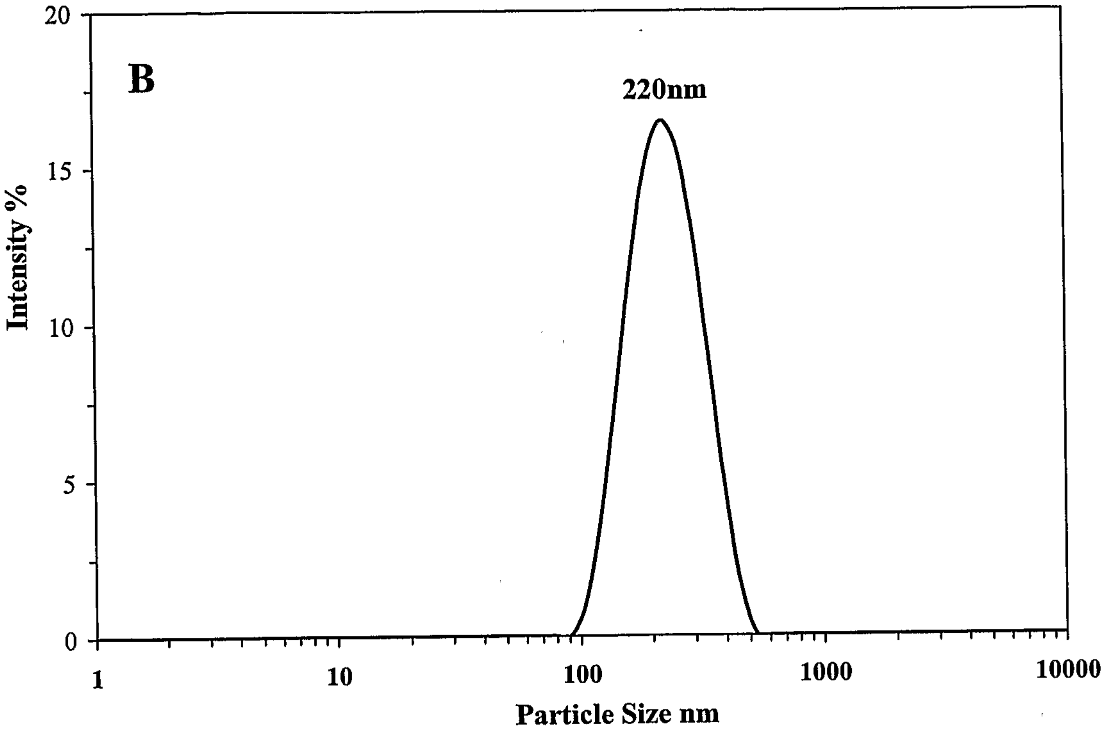
B
220nm
Intensity %
20
15
10
5
0
1
10
100
1000
10000
Particle Size nm

Fig. 40

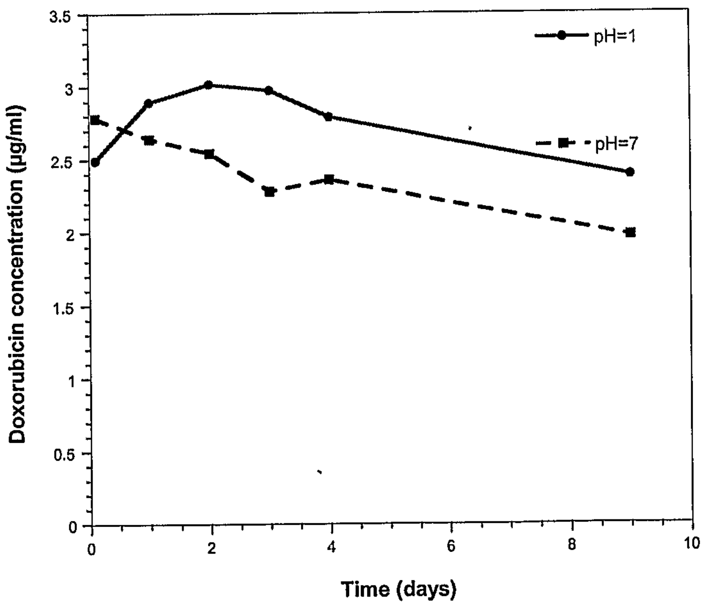
pH=1
pH=7
Doxorubicin concentration (µg/ml)
3.5
3
2.5
2
1.5
1
0.5
0
0
2
4
6
8
10
Time (days)

Fig. 41

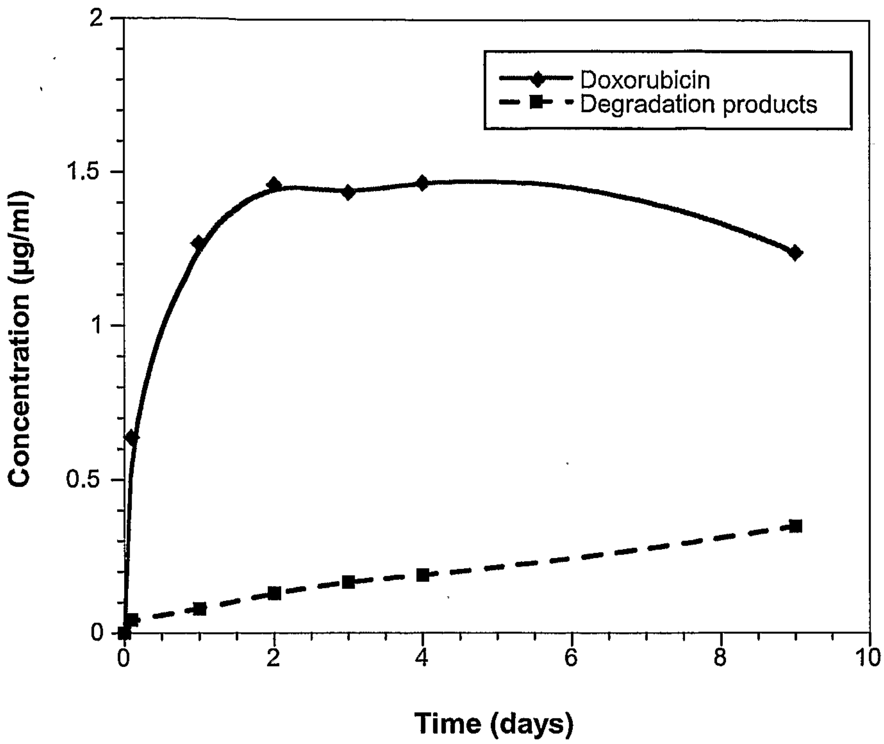
Doxorubicin
Degradation products
Concentration (µg/ml)
Time (days)
0
0.5
1
1.5
2
0
2
4
6
8
10

Fig. 42

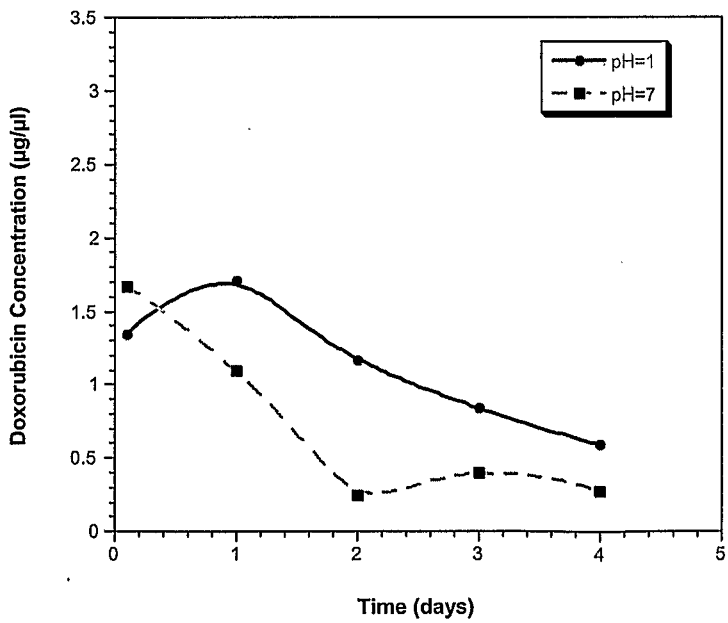
pH=1
pH=7
Doxorubicin Concentration (µg/µl)
Time (days)
0
0.5
1
1.5
2
2.5
3
3.5
0
1
2
3
4
5

Fig. 43

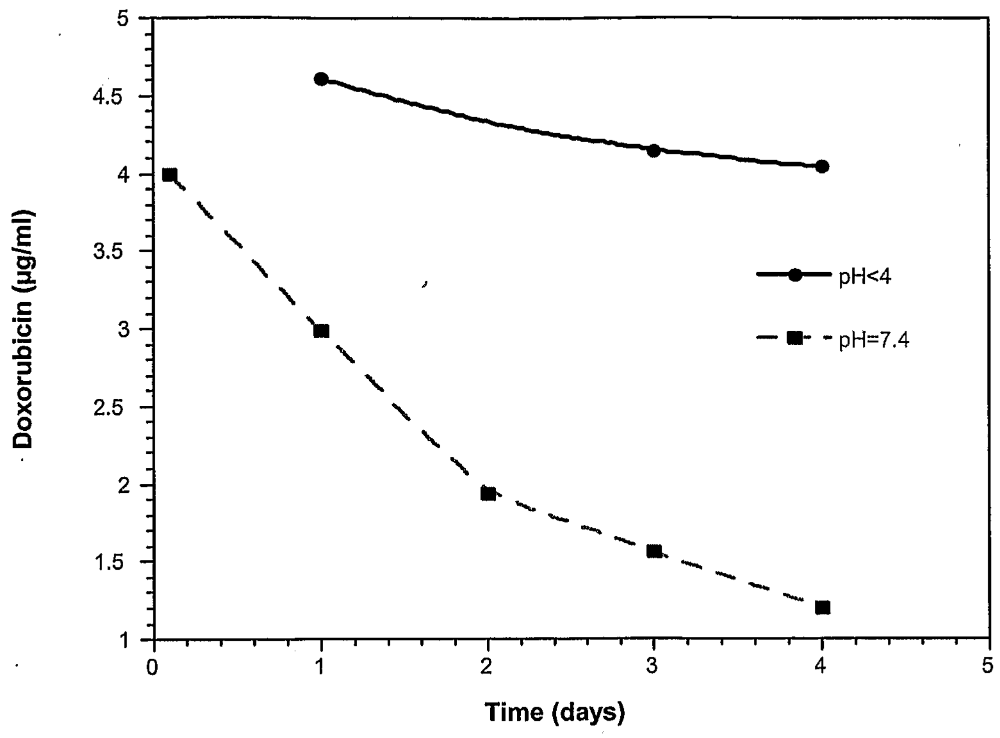
Doxorubicin (µg/ml)
5
4.5
4
3.5
3
2.5
2
1.5
1
0
1
2
3
4
5
Time (days)
pH<4
pH=7.4

Fig. 44

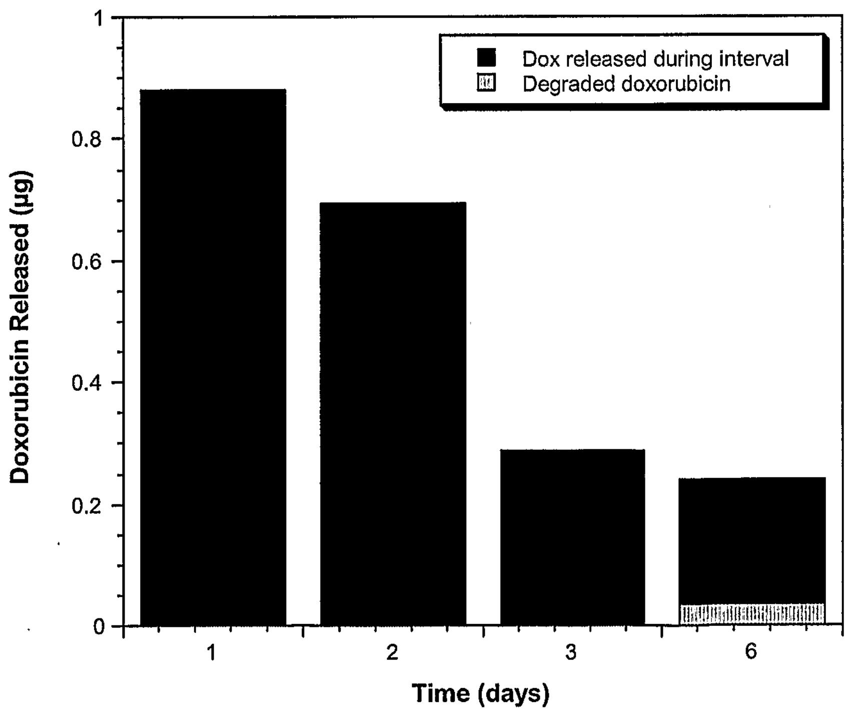
Doxorubicin Released (µg)
1
0.8
0.6
0.4
0.2
0
1
2
3
6
Time (days)
Dox released during interval
Degraded doxorubicin

Fig. 45

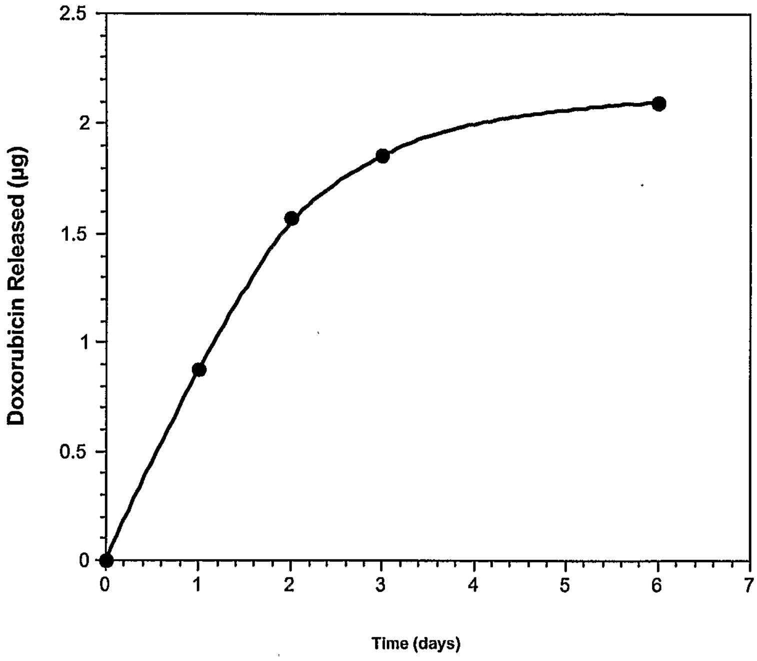
Doxorubicin Released (µg)
Time (days)
2.5
2
1.5
1
0.5
0
0
1
2
3
4
5
6
7

Fig. 46

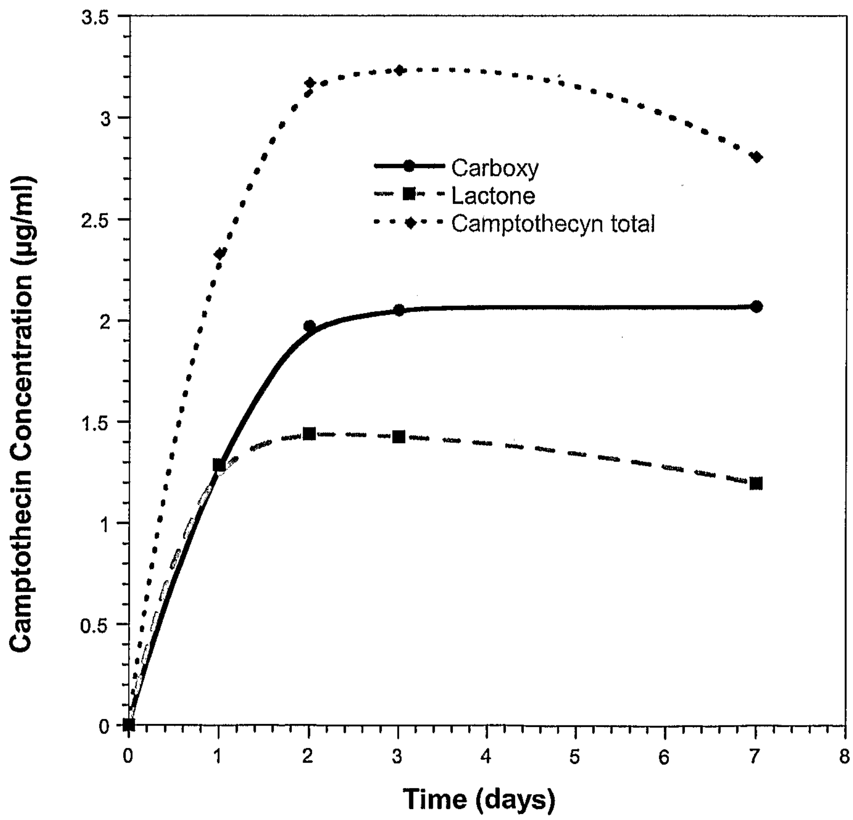
Camptothecin Concentration (µg/ml)
Time (days)
Carboxy
Lactone
Camptothecyn total
3.5
3
2.5
2
1.5
1
0.5
0
0
1
2
3
4
5
6
7
8

Fig. 47

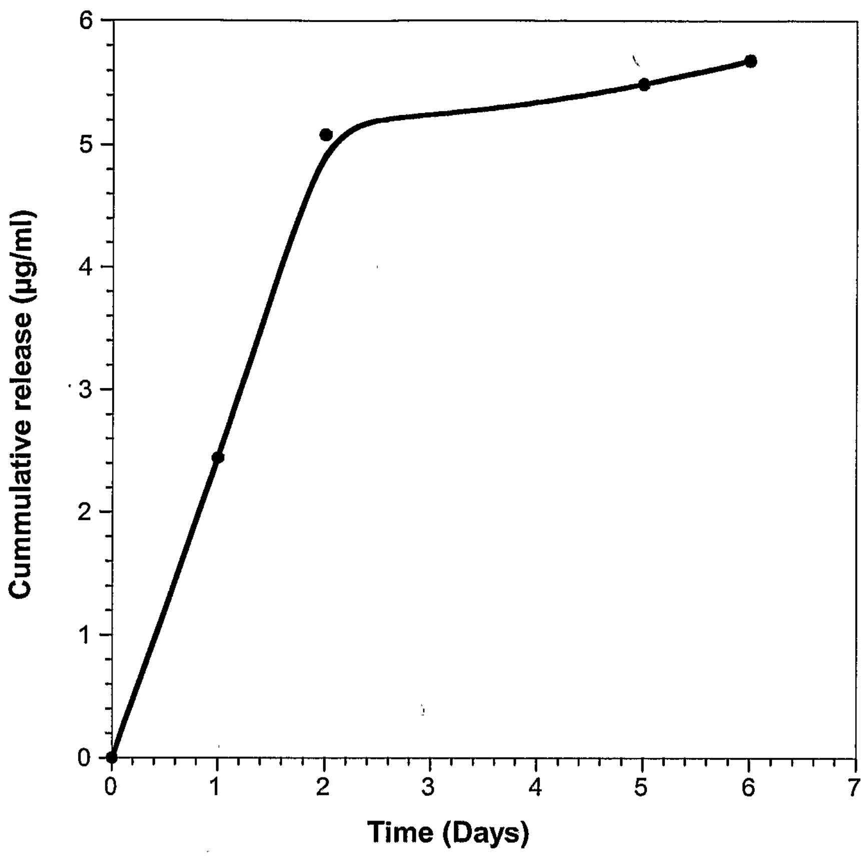


Fig. 48

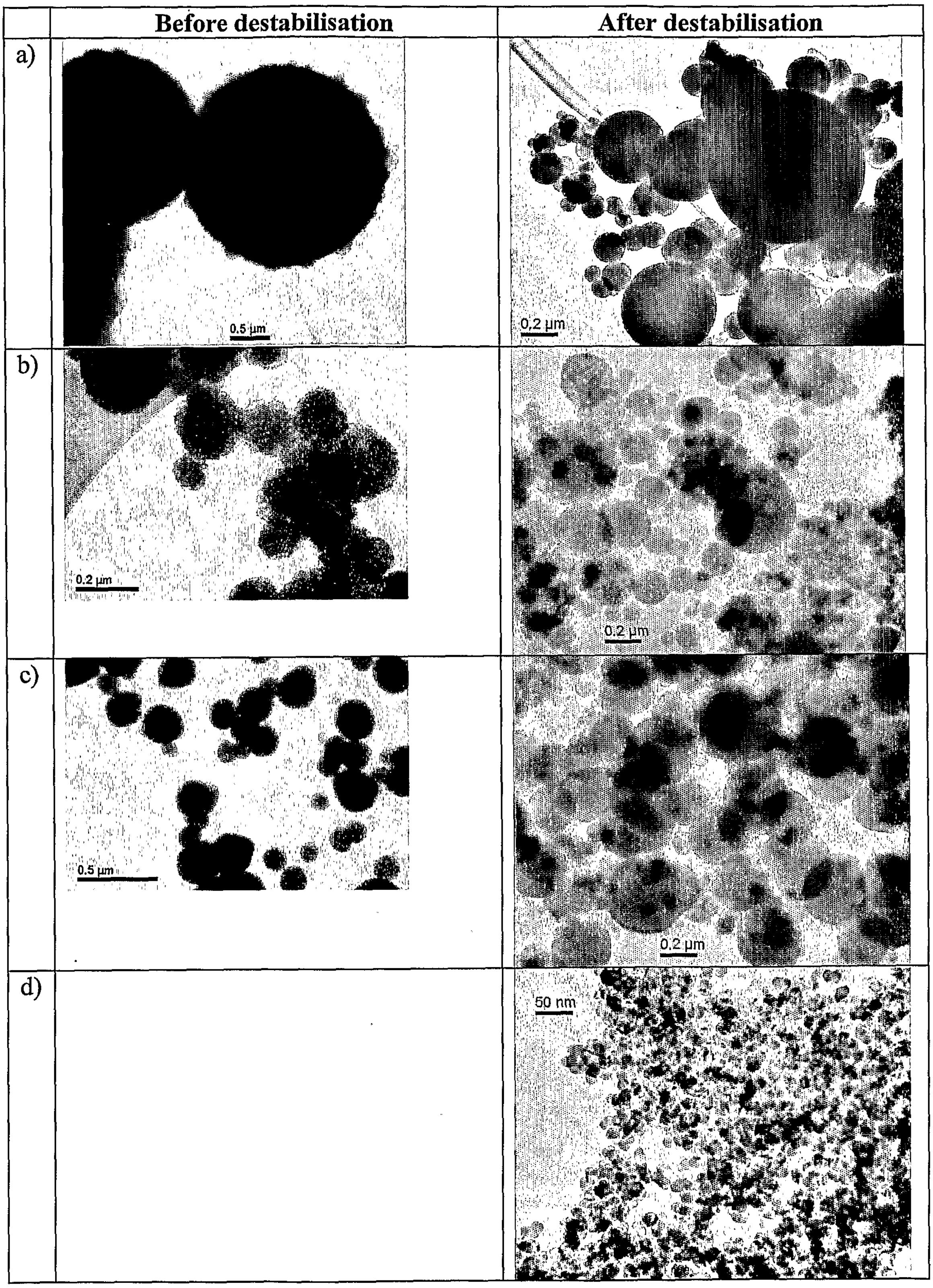
Before destabilisation
After destabilisation
a)
0.5 µm
0,2 µm
b)
0.2 µm
0,2 µm
c)
0.5 µm
0,2 µm
d)
50 nm

Fig. 49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0188540 A
- US 6174512 B
- US 2003059472 A
- WO 162332 A, Barbé & Bartlett


### Non-patent literature cited in the description


- **C-L CHANG ; HS FOGLER.** *Langmuir,* 1997, vol. 13, 3295-3307
- **P.WEBB ; C. ORR.** Analytical Methods in Fine Particles Technology. Micromeretics Corporation Norcross GA, 1997, 81-87
- **M. VASILESCU et al.** *Adv. Colloid Interface Sci.,* 2001, vol. 89-90, 169-194
- **K. OSSEO-ASARE.** *Colloids and Surfaces,* 1990, vol. 50, 321-339
- **CHIA-LU CHANG et al.** *Langmuir,* 1997, vol. 13, 3295-3307
- **ROBERT J. ROBSON et al.** *J. Phys. Chem.,* 1977, vol. 81 (11), 1075-1078
- **D. M. ZHU et al.** *J. Phys. Chem.,* 1992, vol. 96, 2382-2385
- **F.J. ARRIAGADA ; K. OSSEO-ASARE.** *J. Colloid Interface Sci.,* 1995, vol. 170, 8-17